# EUROPEAN PATENT APPLICATION

(11) **EP 1 192 952 A2**
(43) Date of publication of application: **03.04.2002**
(21) Application number: 01307657.5
(22) Date of filing: 10.09.2001
(51) Int. Cl.: A61K 45/06, A61P 25/00

(54) **Combination, for treating depression and anxiety, containing an NK-3 receptor antagonist and a CNS penetrant NK-1 receptor antagonist**

(30) Priority: 28.09.2000 US 236375 P
(71) Applicant: Pfizer Products Inc., Groton, Connecticut 06340 (US)
(72) Inventor: Lowe, John Adams, III, Pfizer Global, Groton, Connecticut 06340 (US); Mclean, Stafford, Pfizer Global, Groton, Connecticut 06340 (US); Sobolov-Jaynes, Susan Beth, Pfizer Global, Groton, Connecticut 06340 (US)
(74) Representative: Ruddock, Keith Stephen

(57) **Abstract**

The present invention relates to a method of treating depression or anxiety in a mammal, including a human, by administering to the mammal a CNS-penetrant NK-1 receptor antagonist (*e.g.* a substance P receptor antagonist) in combination with an NK-3 antagonist agent. It also relates to pharmaceutical compositions containing a pharmaceutically acceptable carrier, a CNS-penetrant NK-1 receptor antagonist and an NK-3 antagonist.

## Description

### Background Of The Invention

The present invention relates to a method of treating depression or anxiety in a mammal, including a human, by administering to a mammal a CNS-penetrant NK-1 receptor antagonist (e.g., a substance P receptor antagonist) in combination with an NK-3 antagonist. It also relates to pharmaceutical compositions containing a pharmaceutically acceptable carrier, a CNS-penetrant NK-1 receptor antagonist and an NK-3 antagonist.

Major depression is characterized by feelings of intense sadness and despair, mental slowing and loss of concentration, pessimistic worry, agitation, and self-deprecation. Physical changes also occur, especially in severe or "melancholic" depression. These include insomnia or hypersomnia, anorexia and weight loss (or sometimes overeating), decreased energy and libido, and disruption of normal circadian rhythms of activity, body temperature, and many endocrine functions.

Treatment regimens commonly include the use of tricyclic antidepressants, monoamine oxidase inhibitors, some psychotropic drugs, lithium carbonate, and electroconvulsive therapy (ECT) (see R. J. Baldessarini in *Goodman & Gilman's The Pharmacological Basis of Therapeutics,* 9th Edition, Chapter 19, McGraw-Hill, 1996 for a review). More recently, new classes of antidepressant drugs are being developed including selective serotonin reuptake inhibitors (SSRIs), specific monoamine reuptake inhibitors and 5-HT_{IA} receptor agonists, antagonists and partial agonists.

Anxiety is an emotional condition characterized by feelings such as apprehension and fear accompanied by physical symptoms such as tachycardia, increased respiration, sweating and tremor. It is a normal emotion but when it is severe and disabling it becomes pathological.

Anxiety disorders are generally treated using benzodiazepine sedative-antianxiety agents. Potent benzodiazepines are effective in panic disorder as well as in generalized anxiety disorder, however, the risks associated with drug dependency may limit their long-term use. 5-HT_{IA} receptor partial agonists also have useful anxiolytic and other psychotropic activity, and less likelihood of sedation and dependence (see R. J. Baldessarini in *Goodman & Gilman's Tite Pharmacological Basis of Therapeutics,* 9th Edition, Chapter 18, McGraw-Hill, 1996 for a review).

### Summary Of The Invention

The present invention relates to a pharmaceutical composition for the treatment of anxiety or depression comprising: (a) an NK-3 antagonist, or a pharmaceutically acceptable salt thereof; (b) a CNS-penetrant NK-1 receptor antagonist or pharmaceutically acceptable salt thereof; and (c) a pharmaceutically acceptable carrier; wherein the active agents "a" and "b" above are present in amounts that render the composition effective in treating, respectively, anxiety or depression.

This invention also relates to a method of treating anxiety or depression in a mammal, comprising administering to said mammal, respectively, an anxiolytic or antidepressant effective amount of a pharmaceutical composition comprising: (a) an NK-3 antagonist, or a pharmaceutically acceptable salt thereof; (b) a CNS-penetrant NK-1 receptor antagonist or pharmaceutically acceptable salt thereof; and (c) a pharmaceutically acceptable carrier; wherein the active agents "a" and "b" above are present in amounts that render the composition effective in treating, respectively, anxiety or depression.

This invention also relates to a method of treating anxiety or depression in a mammal, comprising administering to said mammal: (a) an NK-3 antagonist, or a pharmaceutically acceptable salt thereof; and (b) a CNS-penetrant NK-1 receptor antagonist or pharmaceutically acceptable salt thereof; wherein the active agents "a" and "b" above are administered in amounts that render the combination of the two agents effective in treating, respectively, anxiety or depression.

It will be appreciated that when using a combination method of the present invention, referred to immediately above, both the CNS-penetrant NK-1 receptor antagonist and the NK-3 antagonist will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the NK-3 antagonist may be administered as a tablet and then, within a reasonable period of time, the CNS-penetrant NK-1 receptor antagonist may be administered either as an oral dosage form such as a tablet or a fast-dissolving oral dosage form. By a "fast dissolving oral formulation" is meant, an oral delivery form which when placed on the tongue of a patient, dissolves within about seconds.

The compositions of the present invention that contain an NK-1 receptor antagonist and a NK-3 antagonist are useful for the treatment of depression. As used herein, the term "depression" includes depressive disorders, for example, single episodic or recurrent major depressive disorders, and dysthymic disorders, depressive neurosis, and neurotic depression; melancholic depression including anorexia, weight loss, insomnia and early morning waking, and psychomotor retardation; atypical depression (or reactive depression) including increased appetite, hypersomnia, psychomotor agitation or irritability, anxiety and phobias, seasonal affective disorder, or bipolar disorders or manic depression, for example, bipolar I disorder, bipolar II disorder and cyclothymic disorder.

Other mood disorders encompassed within the term "depression" include dysthymic disorder with early or late onset and with or without atypical features; dementia of the Alzheimer's type, with early or late onset, with depressed mood; vascular dementia with depressed mood, disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

The compositions of the present invention that contain an NK-1 receptor antagonist and an NK-3 antagonist are useful for the treatment of anxiety. As used herein, the term "anxiety" includes anxiety disorders, such as panic disorder with or without agoraphobia, agoraphobia without history of panic disorder, specific phobias, for example, specific animal phobias, social phobias, obsessive-compulsive disorder, stress disorders including post-traumatic stress disorder and acute stress disorder, and generalized anxiety disorders.

"Generalized anxiety" is typically defined as an extended period (e.g. at least six months) of excessive anxiety or worry with symptoms on most days of that period. The anxiety and worry is difficult to control and may be accompanied by restlessness, being easily fatigued, difficulty concentrating, irritability, muscle tension, and disturbed sleep.

"Panic disorder" is defined as the presence of recurrent panic attacks followed by at least one month of persistent concern about having another panic attack. A "panic attack" is a discrete period in which there is a sudden onset of intense apprehension, fearfulness or terror. During a panic attack, the individual may experience a variety of symptoms including palpitations, sweating, trembling, shortness of breath, chest pain, nausea and dizziness. Panic disorder may occur with or without agoraphobia.

"Phobias" includes agoraphobia, specific phobias and social phobias. "Agoraphobia" is characterized by an anxiety about being in places or situations from which escape might be difficult or embarrassing or in which help may not be available in the event of a panic attack. Agoraphobia may occur without history of a panic attack. A "specific phobia" is characterized by clinically significant anxiety provoked by feared object or situation. Specific phobias include the following subtypes: animal type, cued by animals or insects; natural environment type, cued by objects in the natural environment, for example storms, heights or water; blood-injection-injury type, cued by the sight of blood or an injury or by seeing or receiving an injection or other invasive medical procedure; situational type, cued by a specific situation such as public transportation, tunnels, bridges, elevators, flying, driving or enclosed spaces; and other type where fear is cued by other stimuli. Specific phobias may also be referred to as simple phobias. A "social phobia" is characterized by clinically significant anxiety provoked by exposure to certain types of social or performance circumstances. Social phobia may also be referred to as social anxiety disorder.

Other anxiety disorders encompassed within the term "anxiety" include anxiety disorders induced by alcohol, amphetamines, caffeine, cannabis, cocaine, hallucinogens, inhalants, phencycedine, sedatives, hypnotics, anxiolytics and other substances, and adjustment disorders with anxiety or with mixed anxiety and depression.

Anxiety may be present with or without other disorders such as depression in mixed anxiety and depressive disorders. The compositions of the present invention are therefore useful in the treatment of anxiety with or without accompanying depression.

The compositions of the present invention are especially useful for the treatment of depression or anxiety. By the use of a combination of a CNS-penetrant NK-1 receptor antagonist and an NK-3 antagonist in accordance with the present invention, it is possible to treat depression and/or anxiety in patients for whom conventional antidepressant or antianxiety therapy might not be wholly successful or where dependence upon the antidepressant or antianxiety therapy is prevalent.

Suitable classes of NK-3 antagonist that were disclosed in PCT/US95/13058 may be used in the methods and pharmaceutical compositions of this invention are indane amide derivatives selected from the group consisting of
(1α, 2β, 3α)-(+/-)-1-(4-methoxyphenyl)-3-(3,4-methylenedioxyphenyl)indane-2-N, N-dimethyl carboxamide;
(1α,2β,3α)-(+/-)-1-(4-methoxyphenyl)-3-(3,4-methylenedioxyphenyl)indane-2-N-methyl-N-(2-phenylethyl) carboxamide;
(1α,2β,3α)-(+/-)-1-(4-methoxyphenyl)-3-(3,4-methylenedioxyphenyl)indane-2-N--[2-(N,N-diethylaminoethyl)] carboxamide;
(1α,2β,3α)-(+/-)-1-(4-methoxyphenyl)-3-(3,4-methylenedioxyphenyl)indane-2-N--[2-(4-nitrophonylethyl] carboxamide;
(1α,2β,3α)-(+/-)-1-(4-methoxyphenyl)-3-(3,4-methylenedioxyphenyl)indane-2-N-(2-phenylethyl) carboxamide;
(1α,2β,3α)-(+/-)-1-(4-methoxyphenyl)-3-(3,4-methylenedioxyphenyl)indane-2-N-[(2-methoxyphenyl)methyl] carboxamide;
(1α,2β,3α)-(+/-)-1-(4-methoxyphenyl)-3-(3,4-methylenedioxyphenyl)indane-2-N-[2-(2-methoxyphenyl)ethyl] carboxamide;
(1α,2β,3α)-(+/-)-1-(4-methoxyphenyl)-3-(3,4-methylenedioxyphenyl)indane-2-N-[2-(3,4-mothylenedioxyphenyl)ethyl] carboxamide;
(1α,2β,3α)-(+/-)-1-(4-methoxyphenyl)-3-(3,4-methylenedioxyphenyl)indane-2-N-[2-(3,4-dimethoxyphenyl)ethyl] carboxamide;
(1α,2β,3α)-(+/-)-1-(4-methoxyphenyl)-3-(3,4-methylenedioxyphenyl)indane-2-N-[3-(N,N-diethylamino)propyl] carboxamide;
(1α,2β,3α)-(+/-)-1-(4-methoxyphenyl)-3-(3,4-methylenedioxyphenyl)indane-2-N-[(methoxycarbonyl)phenylmethyl] carboxamide;
(1α,2β,3α)-(+/-)-1-(4-methoxyphenyl)-3-(3,4-methylenedioxyphenyl)indane-2-N-[(hydroxycarbonyl)phenylmethyl] carboxamide;
(1α,2β,3α)-(+/-)-1-(4-methoxyphenyl)-3-(3,4-methylenedioxyphenyl)indane-2-N-ethyl carboxamide;
(1α,2β,3α)-(+/-)-1-(4-methoxyphenyl)-3-(3,4-methylenedioxyphenyl)indane-2-N-2-methylpropyl carboxamide;
(1α,2β,3α)-(+/-)-1-(4-methoxyphenyl)-3-(3,4-methylenedioxyphenyl)indane-2-N-phenylmethyl carboxamide;
(1α,2β,3α)-(+/-)-1-(4-methoxyphenyl)-3-(3,4-methylenedioxyphenyl)indane-2-N-phenyl carboxamide;
(1α,2β,3α)-(+/-)-1-(4-methoxyphenyl)-3-(3,4methylenedioxyphenyl)indane-2-N-(2-phenyl) carboxamide;
(1α,2β,3α)-(+/-)-1-(4-methoxyphenyl)-3-(3,4-methylenedioxyphenyl)indane-2-N-piperazinyl carboxamide;
(1α,2β,3α)-(+/-)-1-(4-methoxyphenyl)-3-(3,4-methylenedioxy-phenyl)indane-2-N-[(methoxycarbonyl)phenylethyl] carboxamide;
(1α,2β,3α)-(+/-)-1-(4-methoxyphenyl)-3-(3,4-methylenedioxyphenyl)-indane-2-N,Ndimethylcarboxamide;
(1α,2β,3α)-(+/-)-1-(4-methoxyphenyl)-3-(3,4-methylenedioxyphenyl)-indane-2-carboxylic acid, 2,2-dimethylhydrazide;
(1α,2β,3α)-(+/-)-1-(4-hydroxyphenyl)-3-(3,4-methylenedioxyphenyl)indane-2-N-methylcarboxamide;
(1α,2β,3α)-(+/-)-1,3-bis(3,4-methylenedioxyphenyl)indane-2-N-methylcarboxamide; and
(1α,2β,3α)-(+/-)-5,6-methylenedioxy-1,3-bis(3,4-methylenedioxyphenyl)-indane-2-N-methylcarboxamide.

Another class of NK-3 antagonist disclosed in U.S. Patent No 5,811,553 that may be used in this invention are quinoline derivatives selected from the group consisting of
(R,S)-N-(α-methylbenzyl)-2-phenylquinoline-4-carboxamide;
(+)-(S)-N-(α-methylbenzyl)-2-phenylquinoline-4-carboxamide;
(-)-(R)-N-(α-methylbenzyl)-2-phenylquinoline-4-carboxamide;
(R,S)-N-[α-(methoxycarbonyl)benzyl]-2-phenylquinoline-4 carboxamide;
(+)-(S)-N-[α-(methoxycarbonyl)benzyl]-2-phenylquinoline-4-carboxamide;
(-)-(R)-N-[Basis α-(methoxycarbonyl)benzyl]-2-phenylquinoline-4-carboxamide;
(R,S)-N-[α-(methoxycarbonyl)benzyl]-7-methoxy-2-phenylquinoline-4-carboxamide;
(R,S)-N-[α-(methoxycarbonyl)benzyl]-7-hydroxy-2-phenylquinoline-4-carboxamide;
(R,S)-N-[α-(carboxy)benzyl]-7-methoxy-2-phenylquinoline-4-carboxamide hydrochloride;
(R,S)-N-[α-(methylamninocarbonyl)benzyl]-2-phenylquinoline-4-carboxamide;
(R,S)-N-[α-(methoxycarbonyl)benzyl]-2-(2-thienyl)quinoline-4-carboxamide;
(R,S)-N-[α-(methoxycarbonyl)benzyl]-2-(2-furyl)quinoline-4-carboxamide;
(R,S)-N-[α-(methoxycarbonyl)benzyl]-2-(4-pyridyl)quinoline-4-carboxamide;
(R,S)-N-[α-(methoxycarbonyl)-2-thienylmethyl]-2-phenylquinoline-4-carboxamide;
(R,S)-N-[α-(methoxycarbonylmethyl)benzyl]-2-phenylquinoline-4-carboxamide;
(-)-(R)-N-(α-(methoxycarbonyl)-1,4-cyclohexadienylimethyl]-2-phenylquinoline-4-carboxamide;
(R,S)-N-[α-(I-hydroxyethyl)benzyl]-2-phenylquinoline-4-carboxamide;single diast;
(R,S)-N-(α-ethylbenzyl )-3-methoxy-2-phenylquinoline-4-carboxamide;
(R,S)-N-(α-ethylbenzyl )-3-n-butyl-2-phenylquinoline-4-carboxamide;
(R,S)-N-[α-(methoxycarbonyl)benzyl]benzo-1,3-cycloheptadieno[1,2-,b]quinoline-8-carboxamide;
(R,S)-N-(α-ethylbenzyl)-3-hexyl-2-phenylquinoline4-carboxamide;
(-)-(S)-N-(α-ethylbenzyl)-3-methyl-2-phenylquinoline-4-carboxamide;
(+)-(R)-N-(α-ethylbenzyl),-3-methyl-2-phenylquinoline-4-carboxamide;
(R,S)-N-[α-(methoxycarbonyl )benzyl-2-(2-methoxyphenyl)quinoline-4-carboxamide;
(R,S)-N-(α-ethylbenzyl)-3-phenyl-2-phenylquinoline-4-carboxamide;
(R,S)-N-[α-(methoxycarbonyl)benzyl]-2-(2-fluorophenyl)quinoline-4-carboxamide;
(R,S)-N-[α-(ethyl)-3,4-dichlorobenzyl]-2-phenylquinoline-4-carboxamide;
(R,S)-N-[α-(hydroxymethyl)benzyl]-2-phenylquinoline-4-carboxamide;
(R,S)-N-(α-ethylbenzyl)-2-phenylquinoline-4-carboxamide;
(R,S)-N-[α-(methoxycarbonyl)benzyl]-3-methyl-2-phenylquinoline-4-carboxamide;
(R,S)-N-(α-ethylbenzyl)-3-methyl-2-phenylquinoline-4-carboxamide;
(R,S)-N-[α-(methoxycarbonyl)benzyl]-7-chloro-2-phenylquinoline-4-carboxamide;
(R,S)-N-[α-(methoxycarbonyl)benzyl]-6-methyl-2-phenylquinoline-4-carboxamide;
(R,S)-N-[α-(methoxymethyl)benzyl]-2-phenylquinoline-4-carboxamide;
(R,S)-N-[α-(methoxycarbonyl)benzyl]-6-chloro-2-phenylquinoline-4-carboxamide;
(R,S)-N-[α-(methoxycarbonyl)benzyl]-3-ethyl-2-phenylquinoline-4-carboxamide;
(R,S)-N-(α-n-propylbenzyl)-2-phenylquinoline-4-carboxamide;
(R,S)-N-(α-ethylbenzyl)-3-ethyl-2-phenylquinoline-4-carboxamide;
(R,S)-N-(α-ethylbenzyl)-3-phthamido-2-phenylquinoline-4-carboxamide;
(R,S)-N-(α-ethylbenzyl)-3-n-propyl-2-phenylquinoline-4-carboxamnide;
(-)-(S)-N-(α-ethylbenzyl)-6-bromo-3-methyl-2-(4-bromophenyl)quinoline-4-carboxamide;
(-)-(S)-N-(α-ethylbenzyl)-6-bromo-3-methyl-2-phenylquinoline-4-carboxamide;
(R,S)-N-(α-(methoxycarbonyl)benzyl]-6-methoxy-2-phenylquinoline-4-carboxamide;
(R,S)-N-[α-(methoxycarbonyl)benzyl]-2-(2-benzofuryl)quinoline-4-carboxamide;
(R,S)-N-((1,2-diphenyl)ethyl]-2-phenylquinoline-4-carboxamide;
(R,S)-N-(α-trifluoromethylbenzyl)-2-phenylquinoline-4-carboxamide;
(-)-(S)-N-(α-ethylbenzyl)-3-methoxy-2-phenylquinoline-4-carboxamide;
(-)-(S)-N-(α-ethylbenzyl)-3-ethyl-2-phenylquinoline-4-carboxamide;
(R,S)-N-(α-(ethyl)-4-chlorobenzyl]-2-phenylquinoline-4-carboxamide;
(R,S)-N-[(α-(methoxycarbonyl)benzyl]-N-methyl-2-phenylquinoline-4-carboxamide;
(R,S)-N-[α-(methoxycarbonyl)benzyl]-2-(3-thienyl)quinoline-4-carboxamide;
(R,S)-N-[α-(methoxycarbonyl)benzyl]-5,6-dihydrobenzo[a]acridine-7-carboxamide;
(R,S)-N-[α-(methoxycarbonyl)benzyl]-2-(2-pyrryl)quinoline-4-carboxamide;
(R,S)-N-[(α-(methoxycarbonyl)benzyl]-2-(2-thiazolyl)quinoline-4-carboxamide;
(R,S)-N-(1-indanyl)-2-phenylquinoline-4-carboxamide;
(R,S)-N-(α-n-butylbenzyl)-2-phenylquinoline-4-carboxamide;
(R,S)-N-[α-(methoxycarbonyl)benzyl]-2-(4-methylphenyl)quinoline-4-carboxamide;
(R,S)-N-(α-heptylbenzyl)-2-phenylquinoline-4-carboxamide;
(R,S)-N-[α-(methoxycarbonyl)benzyl]-2-(2-methylphenyl)quinoline-4-carboxamide;
(R,S)-N-[α-(methoxyCarbonyl)benzyl]-2-(4-methoxyphenyl)quinoline-4-carboxamide;
N-(1-phenylcyclopentyl)-2-phenylquinoline-4-carboxamnide;
(R,S)-N-[α-(methoxycarbonyl)benzyl]-2-(4-hydroxyphenyl)quinoline-4-carboxamide;
(R,S)-N-[α-(methoxycarbonyl)benzyl]-2-(3,4-methylendioxyphenyl)quinoline-4-carboxamide;
N-(α,a-dimethylbenzyl)-2-phenylquinoline-4-carboxamide;
(R,S)-N-[α-(ethyl)-4-methylbenzyl]-2-phenylquinoUne4-carboxamide;
(R,S)-N-[α-(methoxycarbonyl)benzyl]-2-(3-pyrryl)quinoline-4-carboxamide;
(R,S)-N-[α-(methoxycarbonyl)benzyl]-2-(3,4-dichlorophenyl)quinoline-4-carboxamide;
(-)-(R)-N-[α-(aminomethyl)benzyl]-2-phenylquinoline-4-carboxamide;
(-)-(S)-N-(α-ethylbenzyl)-3-amino-2-phenylquinoline-4-carboxamide;
(-)-(S)-N-(α-ethylbenzyl)-3-chloro-2-phenylquinoline-4-carboxamide;
(-)-(S)-N-(α-ethylbenzyl)-3-bromo-2-phenylquinoline-4-carboxamide;
(R,S)-N-(α-iso-propylbenzyl)-2-phenylquinoline-4-carboxamide;
(-)-(S)-N-(α-ethylbenzyl)-2-phenylquinoline-4-carboxamide;
(+)-(R)-N-(α-ethylbenzyl)-2-phenylquinoline-4-carboxamide;
(R,S)-N-[α-(methoxycarbonyl)benzyl]-6-fluoro-2-phenylquinoline-4-carboxamide;
(R,S)-N-(α-(methoxycarbonyl)benzyl]-2-cyclohexylquinoline-4-carboxamnide;
(R,S)-N-[α-(methoxycarbonyl)benzyl]-2-(3-chlorohenyl)quinoline-4-carboxamide;
(R,S)-N-(α-(methoxycarbonyl)benzyl]-2-(2-chlorophenyl)quinoline-4-carboxamide;
(R,S)-N-(α-ethylbenzyl)-3-hydroxy-2-phenylquinoline-4-carboxamide;
(R,S)-N-[α-(methoxycarbonyl)benzyl]-8-acetyloxy-2-phenylquinoline-4-carboxamide:
(R,S)-N-[α-(methoxycarbonyl)benzyl]-8-hydroxy-2-phenylquinoline-4-carboxamide;
(R,S)-N-[α-(methoxycarbonyl)benzyl]-2-(2,4-dichlorophenyl)quinoline-4-carboxamide;
(-)-(R)-N-(α-(methoxycarbonyl)-4-hydroxybenzyl]-2-phenylquinoline-4-carboxamide hydrochloride;
N-diphenylmethyl-2-phenylquinoline-4-carboxamide;
(-)-(S)-N-(α-ethylbenzyl)-3-hydroxy-2-phenylquinoline-4-carboxamide;
(+)-(R)-N-(α-ethylbenzyl)-3-hydroxy-2-phenylquinoline-4-carboxamide;
(-)-(R)-N-[α-(methoxycarbonyl)benzyl]-3-hydroxy-2-phenylquinoline-4-carboxamide;
(-)-(R)-N-[α-(dimethylaminomethyl)benzyl]-2-phenylquinoline-4-carboxamide;
(R,S)-N-[α-(dimethylaminocarbonyl)benzyl]-2-phenylquinoline-4-carboxamide;
(R,S)-N-[α-(aminocarbonyl)benzyl]-2-phenylquinoline-4-carboxamide;
(R,S)-N-[α-(1-pyrrolidinylcarbonyl)benzyl]-2-phenylquinoline-4-carboxamide;
(-)-(R)-N-(α-(carboxy)benzyl]-2-phenylquinoline-4-carboxamide hydrochloride;
(R,S)-N-[α-(methoxycarbonyl)benzyl]-2-(4-chlorophenyl)quinoline-4-carboxamide;
(R)-N-[α-(methoxycarbonyl)-4-methoxybenzyl]-2-phenylquinoflne-4-carboxamide;
(R,S)-N-[α-(methoxycarbonyl)-α-(methyl)benzyl]-N-methyl-2-phenylquinoline-4-carboxamide hydrochloride;
(R,S)-N-[α-(methylcarbonyl)benzyl]-2-phenylquinoline-4-carboxamide;
(R,S)-N-[α-(2-hydroxyethyl)benzyl]-2-phenylquinoline-4-carboxamide;
(-)-(S)-N-(α-ethylbenzyl)-3-(2-dimethylaminoethoxy)-2-phenylquinoline-4-carboxamide hydrochloride;
(-)-(S)-N-(α-ethylbenzyl)-3-acetylamino-2-phenylquinoline-4-carboxamide;
(-)-(S)-N-(α-ethylbenzyl)-3-(3-dimethylinopropoxy)-2-phenylquinoline-4-carboxamide hydrochloride;
(-)-(S)-N-(α-ethylbenzyl)-3-[2-(1-phthaloyl)ethoxyl-2-phenylquinoline-4-carboxamide hydrochloride;
(-)-(S)-N-(α-ethylbenzyl)-3-(2-aminoethoxy)-2-phenylquinoline-4-carboxamide hydrochloride;
(+)-(S)-N-(α-ethylbenzyl)-3-[2-(1-pyrrolidinyl)ethoxyl-2-phenylquinoline-4-carboxamide hydrochloride;
(-)-(S)-N-(α-ethylbenzyl)-3-(dimethylaminoacetylamino)-2-phenylquinoline-4-carboxamide;
N-(α,a-dimethylbenzyl)-3-hydroxy-2-phenylquinoline4-carboxamide;
N-(α,α-dimethylbenzyl)-3-amino-2-phenylquinoline-4-carboxamide;
(-)-(S)-N-(α-ethylbenzyl)-5-methyl-2-phenylquinoline-4-carboxamide;
(R,S)-N-[α-(1-hydroxyethyl)benzyl]-3-methyl-2-phenylquinoline-4-carboxide;
(R,S)-N-[α-(methylcarbonyl)benzyl]-3-methyl-2-phenylquinoline-4-carboxamide;
(R,S)-N-[α-(ethyl)-4-pyridylmethyl]-2-phenylquinoline-4-carboxamide;
(R,S)-N-[α-(ethyl)-2-thienylmethyl]-2-phenylquinoline-4-carboxamide;
(+)-(S)-N-(α-ethylbenzyl)-3-dimethylaminomethyl-2-phenylquinoline-4-carboxamide hydrochloride;
(S)-N-(α-ethylbenzyl)-3-methyl-7-methoxy-2-phenylquinoline-4-carboxamide;
(S)-N-(α-ethylbenzyl)-3-amino-5-methyl-2-phenylquinoline-4-carboxamide;
(S)-N-(α-ethylbenzyl)-3-methoxy-5-methyl-2-phenylquinoline-4-carboxamide;
(-)-(S)-N-(α-ethylbenzyl)-3-hydroxy-2-phenylquinoline-4-carboxamide.
(-)-(S)-N-(α-ethylbenzyl)-3-hydroxy-2-phenylquinoline-4-carboxamide, hydrochloride salt.
N-(2-methoxycarbonylphenyl)-2-phenyl)-2-phenylquinoline-4-carboxamide;
(R,S)-N-[(1-methoxycarbonyl-2-phenyl)ethyl]-2-phenylquinoline-4-carboxamide;
4-[(N-benzyl)aminomethyl]-7-methoxy-2-phenylquinoline dihydrochloride;
N-benzyl-7-hydroxy-2-phenylquinoline-4-carboxamide;
N-(3,4-dimethoxybenzyl)-7-methoxy-2-phenylquinoline-4-carboxamide;
N-[3,5-bis(trifluoromethyl)benzyl]-7-methoxy-2-phenylquinoline-4-carboxamide;
N-(2-pyridylmethyl)-7-methoxy-2-phenylquinoline-4-carboxamide;
N-benzyl-2-phenylquinoline-4-carboxamide;
N-phenyl-7-methoxy-2-phenylquinoline-4-carboxamide;
N-(2-methoxybenzyl)-7-methoxy-2-phenylquinoline-4-carboxamide;
N-phenethyl-7-methoxy-2-phenylquinoline-4-carboxamide;
N-benzyl-7-methoxy-2-phenylquinoline-4-carboxamide;
(±)-(R)-N-[1-methoxycarbonyl-2-methyl)-propyl]-2-phenylquinoline-4-carboxamide;
N-benzyl-3-methyl-2-phenylquinoline-4-carboxamide;
(R,S)-N-(α-methylbenzyl)-2-phenylquinoline-4-carboxamide;
(+)-(S)-N-(α-methylbenzyl)-2-phenylquinoline-4-carboxamide;
(-)-(R)-N-(α-methylbenzyl)-2-phenylquinoline-4-carboxamide;
(R,S)-N-[α-(methoxycarbonyl)benzyl]-2-phenylquinoline-4 carboxamide;
(+)-(S)-N-[α-(methoxycarbonyl)benzyl]-2-phenylquinoline-4-carboxamide;
(-)-(R)-N-[α-(methoxycarbonyl)benzyl]-2-phenylquinoline-4-carboxamide;
(R,S)-N-[α-(methoxycarbonyl)benzyl]-7-methoxy-2-phenylquinoline-4-carboxamide;
(R,S)-N-[α-(methoxycarbonyl)benzyl]-7-hydroxy-2-phenylquinoline-4-carboxamide;
(R,S)-N-[α-(carboxy)benzyl]-7-methoxy-2-phenylquinoline-4-carboxamide hydrochloride;
(R,S)-N-([α-(methylaminocarbonyl)benzyl]-2-phenylquinoline-4-carboxamide:
(R,S)-N-[α-(methoxycarbonyl)benzyl]-2-(2-thienyl)quinoline-4-carboxamide;
(R,S)-N-[α-(methoxycarbonyl)benzyl]-2-(2-furyl)quinoline-4-carboxamide;
(R,S)-N-[α-(methoxycarbonyl)benzyl]-2-(4-pyridyl)quinoline-4-carboxamide;
(R,S)-N-(α-(methoxycarbonyl)-2-thienylmethyl]-2-phenylquinoline-4-carboxamide;
(R,S)-N-[α-(methoxycarbonylmethyl)benzyl]-2-phenylquinoline-4-carboxamide;
(-)-(R)-N-[α-(methoxycarbonyl)-1,4-cyclohexadienylmethyl]-2-phenylquinoline-4-carboxamide;
(R,S)-N-(α-(1-hydroxyethyl)benzyl]-2-phenylquinoline-4-carboxamide, single diast;
(R,S)-N-(α-ethylbenzyl)-3-methoxy-2-phenylquinoline-4-carboxamide;
(R,S)-N-(α-ethylbenzyl)-3-n-butyl-2-phenylquinoline-4-carboxamide;
(R,S)-N-[α-(methoxycarbonyl)benzyl]benzo-1,3-cycloheptadieno[1,2-b]quinoline-8-carboxamide;
(R,S)-N-(α-ethylbenzyl)-3-hexyl-2-phenylquinoline-4-carboxamide;
(-)-(S)-N-(α-ethylbenzyl)-3-methyl-2-phenylquinoline-4-carboxamide;
(+)-(R)-N-(α-ethylbenzyl)-3-methyl-2-phenylquinoline-4-carboxamide;
(R,S)-N-[α-(methoxycarbonyl)benzyl]-2-(2-methoxyphenyl)quinoline-4-carboxamide;
(R,S)-N-(α-ethylbenzyl)-3-phenyl-2-phenylquinoline-4-carboxamide;
(R,S)-N-[α-(methoxycarbanyl)benzyl-2-(2-fluorophenyl)quinoline-4-carboxamide;
(R,S)-N-[α-(ethyl)-3,4-dichlorobenzyl-2-phenylquinoline-4-carboxamide;
(R,S)-N-[α-(hydroxymethyl)benzyl]-2-phenylquinoline-4-carboxamide;
(R,S)-N-(α-ethylbenzyl)-2-phenylquinoline-4-carboxamide;
(R,S)-N-[α-(methoxycarbonyl)benzyl]-3-methyl-2-phenylquinoline-4-carboxamide;
(R,S)-N-(α-ethylbenzyl)-3-methyl-2-phenylquinoline-4-carboxamide;
(R,S)-N-[α-(methoxycarbonyl)benzyl]-7-chloro-2-phenylquinoline-4-carboxamide;
(R,S)-N-[α-(methoxycarbonyl)benzyl]-6-methyl-2-phenylquinoline-4-carboxamide;
(R,S)-N-[α-(methoxymethyl)benzyl)-2-phenylquinoline-4-carboxamide;
(R,S)-N-[α-(methoxycarbonyl)benzyl]-6-chloro-2-phenylquinoline-4-carboxamide;
(R,S)-N-[α-(methoxycarbonyl)benzyl]-3-ethyl-2-phenylquinoline-4-carboxamide;
(R,S)-N-(α-n-propylbenzyl)-2-phenylquinoline-4-carboxamide;
(R,S)-N-(α-ethylbenzyl)-3-ethyl-2-phenylquinoline-4-carboxamide;
(R,S)-N-(α-ethylbenzyl)-3-phthalimido-2-phenylquinoline-4-carboxamide;
(R,S)-N-(α-ethylbenzyl)-3-n-propyl-2-phenylquinoline-4-carboxamide;
(-)-S)-N-(α-ethylbenzyl)-6-bromo-3-methyl-2-(4-bromophenyl)quinoline-4-carboxamide;
(-)-(S)-N-(α-ethylbenzyl)-6-bromo-3-methyl-2-phenylquinoline-4-carboxamide;
(R,S)-N-[α-(methoxycarbonyl)benzyl]-6-methoxy-2-phenylquinoline-4-carboxamide;
(R,S)-N-[α-(methoxycarbonyl)benzyl]-2-(2-benzofuryl)quinoline-4-carboxamide;
(R,S)-N-[(1,2-diphenyl)ethyl]2-phenylquinoline-4-carboxamide;
(R,S)-N-(α-trifluoromethylbenzyl)-2-phenylquinoline-4-carboxamide;
(-)-(S)-N-(α-ethylbenzyl)-3-methoxy-2-phenylquinoline-4-carboxamide;
(-)-(S)-N-(α-ethylbenzyl)-3-ethyl-2-phenylquinoline-4-carboxamide;
(R,S)-N-[α-(ethyl)-4-chlorobenzyl]-2-phenylquinoline-4-carboxamide;
(R,S)-N-[α-(methoxycarbonyl)benzyl]-N-methyl-2-phenylquinoline-4-carboxamide;
(R,S)-N-[α-(methoxycarbonyl)benzyl]-2-(3-thienyl)quinoline-4-carboxamide;
(R,S)-N-[α-(methoxycarbonyl)benzyl]-5,6-dihydrobenzo[a]acridine-7-carboxamide;
(R,S)-N-[α-(methoxycarbonyl)benzyl]-2-(2-pyrryl)quinoline-4-carboxamide;
(R,S)-N-[α-(methoxycarbonyl)benzyl]-2-(2-thiazolyl)quinoline-4-carboxamide;
(R,S)-N-(1-indanyl)-2-phenylquinoline-4-carboxamide;
(R,S)-N-(α-n-butylbenzyl)-2-phenylquinoline-4-carboxamide;
(R,S)-N-[α-(methoxycarbonyl)benzyl]-2-(4-methylphenyl)quinoline-4-carboxamide;
(R,S)-N-(α-heptylbenzyl)-2-phenylquinoline-4-carboxamide;
(R,S)-N-[α-(methoxycarbonyl)benzyl]-2-(2-methylphenyl)quinoline-4-carboxamide;
(R,S)-N-[α-(methoxycarbonyl)benzyl]-2-(4-methoxypheno)quinoline-4-carboxamide;
N-(1-phenylcyclopentyl)-2-phenylquinoline-4-carboxamide;
(R,S)-N-[α-(methoxycarbonyl)benzyl]-2-(4-hydroxyphenyl)quinoline-4-carboxamide;
(R,S)-N-[α-(methoxycarbonyl)benzyl]-2-(3,4-methylendioxyphenyl)quinoline-4-carboxamide;
N-(α,α-dimethylbenzyl)-2-phenylquinoline-4-carboxamide;
(R,S)-N-[α-(ethyl)-4-methylbenzyl]-2-phenylquinoline-4-carboxamide;
(R,S)-N-[α-(methoxycarbonyl)benzyl]-2-(3-pyrryl)quinoline-4-carboxamide;
(R,S)-N-[α-(methoxycarbonyl)benzyl]-2-(3,4-dichloropheno)quinoline-4-carboxamide;
(-)-(R)-N-[α-(aminomethyl)benzyl]-2-phenylquinoline-4-carboxamide;
(-)-(S)-N-(α-ethobenzyl)-3-amino-2-phenylquinoline-4-carboxamide;
(-)-(S)-N-(α-ethylbenzyl)-3-chloro-2-phenylquinoline-4-carboxamide;
(-)-(S)-N-(α-ethylbenzyl)-3-bromo-2-phenylquinoline-4-carboxamide;
(R,S)-N-(α-iso-propylbenzyl)-2-phenylquinoline-4-carboxamide;
(-)-(S)-N-(α-ethylbenzyl)-2-phenylquinoline-4-carboxamide;
(+)-(R)-N-(α-ethylbenzyl)-2-phenylquinoline-4-carboxamide;
(R,S)-N-(α-(methoxycarbonyl)benzyl]-6-fluoro-2-phenylquinoline-4-carboxamide;
(R,S)-N-[α-(methoxycarbonyl)benzyl]-2-cyclohexylquinoline-4-carboxamide;
(R,S)-N-[α-(methoxycarbonyl)benzyl]-2-(3-chlorophenyl)quinoline-4-carboxamide;
(R,S)-N-[α-(methoxycarbonyl)benzyl]-2-(2-chlorophenyl)quinoline-4-carboxamide;
(R,S)-N-(α-ethylbenzyl)-3-hydroxy-2-phenylquinoline-4-carboxamide;
(R,S)-N-[α-(methoxycarbonyl)benzyl]-8-acetyloxy-2-phenylquinoline-4-carboxamide;
(R,S)-N-[α-(methoxycarbonyl)benzyl]-hydroxy-2-phenylquinoline-4-carboxamide;
(R,S)-N-[α-(methoxycarbonyl)benzyl]-2-(2,4-dichlorophenyl)quinoline-4-carboxamide;
(-)-(R)-N-[α-(methoxycarbonyl)-4-hydroxybenzyl]-2-phenylquinoline-4-carboxamide hydrochloride;
N-diphenylmethyl-2-phenylquinoline-4-carboxamide;
(-)-(S)- N-(α-ethylbenzyl)-3-hydroxy-2-phenylquinoline-4-carboxamide; (+)-(R)-N-(α-ethylbenzyl)-3-hydroxy-2-phenylquinoline-4-carboxamide;
(-)-(R)-N-[α-(methoxycarbonyl)benzyl]-3-hydroxy-2-phenylquinoline-4-carboxamide;
(-)-(R)-N-[α-(dimethylaminomehyl)benzyl]-2-phenylquinoline-4-carboxamide;
(R,S)-N-[α-(dimethylaminocarbonyl)benzyl]-2-phenylquinoline-4-carboxamide;
(R,S)-N-[α (aminocarbonyl)benzyl]-2-phenylquinoline-4-carboxamide;
(R,S)-N-[α-(1-pyrrolidinylcarbonyl)benzyl]-2-phenylquinoline-4-carboxamide;
(-)-(R)-N-[α-(carboxy)benzyl-2-phenylquinoline-4-carboxamidehydrochloride;
(R,S)-N-[α(methoxycarbonyl)benzyl]-2-(4-chlorophenyl)quinoline-4-carboxamide:
(R)-N-[α-(methoxycarbonyl)-4-methoxybenzyl]-2-phenylquinoline-4-carboxamide;
(R,S)-N-[α-(methoxycarbonyl)-α-(methyl)benzyl]-N-methyl-2-phenylquinoline-4-carboxamide hydrochloride;
(R,S)-N-[α-(methylcarbonyl)benzyl]-2-phenylquinoline-4-carboxamide;
(R,S)-N-[α-(2-hydroxyethyl)benzyl]-2-phenylquinoline-4-carboxamide;
(-)-(S)-N-(α-ethylbenzyl)-3-(3-dimethylaminopropoxy)-2-phenylquinoline-4-carboxamide hydrochloride;
(-)-(S)-N-(α-ethylbenzyl)-3-[2-(1-phthaloyl)ethoxy]-2-phenylquinoline-4-carboxamide hydrochloride;
(-)-(S)-N-(α-ethylbenzyl)-3-(2-aminoethoxy)-2-phenylquinoline-4-carboxamide hydrochloride;
(+)-(S)-N-(α-ethylbenzyl)-3-(2-(1-pyrrolidinyl)ethoxyl-2-phenylquinoline-4-carboxamide hydrochloride;
(-)-(S)-N-(α-ethylbenzyl)-3-(dimethylaminoacetylamino)-2-phenylquinoline-4-carboxamide;
N-(α,α-dimethylbenzyl)-3-hydroxy-2-phenylquinoline-4-carboxamide;
N-(α,α-dimethylbenzyl)-3-amino-2-phenylquinoline-4-carboxamide;
(-)-(S)-N-(α-ethylbenzyl)-S-methyl-2-phenylquinoline-4-carboxamide;
(R,S)-N-[α-(1-hydroxyethyl)benzyl-3-methyl-2-phenylquinoline-4-carboxamide;
(R,S)-N-[α-(methylcarbonyl)benzyl]-3-methyl-2-phenylquinoline-4-carboxamide;
(R,S)-N-[α-(ethyl-4-pyridylmethyl] 2-phenylquinoline-4-carboxamine;
(R,S)-N-(α-(ethyl)-2-thienylmethyl]-2-phenylquinoline-4-carboxamide;
(+)-(S)-N-(α-ethylbenzyl)-3-dimethylaminomethyl-2-phenyiquinoiine-4-carboxamide hydrochloride;
(S)-N-(α-ethylbenzyl)-3-methyl-7-methoxy-2-phenylquinoline-4-carboxamide;
(S)-N-(α-ethylbenzyl)-3-amino-5-methyl-2-phenylquinoline-4-carboxamide;
(S)-N-(α-ethylbenzyl)-3-methoxy-5-methyl-2-phenylquinoline-4-carboxamide;
(S)-N-(α-ethylbenzyl)-3-[(S)-2-(methoxycarbonyl)pyrrolidin-1-yl]-2-phenylquinoline-4-carboxamide hydrochloride;
(S)-N-(α-ethylbenzyl)-3-[(S)-2-(hydroxymethyl)pyrrolidin-1-yl]-2-phenylquinoline-4-carboxamide;
(S)-N-(α-ethylbenzyl)-3-[(S)-2-carboxypyrrolidine-1-yl]-2-phenylquinoline-4-carboxamide;
(S)-N-(α-ethylbenzyl)-3-[(4-oxopiperidin-1-yl)methyl]-2-phenylquinoline-4-carboxamide;
(S)-N-(α-ethylbenzyl)-3-[(4-hydroxypiperidin-1-yl)methyl]-2-phenylquinoline-4-carboxamide;
(S)-N-(α-ethylbenzyl)-3-[(piperazin-1-yl)methyl]-2-phenylquinoline-4-carboxamide; and
(S)-N-(α-ethylbenzyl)-3-[(3-oxo)pyrrolidin-1-ylmethyl]-2-phenylquinoline-4-carboxamide.

Other suitable NK-3 antagonists disclosed in PCT/FR96/01416 that may be used in this invention include piperidine derivatives selected from the group consisting of
1-benzoyl-3-(3,4-dichlorophenyl)-3-[3-[4-(pyrrolidin-1-yl)carbonyl)piperid-1-yl]propyl]piperidine;
1-benzoyl-3-(3,4-dichlorophenyl)-3-[3-(4-piperidinopiperid-1-yl)propyl]piperidine;
1-benzoyl-3-(3,4-dichlorophenyl)-3-[3-(4-carbamoyl-4-piperidinopiperd-1-yl)propyl]piperidine;
3-[3-[4-(acryloyl-N-methylamino)-4-phenylpiperid-1-yl]propyl]-1-benzoyl-3-(3,4-dichlorophenyl)piperidine;
3-[3-(4-(2-aminothiazol-4-yl)-4-phenylpiperid-1-yl]propyl]-1-benzoyl-3-(3,4-dichlorophenyl)piperidine;
3-[3-(4-acetyl-4-benzylpiperid-1-yl)propyl]-1-benzoyl-3-(3,4-dichlorophenyl) piperidine;
3-[3-[4-(acetylamino)-4-benzylpiperid-1-yl]propyl]-1benzoyl-3-(3,4-dichlorophenyl) piperdine;
1-benzoyl-3-[3-[4-benzyl-4-(propionylaminomethyl)piperid-1-yl]propyl]-3-(3,4-dichlorophphenyl)piperidine;
1-benzoyl-3-[3-[4-benzyl-4-(ethoxycarbonylamino)piperid-1-yl]propyl]-3-(3,4-dichlorophenyl)piperdine;
1-benzoyl-3-[3-[4-benzyl-4-(pyrrolidin-1-ylcarbonyl)piperid-1'-yl]propyl]-3-(3,4-dichlorophenyl)piperidine ;
1-benzoyl-3-(3,4-dichlorophenyl)-3-[3-[4-(dimethylaminocarbonyl)-4-phenylpiperid-1-yl]propyl]perhydroazepine;
1-benzoyl-3-(3,4-dichlorophenyl)-3-[3-[4-(2-hydroxyethoxy)-4-phenylpiperid-1-yl]propyl]piperidine;
3-[3-[4-(2-acetyloxyethoxy)-4-phenylpiperid-1-yl]propyl]-1-benzoyl-3-(3,4-dichlorophenyl)piperidine;
1-benzoyl-3-(3,4-dichlorophenyl)-3-[3-[4-(2-furoylamino)-4-phenylpiperid-1-yl]propyl]piperidine;
1-benzoyl-3-(3,4-dichlorophenyl)-3-[3-[4-(2-thenoylamino)-4-phenylpiperid-1-yl]propyl]piperidine;
3-(3,4-dichlorophenyl)-1-isonicotinoyl-3-[3-[4-phenyl-4-(pyrrolidin-1-ylcarbonyl)piperid-1-yl]propyl]piperidine;
1-benzoyl-3-(3,4-dichlorophenyl)-3-[3-spiro(indoline-3,4'-piperid-1-yl)propyl]piperidine;
1-benzoyl-3-(3,4-dichlorophenyl)-3-[3-1-acetyl-spiro(indoline-3,4'-piperid-1'-yl)lpropyl]piperidine;
3-(3,4-dichlorophenyl)-3-[3-[4-phenyl-4-(pyrrolidin-1-ylcarbonyl)piperid-1-yl] propyl]-1-(2-thenoyl)piperidine;
3-(3,4-dichlorophenyl)-3-[3-[4-phenyl-4-(pyrrolidin-1-yllcarbonyl)piperid-1-yl]-propyl]-1-(3-thenoyl)piperidine ;
3-(3,4-dichlorophenyl)-1-(2-furoyl)-3-[3-[4-phenyl-4-(pyrrolidin-1-ylcarbonyl) piperid-1-yl]propyl]piperidine;
3-(3,4-dichlorophenyl)-1-(3-furoyl)-3-[3-[4-phenyl-4-(pyrrolidin-1-ylcarbonyl) piperid-1-yl]propyl]piperidine;
3-[3-[4-(2-amino-1,3,4-oxadiazol-5-yl )-4phenylpiperid-1-yl]propyl]-1-benzoyl-3-(3,4-dichlorophenyl)piperidine;
1-benzoyl-3-(3,4-dichlorophenyl)-3-[3-[4-(ethoxalylamino)-4-phenylpipeid-1-yl]propyl]piperidine;
1-benzoyl-3-(3,4-dichlorophenyl)-3-[3-(4-carbamoyl-4-morpholinopiperid-1-yl)propyl]piperidine;
1-benzoyl-3-(3,4-dichlorophenyl)-3-[3-[1-(methoxycarbonyl)-spiro(indoline-3,4'-piperid-1'yl)]propyl]piperidine;
1-benzoyl-3-(3,4-dichlorophenyl)-3-[3-[1-(N,N-dimethylcarbamoyl)-spiro(indoline-3,4'-piperid-1'-yl)]propyl]piperidine ;
1-benzoyl-3-(3,4-dichlorophenyl)-3-(3-[1-(methanesulfonyl)-spiro(indoline-3,4'-piperid-1'-yl)]propyl]piperidine;

Another NK-3 antagonist from PCT/US97/15443 that may be used in the present invention are 3alky-3phenyl piperidine derivatives selected from the group consisting of
(R)-{3-(3,4-Dichlorophenyl)-3-[3-(4-phenyl-piperidin-1-yl)-propyl)-piperidin-1-yl)-phenyl-methanone monohydrochloride;
(S)-{3-(3,4-Dichlorophenyl)-3-[3-(4-hydroxy-4-phenyl-piperidin-1-yl)-propyl}-piperidin-1-yl)-phenyl-methanone monohydrochloride;
(S)-[3-[3-(4-Benzenesulfonylmethyl-4-hydroxy-piperidin-1-yl)-propyl]-3-(3,4-dichloro-phenyl)-piperidin-1-yl]-phenyl-methanone monohydrochloride;
(R)-(3-(3,4-Dichlorophenyl)-3-[3-(4-hydroxy-4-phenyl-piperidin-1-yl)-propyl]-piperidin-1-yl}-naphthalene-2-yl-methanone;
(R)-{3-(3,4-Dichlorophenyl)-3-[3-(4-hydroxy-4-phenyl-piperidin-1-yl)-propyl]-piperidin-1-yl)-pyridin-4-yl-methanone;
N-(1-{3-[3-(3,4-Dichlorophenyl)-I-(1H-imidazole-2-carbonyl)-piperidin-3-yl]-propyl)-4-phenyl-piperidin-4-yl)-acetamide;
(R)-[3-(3,4-Dichlorophenyl)-3-(3-(4-hydroxy-4-phenyl-piperidin-1-yl)-propyl]-piperidin-1-yl}-phenyl-methanone;
(R)-(3-(3,4-Dichlorophenyl)-3-[3.-(4-phenyl-piperidin-1-yl)-propyl]-piperidin-1-yl)-phenyl-methanone;
(3-(3,4-Dichlorophenyl)-3-[3-[4-(4-fluoro-phenyl)-4-hydroxy-piperidin-1-yl]-propyl)-piperidin-1-yl)-phenyl-methanone;
[3-[3-(4-Benzenesulfonylmethyl-4-hydroxy-piperidin-1-yl)propyl]-3-(3,4-dichloro-phenyl)-piperidin-1-yl]-phenyl-methanone;
(3-(4-Fluorophenyl)-3-[3-(4-hydroxy-4-phenyl-piperidin-1-yl)-propyl]-piperidin-1-yl)-phenyl-methanone;
{3-(34-Dimethoxyphenyl)-3-[3-(4-hydroxy-4-phenyl-piperidin-1-yl)-propyl]-piperidin-1-yl]-phenyl-methanone;
{3-(3,4-Dimethylphenyl)-3-[3-(4-hydroxy-4-phenyl-piperidin-1-yl)-propyl]-piperidin-1-yl)-phenyl-methanone;
[3-[3-(4-Hydroxy-4-phenyl-piperidin-1-yl)-propyl]-3-(3,4,5-trichlorophenyl)-piperidin-1-yl]-phenyl-methanone;
{3-(3,4-Dichlorophenyl)-3-[4-(4-hydroxy-4-phenylpiperidin-1-yl)-butyl]-piperidin-1-yl)-phenyl-methanone; and
{3-(3,4-Dichlorophenyl)-3-[6-(4-hydroxy-4-phenylpiperidin-1-yl)-hexyl]-piperidin-1-yl)-phenyl-methanone.

Other suitable selective NK-3 antagonists disclosed in PCT/FR99/02355 that may be used in this invention also include derivatives of ureido piperidine selected from the group consisting of

Benzenesulfonate of 4-phenyl-4-ureidopiperidine
*p*-Toluenesulfonate of 4-(N¹,N¹,-diethylureido)-4-phenylpiperidine
*p*-Toluenesulfonate of 4-(N¹,N¹,-diethylureido)-4-phenylpiperidine and
4-(N', N'-diethylureido)-4'-phenyl-1-(tert-butoxycarbonyl)piperidine.

Other suitable selective NK-3 antagonists disclosed in United States Patent No. 5,968,929 that may be used in this invention include piperazine derivatives selected from the group consisting of
2-(3,4-dichlorophenyl)piperazine2(R)-(3,4-Dichlorophenyl)-4-(3,5-dimethylbenzoyl)-1-[[[2-(phenylmethyl)-azabicyclo[2.2.1]heptan-5-yl]amino]acetyl]piperazine;
N-[2-[5-[3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]-2-oxo-1-phenylethyl]acetamide;
(±)-1,1-Dimethylethyl-4-[[2-[2-(3,4-dichlorophenyl)-1-(3,5-dimethylbenzoyl)-1-piperazinyl]-2-oxoethyl]amino]-1-piperidine carboxylate;
(-)-1,1-Dimethylethyl4-[[2-[2(R)-(3,4-dichlorophenyl)-1-(3,5-dimethylbenzoyl)-1-piperazinyl]-2-oxoethyl]amino]-1-piperidinecarboxylate;
(±)-2-(3,4-Dichlorophenyl)-4-[3,5-dimethylbenzoyl]-1-[(4-piperidinylamin o)acetyl]piperazine, dihydrochloride;
(-)-2(R)-(3,4-Dichlorophenyl)-4-[3,5-dimethylbenzoyl]-1-[(4-piperidinylamino)acetyl]piperazine, dihydrochloride;
5-[1-Cyanoimino)-1-methylthio]-2-[3-[2(R)-(3,4-dichlorophenyl)-4-(3,5-dimethylbenzoyl)-1-piperazinyl]-3-oxopropyl-2,5-diazabicyclo[2.2.1]heptane;
5-[1-(cyanoimino)-1-phenylaminomethyl]-2-[3-[2(R)-(3,4-dichlorophenyl)-4-(3,5-dimethylbenzoyl)-1-piperazinyl]-3-oxopropyl-2,5-diazabicyclo[2.2.1]heptane;
5-[1-(Cyanoimino)-1-phenylmethylaminomethyl]-2-[3-[2(R)-(3,4-dichlorophenyl)-4-(3,5-dimethylbenzoyl)-1-piperazinyl]-3-oxopropyl-2,5-diazabicyclo[2.2.1]heptane;
2(R)-(3,4-dichlorophenyl)-4-(3,5-dimethylbenzoyl)-1-[[6-(phenylmethyl)-2-azabicyclo[2.2.2]octan-6-yl]methylamino]acetyl]piperazine;
2(R)-(3,4-Dichlorophenyl)-4-(3,5-dimethylbenzoyl)-1-[[6-(phenylmethyl)-6-azabicyclo[3.2.2.]nonan-3-yl]amino]acetyl]piperazine;
Methyl [1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenyl]carbamate;
N-[1(R)-[(5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenylethyl]-N'-methylurea;
5-[3-[2(R)-(3,4-Dichlorophenyl)-4-(3,5-dimethylbenzoyl)-1-piperazinyl]-3-oxopropyl]-2-[2(R)-[[(methylamino)carbonyl]amino]-1-oxo-3-(2-thienyl)propyl]-1(S),4(S)-2,5-diazabicyclo[2.2.1]heptane;
2-[3-[2-(R)-(3,4-Dichlorophenyl)-4-(3,5-dimethylbenzyoyl)-1-piperazinyl]-3-oxopropyl]-5-[2-[[imino(methylamino)methyl]amino]-1-oxo-3-phenylpropyl]-1(S),4(S)-2,5-diazobicyclo[2.2.1]heptane;
5-[3-[2(R)-(3,4-dichlorophenyl)-4-(3,5-dimethylbenzoyl)-1-piperazinyl]-3-oxopropyl]-2-(2(R)-hydroxy-1-oxo-3-phenylpropyl)-1(S),4(S)-2,5-diazabicyclo-[2.2.1]heptane;
5-[3-[2(R)-(3,4-dichlorophenyl)-4-(3,5-dimethylbenzoyl)-1-piperazinyl]-3-oxopropyl]-2-(2(S)-hydroxy-1-oxo-3-phenylpropyl)-1(S),4(S)-2,5-diazabicyclo-[2.2.1]heptane;
2-[2(S)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabi cyclo-[2.2.1];
2-[2(R)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabi cyclo-[2.2.1]heptane;
2-[2(R)-2-(Aminohydroxyimino)ethoyl]-1-oxo-3-phenylpropyl]-5-[3-[2(R)-(3,4-dichlorophenyl)-4-(3,5-dimethylbenzoyl)-1-piperazinyl]-3-oxopropyl)-1(S),4(S)-2,5-diazabicyclo[2.2.1]heptane;
[1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate;
[1(S)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate;
2-[2(S)-Methoxy-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1]heptane;
(1R,4R)-1,1-Dimethylethyl-5-hydroxy-2-azabicyclo[2.2.1]heptane-2-carboxylate and
(1S,4S)-1,1-dimethylethyl5-hydroxy-2-azabicyclo[2.2.1]heptane-2-carboxylate;
(Exo)-1,1-dimethylethyl-5-[2-[2(R)-(3,4-dichlorophenyl)-4-(3,5-dimethylbenzoyl)-1-piperazinyl]-2-oxoethoxy]-1-(R),4(R)-2-azabicyclo[2.2.1]heptane-2-carboxylate;
2(R)-(3,4-Dichlorophenyl)-4-(3,5-dimethylbenzoyl)-1-[[[exo-1(R),4(R)-2-azabicyclo[2.2.1]heptan-5-yl]oxy]acetyl]piperazine;
2(R)-(3,4-Dichlorophenyl)-4-(3,5-dimethylbenzoyl)-1-[[[exo-2-(3-thienylmethyl)-1(S),4(S)-2-azabicyclo[2.2.1]heptan-5-yl)oxy]acetyl]piperazine;
(Exo)-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[exo-8-aza-[3.2.1]octan-3-yl]oxy]acetyl]piperazine (enantiomer) hydrochloride salt;
(Endo)-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[endo-8-aza-[3.2.1]octan-3-yl]oxy]acetyl]piperazine (enantiomer) hydrochloride salt;
N-1(R)-[[5-[3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylsulfonamide (enantiomer);
2-[2(R)-(Cyanomethylamino)-1-oxo-3-phenylpropyl]-5-[3-[2-(R)-(3,4-dichlorophenyl)-4-(3,5-dimethylbenzoyl)-1-piperazinyl]-3-oxopropyl]-1(S),4(S)-2,5-di azabicyclo[2.2.1]heptane;
2-[2(R)-[[2-(Aminohydroxyimino)ethyl]amino]-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
(±)-2(R)-(3,4-Dichlorophenyl)-4-(3,5-dimethylbenzoyl)-1-[[[endo-2-[(3,5-dimethyl-4-isoxazoyl)methyl]-2-azabicyclo[2.2.1]heptan-5-yl]amino]-acetyl]piperazine;
1,1-Dimethylethyl[1(R)-[[endo-5-[[2-[2(R)-(3,4-dichlorophenyl)-4-(3,5-dimethylbenzoyl)-1-piperazinyl]-2-oxoethyl]amino]-2-azabicyclo[2.2.1]-heptan-2-yl]carbonyl]-2-phenylethyl]carbamate;
Endo-2-(2(R)-amino-1-oxo-3-phenylpropyl)-5-[[2-[2(R)-(3,4-dichlorophenyl)-4-(3,5-dimethylbenzoyl)-1-piperazinyl]-2-oxoethyl]amino]-2-aza[2.2.1]heptane , dihydrochloride
2-(R)-(3,4-Dichlorophenyl)-4-(3,5-dimethylbenzoyl)-1-[[[8-azabicyclo-[3.2.1]octan-3-yl]endo-amino]acetyl]piperazinehydrochloride;
2-(R)-(3,4-Dichlorophenyl)-4-(3,5-dimethylbenzoyl)-1-[[[8-azabicyclo-[3.2.1]octan-3-yl]exo-amino]acetyl]piperazinehydrochloride;
1,1-Dimethylethyl [1(S)-[[exo-3-[[2-2(R)-(3,4-dichlorophenyl)-4-(3,5-dimethy-lbenzoyl)-1-piperazinyl]-2-oxoethyl]amino]-8-aza[3.2.1]octan-8-yl]carbonyl]-2-phenylethyl] carbamate (enantiomer);
Exo-8-(2(S)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
1,1-Dimethylethyl[1(S)-[[endo-3-[[2-2(R)-(3,4-dichlorophenyl)-4-(3,5-dimethylbenzoyl)-1-piperazinyl]-2-oxoethyl]amino]-8-aza[3.2.1]octan-8-yl]carbonyl]-2-phenylethyl] carbamate (enantiomer);
Endo-8-(2(S)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
1,1-Dimethylethyl [1(R)-[[exo-3-[[2-2(R)-(3,4-dichlorophenyl)-4-(3,5-dimethylbenzoyl)-1-piperazinyl]-2-oxoethyl]amino]-8-aza[3.2.1]octan-8-yl]carbonyl]-2-phenylethyl]carbamate (enantiomer);
Exo-8-(2(R)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
(±)-N-[4-[[Endo-5-[[2-[2(R)-(3,4-dichlorophenyl)-4-(3,5-dimethyl-benzoyl)-1-piperazinyl]-2-oxoethyl]amino]-2-azabicyclo[2.2.1]heptan-2-yl]methyl]-phenyl]acetamide
(±)-N-[3-[[Endo-5-[[2-[2(R)-(3,4-dichlorophenyl)-4-(3,5-dimethyl-benzoyl)-1-piperazinyl]-2-oxoethyl]amino]-2-azabicyclo[2.2.1]heptan-2-yl]methyl]-phenyl]acetamide
(±)-1,1-Dimethylethyltrans-2-[[5-[3-[2(R)-(3,4-dichlorophenyl)-4-(3,5-dimethylbenzoyl)-1-piperazinyl]-3-oxopropyl]-1(S),4(S)-2,5-diaza-bicyclo-[2.2.1]heptan-2-yl]carbonyl ]-3-phenyl-1-azetidinecarboxylate
(±)-5-[3-[2(R)-(3,4-Dichlorophenyl)-4-(3,5-dimethylbenzoyl)-1-piper-azinyl]-3-oxopropyl]-2-[(trans-3-phenyl-2-azetidinyl)carbonyl]-1(S),4(S)-2,5-diazabicyclo[2.2.1]heptane, dihydrochloride;
(±)-[3,5-dimethylbenzoyl]-3-(3,4-dichlorophenyl)piperazine;
(±)bromoacetyl-2-(3,4-dichlorophenyl)-4-(3,5-dimethylbenzoyl)piperazine;
(±)-[3,5-dimethylbenzoyl]-3-(3,4-dichlorophenyl)piperazine;
(±)-bromoacetyl-2-(3,4-dichlorophenyl)-4-(3,5-dimethylbenzoyl)piperazine;
(±)-2-(3,4-dichlorophenyl)-4-(3,5-dimethylbenzoyl)-1-[[[1-[1-oxo-2-phenyl)ethyl]-4-piperidinyl]amino]acetyl]piperazine;
(±)-1,1-dimethylethyl-4-[[2-[2-(3,4-dichlorophenyl)-1-(3,5-dimethylbenzoyl)-1-piperazinyl]-2-oxoethyl]amino]-1-piperidinecarboxylate;
(±)-1,1-dimethylethyl-4-[[2-[2-(3,4-dichlorophenyl)-1-(3,5-dimethylbenzoyl)-1-piperazinyl]-2-oxoethyl]amino]-1-piperidinecarboxylate;
(±)-2-(3,4-dichlorophenyl)-4-[3,5-dimethylbenzoyl]-1-[(4-piperidinylamino)acetyl]piperazine, dihydrochloride;
(±)-1-benzoyl-4-[[2-]2-(3,4-dichlorophenyl)-4-(3,5-dimethylbenzoyl)-1-piperazinyl]-2-oxoethyl]amino]piperidine;
(±)-2-(3,4-dichlorophenyl)-4-(3,5-dimethylbenzoyl)-1-[[[1-(2-oxo-2-phenylethyl)-4-piperidinyl]amino]acetyl]piperazine;
(±)-2-(3,4-dichlorophenyl)-4-(3,5-dimethylbenzoyl)-1-[[[1-(3-phenylpropyl)-4-piperidinyl]amino]acetyl]piperazine;
(±)-N-[4-[[[-[[[2-[2-(3,4-dichlorophenyl)-4-(3,5-dimethylbenzoyl)-1-piperazinyl]-2-oxoethyl]amino]-1-piperidinyl]methyl]-2-thiazoyl]acetamide;
(±)-2-(3,4-dichlorophenyl)-4-[3,5-dimethylbenzoyl]-1-[[[3-methyl-1-(phenylmethyl)-4-piperidinyl]amino]acetyl]piperazine;
2-(3,4-dichlorophenyl)-4-[3,5-dimethylbenzoyl]-1-[[[1-(phenylmethyl)-3-piperidinyl]amino]acetyl]piperazine, diastereomers;
2-(3,4-dichlorophenyl)-4-(3,5-dimethylbenzoyl)-1-[[[8-(phenylmethyl)-8-azabicyclo[3.2.1]oct-3-yl]amino]acetyl]piperazine;
2-(3,4-dichlorophenyl)-4-(3,5-dimethylbenzoyl)-1-[[[8-methyl-8-azabicyclo]3.2.1]oct-3-yl]amino]acetyl]piperazine;
2-(3,4-dichlorophenyl)-4-[3,5-dimethylbenzoyl]-1-[[[1,3-bis(phenylmethyl)-4-piperidinyl]amino]acetyl]piperazine, diastereomers;
2-(3,4-dichlorophenyl)-4-[3,5-dimethylbenzoyl]-1-[[[3-methyl-1-(phenylmethyl)-4-piperidinyl]amino]acetyl]piperazine;
2-(3,4-dichlorophenyl)-4-[3,5-dimethylbenzoyl]-1-[[[1-(phenylmethyl)-3-(2-propenyl)-4-piperidinyl]amino]acetyl]piperazine;
trichloroethyl-4-[[[-(3,4-dichlorophenyl)-4-(3,5-dimethylbenzoyl)-1-piperazinyl]-2-oxoethyl]amino]-2-phenyl-1-piperidinecarboxylate;
2-(3,4-dichlorophenyl)-4-(3,5-dimethylbenzoyl)-1-[3-[[5-(phenylmethyl)-(1S,4S)-2,5-diazabicyclo[2.2.1]heptane-2-yl]-1-oxopropyl]piperazine;
(-)-phenyl-4-[[2-[2-(3,4-dichlorophenyl)-4-(3,5-dimethylbenzoyl)-1-piperazinyl-2-oxoethyl]amino]-1-piperidinecarboxylate, hemihydrate;
2-(3,4-dichlorophenyl)-4-(3,5-dimethylbenzoyl)-1-[[[1-[(1H-pyrrol-2-yl)methyl]-4-piperidinyl]amino]acetyl]piperazine, hemihydrate;
2-(3,4-dichlorophenyl)-4-(3,5-dimethylbenzoyl)-1-[[[1-[(1H-pyrrol-2-yl)carbonyl]-4-piperidinyl]amino]acetyl]piperazine, hemihydrate
(-)-2-(3,4-dichlorophenyl)-4-(3,5-dimethylbenzoyl)-1-[[[1-[(1H-imidazol-2-yl)methyl]-4-piperidinyl]amino]acetyl]piperazine, dihydrate;
2-(3,4-dichlorophenyl)-4-(3,5-dimethylbenzoyl)-1-[1-oxo-3-[[1-(phenylmethyl)-4-piperidinyl]amino]propyl]piperazine;
2-(3,4-dichlorophenyl)-4-(3,5-dimethylbenzoyl)-1-[1-oxo-3-N-methyl-[[1-(phenylmethyl)-4-piperidinyl]amino]propyl]piperazine;
(-)-1,2-dimethylethyl-5-[3-[2-(3,4-dichlorophenyl)-4-(3,5-dimethylbenzoyl)-1-piperazinyl]-3-oxopropyl]-( 1S,4S)-2,5-diazabicyclo[2.2.1]heptane-2-carboxylate;
(-)-1-[3-(1S,4S)-(2,5-diazabicyclo[2.2.1]heptan-2-yl)-1-oxopropyl]-2-(3,4-dichlorophenyl)-4-(3,5-dimethylbenzoyl) piperazine dihydrochloride;
(-)-N-[4-[[5-[3-[2-(3,4-dichlorophenyl-4-(3,5-dimethylbenzoyl]-1-piperazinyl]-3-oxopropyl]-(1S,4S)-2,5-diazabicyclo[2.2.1]heptan-2-yl]methyl-2-thiazolyl]acetamide;
(-)-N-[4-[[5-[3-[2-(3,4-dichlorophenyl-4-(3,5-dimethylbenzoyl)-1-piperazinyl]-3-oxopropyl]-(1S,4S)-2,5-diazabicyclo[2.2.1]heptan-2-yl]methyl]phenyl]acetamide;
2-(3,4-dichlorophenyl-4-(3,5-dimethylbenzoyl]-1-[3-[5-(1-H-pyrroll-2-yl)methyl]-(1S,4S)-2,5-diazabicyclo[2.2.1]heptan-2-yl]-1-oxopropyl]piperazine;
(+,-)-2-(3,4-dichlorophenyl)-4-[(4-fluoro-1-naphthalenyl)carbonyl]-1-[[[1-(phenylmethyl)-4-piperidinyl]amino]acetyl]piperazine;
(+/-)-1-[[3,5-bis(trifluoromethyl)phenyl]methyl]-3-phenyl-piperazine, dihydrochloride salt, quarter hydrate;
(+,-)-4-[[3,5-bis(trifluoromethyl)phenyl]methyl]-2-phenyl-1-[[[1-(phenylmethyl)-4-piperidinyl]amino]acetyl]piperazine, trihydrochloride salt, dihydrate;
(+,-)-4-[[3,5-bis(trifluoromethyl)phenyl]methyl]-2-phenyl-N-[1-(phenylmethyl)-4-piperidinyl]-1-piperazineethanamine, tetrahydrochloride salt, monohydrate;
2-(3,4-dichlorophenyl)piperazine;
(+,-)-4-[[3,5-bis(trifluoromethyl)phenyl]methyl]-2-(3,4-dichlorophenyl)-1-[[[1-(phenylmethyl)-4-piperidinyl]amino]acety]]-piperazine;
(+,-)-4-[[3,5-bis(trifluoromethyl)phenyl]acetyl]-2-phenyl-1-[[[1-(phenylmethyl)-4-piperidinyl]amino]acetyl]piperazine;
(+,-)-4-[2-[3,5-bis(trifluoromethyl)phenyl]ethyl]-2-phenyl-N-[1-(phenylmethyl)-4-piperidinyl]-1-piperazineethanamine, four hydrochloride salt, hemihydrate;
(+,-)-2-phenyl-1-[[[(1-phenylmethyl)-4-piperidinyl]amino]acetyl]-4-[(3,4,5-trimethoxylphenyl)acetyl]piperazine, hemihydrate;
(+/-)-4-[2-[3,5-bis(trifluoromethyl)phenyl)ethyl]-2-phenyl-1-[[[1-(phenylmethyl)-4-piperidinyl]amino]acetyl]piperazine, trihydrochloride salt;
(+,-)-2-phenyl-1-[[[(1-phenylmethyl)-4-piperidinyl]amino]acetyl]-4-[2-(3,4,5-trimethoxyphenyl)ethyl]piperazine;
(+,-)-[3,5-bis(trifluoromethyl)benzoyl]-3-(3,4-dichlorophenyl)piperazine;
(+,-)-4-[3,5-bis(trifluoromethyl)benzoyl]-2-(3,4-dichlorophenyl)-1-[[[1-(phenylmethyl)-4-piperidinyl]amino]acetyl]piperazine;
(+)-4-[3,5-bis(trifluoromethyl)benzoyl]-2-(3,4-dichlorophenyl)-1-[[[1-(phenylmethyl)-4-piperidinyl]amino]acetyl]piperazine dihydrochloride dihydrate;
(-)-4-[3,5-bis(trifluoromethyl)benzoyl]-2-(3,4-dichlorophenyl)-1-[[[1-(phenylmethyl)-4-piperidinyl]amino]acetyl]piperazine dihydrochloride dihydrate;
(+,-)-2-(3,4-dichlorophenyl)-1-[[[1-(phenylmethyl)-4-piperidinyl]amino]acetyl]-4-(3,4,5-trimethoxybenzoyl)piperazine;
(+,-)-2-(3,4-dichlorophenyl)-4-[3-(1-methylethoxy)benzoyl]-1-[[[l-(phenylmethyl)-4-piperidinyl]amino]acetyl]piperazine, hemihydrate;
(+,-)-2-(3,4-dichlorophenyl)-4-[2-methoxybenzoyl]-1-[([1-(phenylmethyl)-4-piperidinylamino]acetyl]piperazine;
(+,-)-4-[3,5-bis(trifluoromethyl)benzoyl]-2-(3,4-dichlorophenyl)-1-[[5-(phenylmethyl)-2,5-diazabicyclo[2.2.1]heptan-2-yl]acetyl]piperazine;
(+,-)-4-[[3,5-bis(trifluoromethyl)phenyl]acetyl]-2-phenyl-1-[[[I-1-(phenylmethyl)-4-piperidinyl]amino]acetyl]piperazine.
(+,-)-2-phenyl-1-[[[(1-phenylmethyl)-4-piperidinyl]aminolacetyl]-4-[(3,4,5-trimethoxylphenyl)acetyl]piperazine, hemihydrate;
(+,-)-4-[3,5-bis(trifluoromethyl)benzoyl]-2-phenyl-1-[[[1-(phenylmethyl)-4-piperidinyl]amino]acetyl]piperazine;
(+,-)-4-[3,5-dimethylbenzoyl]-2-(3,4-dichlorophenyl)-1-[[[1-(phenylmethyl)-4-piperidinyl]amino]acetyl]piperazine;
(+,-)-2-(3,4-dichlorophenyl)-4-(3,5-dimethylbenzoyl)-1-(([1-(2-furanylmethyl)-4-piperidinyl)amino]acetyl]]piperazine;
(+,-)-1-[[[1-[[[1,1'-biphenyl]-4-yl]methyl]-4-piperidinyl]amino]acetyl]-2-(3,4-dichlorophenyl)-4-(3,5-dimethylbenzoyl)piperazine;
(+,-)-2-(3,4-dichlorophenyl)-4-[(4-fluoro-1-5-naphthalenyl)carbonyl]-1-[[[1-(phenylmethyl)-4-piperidinyl]amino]acetyl]piperazine;
(+,-)-4-(3,5-dimethylbenzoyl)-1-[[[1-(phenylmethyl)-4-piperidinyl]amino]acetyl]-2-[4-(trifluoromethyl)phenyl]piperazine,1.2 hydrate;
(+,-)-4-[3,5-bis(trifluoromethyl)benzoyl]-2-(3-hydroxyphenyl)-1-[[[1-(phenylmethyl)-4-piperidinyl]amino]acetyl]piperazine, dimaleate, hemihydrate;
(+,-)-4-[3,5-bis(trifluoromethyl)benzoyl]-2-(4-hydroxyphenyl)-1-[[[1-(phenylmethyl)-4-piperidinyl]amino]acetyl]piperazine, dimaleate, hemihydrate;
2-(R,S)-(3,4-dichlorophenyl)-4-(3,5-dimethylbenzoyl)-1-[3-hydroxy-1-oxo-2(S)-[[1-(phenylmethyl)-4-piperidinyl]amino]propyl]piperazine, demihydrate;
2-(R,S)-(3,4-dichlorophenyl)-4-(3,5-dimethylbenzoyl)-1-[4-methyl-1-oxo-2(R,S)-[[1-(phenylmethyl)-4-piperidinyl]amino]pentyl]piperazine;
(+,-)-N-[2-[2-(3,4-dichlorophenyl)-4-(3,5-dimethylbenzoyl)-1-(piperazinyl]-2-oxo-ethyl]-N-[1-phenylmethyl)-4-piperidinyl]acetamide, demihydrate;
(+,-)-2-(3,4-dichlorophenyl)-4-(3,5-dimethylbenzoyl)-1-[[[2-hydroxyethyl][1-(phenylmethyl)-4-piperidinyl]amino]acetyl]piperazine, hemihydrate;
(+,-)-N-[2-[2-(3,4-dichlorophenyl)-4-(3,5-dimethylbenzoyl)-1-piperazinyl]-2-oxoethyl]-N,N-dimethylamino-1-(phenylmethyl)-4-piperidinaminium bromide, dimethanolate;
(+,-)-2-(3,4-dichlorophenyl)-4-(3,5-dimethylbenzoyl)-1-[[methyl[1-(phenylmethyl)-4-piperidinyl]amino]acetyl]piperazine;
(+,-)-2-(3,4-dichlorophenyl)-4-(3,5-dimethylbenzoyl)-2-methyl-1-[[[1-(phenylmethyl)-4-piperidinyl]amino]acetyl]piperazine, 0.6 methanol;
(+,-)-2-(3,4-dichlorophenyl)-4-(4-fluoro-1-naphthalenylcarbonyl)-2-methyl-1-[[[1-(phenylmethyl)-4-piperidinyl]amino]acetyl]piperazine;
(+,-)-1-[3,5-bis(trifluoromethyl)benzoyl]-2-(3,4-dichlorophenyl)-4-[[(1-(phenylmethyl)-4-piperidinyl]amino]acetyl]piperazine, dimaleate, monohydrate;
(+,-)-2-(3,4-dichlorophenyl)-4-(3,5-dimethylbenzoyl)-1-[[[1-[1-oxo-2-phenyl)ethyl]4-piperidinyl]amino]acetyl]piperazine;
(+,-)-1-dimethylethyl-4-[[2-[2-(3,4-dichlorophenyl)-1-(,3,5-dimethylbenzoyl)-1-piperazinyl]-2-oxoethyl]amino]-1-piperidinecarboxylate;
(+,-)-2-(3,4-dichlorophenyl)-4-[3,5-dimethylbenzoyl]-1-[(4-piperidinylamino) acetyl]piperazine, dihydrochloride;
(-)-2-(3,4-dichlorophenyl)-4-[3,5-dimethylbenzoyl]-1-[(4-piperidinyl-amino) acetyl]piperazine, dihydrochloride;
(+,-)-1-benzoyl-4-[[2-[2-(3,4-dichlorophenyl)-4-(3,5-dimethylbenzoyl)-1-piperazinyl]-2-oxoethyl]amino]piperidine;
(+,-)-2-(3,4-dichlorophenyl)-4-(3,5-dimethylbenzoyl)-1-[[[1-(2-oxo-2-phenylethyl)-4-piperidinyl]amino]acetyl]piperazine;
(+,-)-2-(3,4-dichlorophenyl)-4-(3,5-dimethylbenzoyl)-1-[[[1-(3-phenylpropyl)-4-piperidinyl]amino]acetyl]piperazine;
2-(3,4-dichlorophenyl)-4-[3,5-dimethylbenzoyl]-1-[[[3-methyl-1-(phenylmethyl)-4-piperidinyl]amino]acetyl]piperazine diastereomers;
2-(3,4-dichlorophenyl)-4-[3,5-dimethylbenzoyl]-1-[[[1-(phenylmethyl)-3-pip eridinyl]amino]acetyl]piperazine;
(+,-)-[3,5-dimethylbenzoyl]-3-(3,4-dichlorophenyl)piperazine;
(+,-)-bromoacetyl-2-(3,4-dichlorophenyl)-4-(3,5-dimethylbenzoyl)piperazine;
(+)-[3,5-dimethylbenzoyl]-3-(3,4-dichlorophenyl)piperazine (Enantiomer);
2-(3,4-Dichlorophenyl)-4-(3,5-dimethylbenzoyl)-1-[3-[1,2-dihydro-1-(methanesulfonyl)spiro[3H-indole-3,4'-piperidine]-1'-yl]-1-oxopropyl]-piperazine (Enantiomer);
2-(3,4-Dichlorophenyl)-1-[3-[3,4-dihydro-4-oxo-6-methoxy-spiro[2H-1-benzopyran-2,4'-piperidin]-1'-yl]- -oxopropyl]-4-(3,5-dimethylbenzoyl)piperazine (Enantiomer);
2-(3,4-Dichlorophenyl)-4-(3,5-dimethylbenzoyl)-1-[3-(1-phenyl-4-oxo-1,3,8-triazaspiro[4.5]decan-8-yl)-1-oxopropyl]piperazine (Enantiomer);
(+,-)-1-[2-[4-[[3,5-bis(trifluoromethyl)phenyl]methyl]-2-(3,4-dichlorophenyl)-1-piperazinyl]ethyl]-4-phenyl-4-piperidinol;
(+,-)-[3,5-bis(trifluoromethyl)benzoyl]-3-(3,4-dichlorophenyl)piperazine;
(+,-)-4-[3,5-bis(trifluoromethyl)benzoyl]-2-(3,4-dichlorophenyl)-1-[[(4-hydroxy-4-phenyl-1-piperidinyl)]acetyl]piperazine;
(+,-)-2-(3,4-dichlorophenyl)-4-(2-methoxybenzoyl)-1-[[(4-hydroxy-4-phenyl-1-piperidinyl)]acetyl]piperazine;
(+,-)-4-[3,5-bis(trifluoromethyl)benzoyl]-2-phenyl-1-[[(4-hydroxy-4-phenyl-1-piperidinyl)]acetyl]piperazine;
(+,-)-[3,5-dimethylbenzoyl]-3-(3,4-dichlorophenyl)piperazine;
(+)-[3,5-dimethylbenzoyl]-3-(3,4-dichlorophenyl)piperazine;
4-[3,5-bis(trifluoromethyl)benzoyl]-2-(3,4-dichlorophenyl)-1[1,2-dioxo-2-[4-(phenylmethyl)-1-piperazinyl]ethyl]piperazine;
1-[3,5-dimethylbenzoyi]-3-(3,4-dichlorophenyl)piperazine (enantiomer);
2-(3,4-dichlorophenyl)-4-(3,5-dimethylbenzoyl)-1-[[(4-carbethoycyclohexyl) amino]acetyl]piperazine (enantiomer);
2-(3,4-dichlorophenyl)-4-(3,5-dimethylbenzoyl)-1-[[(3-methylcyclohexyl) amino)acetyl]piperazine (diasteromers);
1-[(cyclohexylamino)acetyl]-2-(3,4-dichlorophenyl)-4-(3,5-dimethylbenzoyl) piperazine (enantiomer);
1-[(cycloheptylamino)acetyl]-2-(3,4-dichlorophenyl)-4-(3,5-dimethylbenzoyl) piperazine (enantiomer);
1-[[(4-cyano-4-phenylcyclohexyl)amino]acetyl-2-(3,4-dichlorophenyl)-4-(3,5-dimethylbenzoyl)piperazine (enantiomer);
(+/-)-2-(3,4-dichlorophenyl)-4-(3,5-dimethylbenzoyl)-1-[[(4-phenylcyclohexyl] amino]acetyl]-piperazine;
2-(3,4-dichlorophenyl)-4-(3,5-dimethylbenzoyl)-1-[3-[4-(2-keto-1-benzimidazolinyl)piperidinyl]-1-oxopropyl]piperazine (enantiomer);
2-(3,4-dichlorophenyl)-4-(3,5-dimethylbenzoyl)-1-[3-[4-(phenylmethyl)-1-piperidinyl]-1-oxopropyl]piperazine (enantiomer);
2-(3,4-dichlorophenyl)-4-(3,5-dimethylbenzoyl)-1-[1-oxo-3-[4-(phenylmethyl)-1-piperidinyl]propyl]piperazine (enantiomer);
2-(3,4-dichlorophenyl)-4-(3,5-dimethylbenzoyl)-1-[3-[4-hydroxy-4-phenyl-1-piperidinyl]-1-oxopropyl]piperazine (enantiomer);
(+,-)-[3,5-dimethylbenzoyl]-3-(3,4-dichlorophenyl)piperazine;
(+,-)-bromoacetyl-2-(3,4-dichlorophenyl)-4-(3,5-dimethylbenzoyl)piperazine;
(+)-[3,5-dimethylbenzoyl]-3(R)-(3,4-dichlorophenyl)piperazine;
(-)-bromoacetyl-2(R)-(3,4-dichlorophenyl)-4-(3,5-dimethylbenzoyl)piperazine (Enantiomer);
1,1-Dimethylethyl-5-[[2(R)-[2-(3,4-dichlorophenyl)4-(3,5-dimethylbenzoyl)-1-piperazinyl]-2-oxoethyllamino]-2-azabicyclo[2.2.1]heptane-2-carboxylate, diastereomers;
2(R)-(3,4-Dichlorophenyl)-4-(3,5-dimethylbenzoyl)-1-[[[2-azabicyclo[2.2.1]heptan-5-yl]amino]acetyl]piperazine, diastereomers;
2(R)-(3,4-Dichlorophenyl)-4-(3,5-dimethylbenzoyl)-1-[[[2-(phenylmethyl)-azabicyclo[2.2.1]heptan-5-yl]amino]acetyl]piperazine, diastereomers;
2(R)-(3,4-Dichlorophenyl)-4-(3,5-dimethylbenzoyl)-1-[[[2-(phenylmethyl)-azabicyclo[2.2.1]heptan-5-yl]amino]acetyl]piperazine, diastereomers;
2(R)-(3,4-Dichlorophenyl)-4-(3,5-dimethylbenzoyl)-1-[[[2-(phenylmethyl)-azabicyclo[2.2.1]heptan-5-yl]amino]acetyl]piperazine, enantiomers;
2(R)-(3,4-Dichlorophenyl)-4-(3,5-dimethylbenzoyl)-1-[[[2-(phenylmethyl)-azabicyclo[2.2.1]heptan-5-yl]amino]acetyl]piperazine, enantiomers;
2(R)-(3,4-Dichlorophenyl)-4-(3,5-dimethylbenzoyl)-1-[[[2-(4-acetyaminophenylmethyl)-azabicyclo[2.2.1]heptan-5-yl]amino]acetyl]piperazine;
N-[4-[[5-[[2-[2(R)-(3,4-Dichlorophenyl)-4-(3,5-dimethylbenzoyl)-1-piperazinyl]-2-oxoethyl]amino]-2-azabicyclo[2.2.1]heptan-2-yl]methyl-2-thiazolyl]acetamide (diastereomers);
(-)-1,1-Dimethylethyl-2-[3-[2(R)-(3,4-dichlorophenyl)-4-(dimethylbenzoyl)-1-piperazinyl]-3-oxopropyl]-1(S),4(S)-2,5-diazabicyclo[2.2.1]heptane-5-carboxylate;
(-)-1-[3-[(1S),4(S)-2,5-Diazabicyclo[2.2.1]heptan-2-yl)-1-oxopropyl]-2(R)-(3,4-dichlorophenyl)-4-(3,5-dimethylbenzoyl)piperazine;
1,1-Dimethylethyl-[2-[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]-2-oxo-1(R)-phenyl ethyl] carbamate;
2-[(R)-Amino(phenyl)acetyl]-5-[3-[2(R)-(3,4-dichlorophenyl)-4-(3,5-dimethylbenzoyl)-1-piperazinyl)-3-oxopropyl]-1(S),4(S)-2,5-diazabicyclo[2.2.1]heptane, dihydrochloride (Enantiomer);
(-)-1,1-Dimethylethyl-4-[[2-[2(R)-(3,4-dichlorophenyl)-1-(3,5-dimethylbenzoyl)-1-piperazinyl]-2-oxoethyl]amino]-1-piperidinecarboxylate (Enantiomer);
(+,-)-2-(3,4-Dichlorophenyl)-4-[3,5-dimethylbenzoyl]-1-[(4-piperidinlyamino)acetyl]piperazine, dihydrochloride;
(-)-2(R)-(3,4-Dichlorophenyl)-4-[3,5-dimethylbenzoyl]-1-[(4-piperidinyl-amino) acetyl]piperazine, dihydrochloride (Enantiomer);
2(R)-(3,4-Dichlorophenyl)-4-(3,5-dimethylbenzoyl)-1-[1-oxo-3-[5-phenylsulfonyl)-2,5-diazabicyclo[2.2.1]heptan-2-yl]propyl]piperazine;
5-[1-Cyanoimino)-1-methylthio]-2-[3-[2(R)-(3,4-dichlorophenyl)-4-(3,5-dimethylbenzoyl)-1-piperazinyl]-3-oxopropyl-2,5-diazabicyclo[2.2.1]heptane;
5-[1-(cyanoimino)-1-phenylaminomethyl]-2-[3-[2(R)-(3,4-dichloro-phenyl)-4-(3,5-dimethylbenzoyl)-1-piperazinyl]-3-oxopropyl-2,5-diazabicyclo[2.2.1]heptane;
5-[1-(Cyanoimino)-1-phenylmethylaminomethyl]-2-[3-[2(R)-(3,4-dichlorophenyl)-4-(3,5-dimethylbenzoyl)-1-piperazinyl]-3-oxopropyl-2,5-diazabicyclo[2.2.1]heptane;
2(R)-(3,4-dichlorophenyl)-4-(3,5-dimethylbenzoyl)-1-[[6-(phenylmethyl)-2-azabicyclo[2.2.2]octan-6-yl]methylamino]acetyl]piperazine;
2(R)-(3,4-Dichlorophenyl)-4-(3,5-dimethylbenzoyl)-1-[[6-(phenylmethyl)-6-azabicyclo[3.2.2.]nonan-3-yl]amino]acetyl]piperazine;
Methyl [1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenyl]carbamate (enantiomer);
N-[1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenylethyl]-N'-methylurea (enantiomer);
5-[3-[2(R)-(3,4-Dichlorophenyl)-4-(3,5-dimethylbenzoyl)-1-piperazinyl]-3-oxopropyl]-2-[2(R)-[[(methylamino)carbonyl]amino]-1-oxo-3-(2-thienyl)propyl]-1(S),4(S)-2,5-diazabicyclo[2.2.1]heptane (enantiomer);
2-[3-[2-(R)-(3,4-Dichlorophenyl)-4-(3,5-dimethylbenzyoyl)-1-piperazinyl]-3-oxopropyl]-5-[2-[[imino(methylamino)methyl]amino]-1-oxo-3-phenylpropyl]-1(S),4(S)-2,5-diazobicyclo[2.2.1]heptane;
5-[3-[2(R)-(3,4-dichlorophenyl)-4-(3,5-dimethylbenzoyl)-1-piperazinyl]-3-oxopropyl]-2-(2(R)-hydroxy-1-oxo-3-phenylpropyl)-1(S),4(S)-2,5-diazabicyclo-[2.2.1]heptane (Enantiomer);
5-[3-[2(R)-(3,4-dichlorophenyl)-4-(3,5-dimethylbenzoyl)-1-piperazinyl]-3-oxopropyl]-2-(2(S)-hydroxy-1-oxo-3-phenylpropyl)-1(S),4(S)-2,5-diazabicyclo-[2.2.1]heptane (Enantiomer);
2-[2(S)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1heptane (Enantiomer);
2-[2(R)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1]heptane (Enantiomer);
2-[2(R)-2-(Aminohydroxyimino)ethoyl]-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
[1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate (enantiomer);
[1(S)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate (enantiomer);
2-[2(S)-Methoxy-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1]heptane (Enantiomer);
(1R,4R) -1,1-Dimethylethyl-5-hydroxy-2-azabicyclo[2.2.1]heptane-2-carboxylate and
(1S,4S)-1,1-dimethylethyl5-hydroxy-2-azabicyclo [2.2.1]heptane-2-carboxylate;
(Exo)-1,1-dimethylethyl-5-[2-[2(R)-(3,4-dichlorophenyl)-4-(3,5-dimethylbenzoyl)-1-piperazinyl]-2-oxoethoxy]-1-(R),4(R)-2-azabicyclo [2.2.1]heptane-2-carboxylate (enantiomer);
2(R)-(3,4-Dichlorophenyl)-4-(3,5-dimethylbenzoyl)-1-[[[exo-1(R),4(R)-2-azabicyclo [2.2.1]heptan-5-yl]oxy]acetyl]piperazine (enantiomer) hydrochloride salt;
2(R)-(3,4-Dichlorophenyl)-4-(3,5-dimethylbenzoyl)-1-[[[exo-2-(3-thienylmethyl)-1(S),4(S)-2-azabicyclo[2.2.1]heptan-5-yl]oxy]acetyl]piperazine (enantiomer);
1,1-Dimethylethyl-3-exo-hydroxy-8-azabicyclo[3.2.1]octane-8-carboxylate and
1,1-dimethylethyl3-endo-hydroxy-8-azabicyclo [3.2.1]octane-8-carboxylate;
(Exo)-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[exo-8-aza-3.2.1]1]octan-3-yl]oxy]acetyl]piperazine (enantiomer) hydrochloride salt;
(Endo)-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[endo-8-aza-3.2.1]octan-3-yl]oxy]acetyl]piperazine (enantiomer) hydrochloride salt;
N-1(R)-[[5-[3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylsulfonamide (enantiomer);
2-[2(R)-(Cyanomethylamino)-1-oxo-3-phenylpropyl]-5-[3-[2-(R)-(3,4-dichlorophenyl)-4-(3,5-dimethylbenzoyl)-1-piperazinyl]-3-oxopropyl]-1(S),4(S)-2,5-di azabicyclo[2.2.1]heptane;
1,1-Dimethylethyl[1(R)-[[endo-5-[[2-[2(R)-(3,4-dichlorophenyl)-4-(3,5-dimethylbenzoyl)-1-piperazinyl]-2-oxoethyl]amino]-2-azabicyclo[2.2.1]-heptan-2-yl]carbonyl]-2-phen ylethyl]carbamate racemic mixture;
Endo-2-(2(R)-amino-1-oxo-3-phenylpropyl)-5-[[2-[2(R)-(3,4-dichlorophenyl)-4-(3,5-dimethylbenzoyl)-1-piperazinyl]-2-oxoethyl]amino]-2-aza[2.2.1]heptane, dihydrochloride (enantiomer);
1,1-Dimethylethyl-3-[(phenylmethyl)amino]-8-azabicyclo[3.2.1]octane-8-carboxylate (exo and endo products);
1,1-Dimethylethyl-3-[[2-[2-(R)-(3,4-dichlorophenyl)-2-oxoethyl]endo-amino]-8-azabicyclo[3.2. 1]octane-8-carboxylate;
2-(R)-(3,4-Dichlorophenyl)-4-(3,5-dimethylbenzoyl)-1-[[[8-azabicyclo-[3.2.1]octan-3-yl]endo-amino]acetyl]piperazine hydrochloride;
1,1-Dimethylethyl-3-[[2-[2-(R)-(3,4-dichlorophenyl)-2-oxoethyl]exo-amino]-8-azabicyclo[3.2.1]octane-8-carboxylate;
2-(R)-(3,4-Dichlorophenyl)-4-(3,5-dimethylbenzoyl)-1-[[[8-azabicyclo-[3.2.1]octan-3-yl]exo-amino]acetyl]piperazine hydrochloride;
Exo-8-(2(S)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
1,1-Dimethylethyl-[1(S)-[[endo-3-[[2-2(R)-(3,4-dichlorophenyl)-4-(3,5-dimethylbenzoyl)-1-piperazinyl]-2-oxoethyl]amino]-8-aza[3.2.1]octan-8-yl]carbonyl]-2-phenylethyl]carbamate (enantiomer);
Endo-8-(2(S)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
1,1-Dimethylethyl-[1(R)-[[exo-3-[[2-2(R)-(3,4-dichlorophenyl)4-(3,5-dimethylbenzoyl)-1-piperazinyl]-2-oxoethyl]amino]-8-aza[3.2.1]octan-8-yl]carbonyl]-2-phenylethyl]carbamate (enantiomer);
Exo-8-(2(R)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
(+,-)-N-[4-[[Endo-5-[[2-[2(R)-(3,4-dichlorophenyl)-4-(3,5-dimethylbenzoyl)-1-piperazinyl]-2-oxoethyl]amino]-2-azabicyclo[2.2.1]heptan-2-yl]methyl]-phenyl]acetamide;
(+,-)-N-[3-[[Endo-5-[[2-[2(R)-(3,4-dichlorophenyl)-4-(3,5-dimethyl-benzoyl)-1-piperazinyl]-2-oxoethyl]amino]-2-azabicyclo[2.2.1]heptan-2-yl]methyl]-phenyl]acetamide ;
(+,-)-1,1-Dimethylethyltrans-2-[[5-[3-[2(R)-(3,4-dichlorophenyl)-4-(3,5-dimethylbenzoyl)-1-piperazinyl]-3-oxopropyl)-1(S),4(S)-2,5-diaza-bicyclo-[2.2.1]heptan-2-yl]carbonyl]-3-phenyl-1-azetidinecarboxylate (enantiomer);
(+,-)-5-[3-[2(R)-(3,4-Dichlorophenyl)-4-(3,5-dimethylbenzoyl)-1-piperazinyl]-3-oxopropyl]-2-[(trans-3-phenyl-2-azetidinyl)carbonyl]-1(S),4(S)-2,5-diazabicyclo[2.2.1]heptane, dihydrochloride(enantiomer);

Examples of NK-1 receptor antagonists that may be used in the methods and pharmaceutical compositions of this invention are compounds of the formula 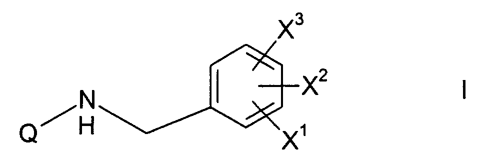
and their pharmaceutically acceptable salts, wherein X¹ is hydrogen, (C₁-C₁₀) alkoxy optionally substituted with from one to three flourine atoms or (C₁-C₁₀) alkyl optionally substituted with from one to three fluorine atoms;
X² and X³ are independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆) alkyl-C(=O)-NH-(C₁-C₆) alkyl, hydroxy(C₁-C₄)alkyl, (C₁-C₄)alkoxy(C₁-C₄)alkyl, -NHC(=O)H and -NHC(=O)-(C₁-C₆) alkyl; and
Q is a group of the formula 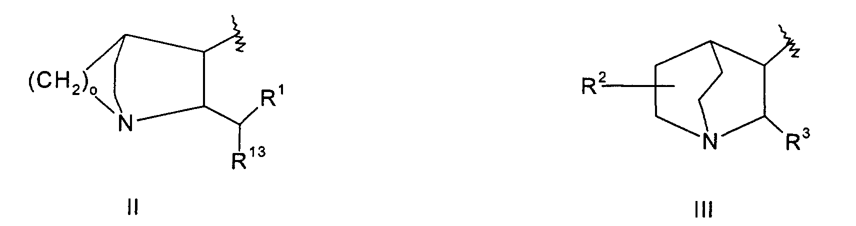 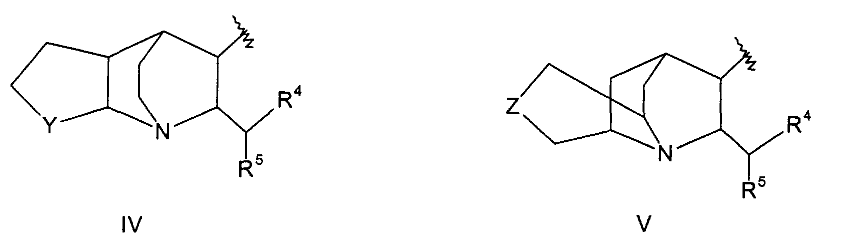 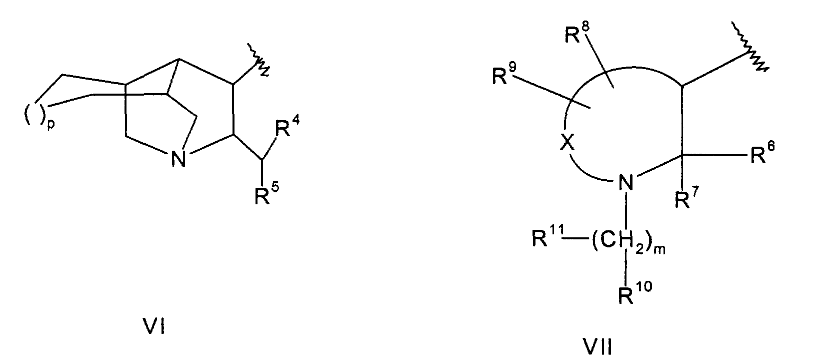 OR 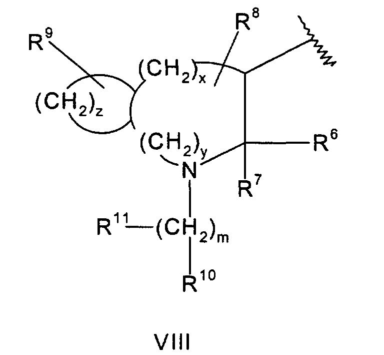
wherein R¹ is a radical selected from furyl, thienyl, pyridyl, indolyl, biphenyl and phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R¹³ is selected from (C₃-C₄) branched alkyl, (C₅-C₆) branched alkenyl, (C₅-C₇) cycloalkyl, and the radicals named in the definition of R¹;
R² is hydrogen or (C₁-C₆) alkyl;
R³ is phenyl, biphenyl, naphthyl, pyridyl, benzhydryl, thienyl or furyl, and R³ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂), wherein I is an integer from one to three, or Y is a group of the formula 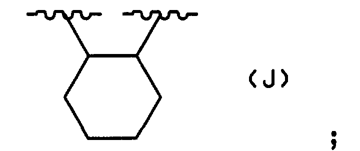
Z is oxygen, sulfur, amino, (C₁-C₃)alkylamino or (CH₂)ₙ wherein n is zero, one or two;
o is two or three;
p is zero or one;
R⁴ is furyl, thienyl, pyridyl, indolyl, biphenyl, or phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, (C₁-C₃) alkoxy-carbonyl and benzyloxycarbonyl;
R⁵ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
R⁶ is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-(C₁-C₆)alkyl-, di-(C₁-C₆)alkylamino, -C(=O)NH-(C₁-C₆)alkyl,(C₁-C₆)-alkyl-C(=O)-NH-(C₁-C₆)alkyl,-NHC(=O)H and -NHC(=O)-(C₁-C₆) alkyl; and wherein one of the phenyl moieties of said benzhydryl may optionally be replaced by naphthyl, thienyl, furyl or pyridyl;
R⁷ is hydrogen, phenyl or (C₁-C₆)alkyl;
or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
R⁸ and R⁹ are each independently selected from hydrogen, hydroxy, halo, amino, oxo (=O), nitrile, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, (C₁-C₆)alkyl-O-C(=O)-, (C₁-C₆)alkyl-O-C(=O)-(C₁-C₆)alkyl,-(C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-(C₁-C₆)alkyl-, and the radicals set forth in the definition of R⁶;
R¹⁰ is NHCR¹², NHCH₂R¹², NHSO₂R¹² or one of the radicals set forth in any of the definitions of R⁶, R⁸ and R⁹;
R¹¹ is oximino (=NOH) or one of the radicals set forth in any of the definitions of R⁶, R⁸ and R⁹; and
R¹² is (C₁-C₆)alkyl, hydrogen, phenyl(C₁-C₆)alkyl or phenyl optionally substituted with (C₁-C₆) alkyl; and
with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, and (d) when R⁸ and R⁹ are attached to the same carbon atom, then either each of R⁸ and R⁹ is independently selected from hydrogen, fluoro, (C₁-C₆) alkyl, hydroxy-(C₁-C₆)alkyl and (C₁-C₆)alkoxy-(C₁-C₆)alkyl, or R⁸ and R⁹, together with the carbon to which they are attached, form a (C₃-C₆) saturated carbocyclic ring that forms a spiro compound with the nitrogen-containing ring to which they are attached.

Other examples of NK-1 receptor antagonists that can be used in the methods and pharmaceutical compositions of this invention are compounds of the formula I, as defined above, with the further proviso that when neither X¹, X² nor X³ is a fluorinated alkoxy group, at least one of R¹, R³, R⁴, R⁵, R⁶, R⁷ and R¹³ is an aryl group substituted with a fluorinated alkoxy group. Such compounds are hereinafter referred to as "compounds of the formula la".

Other examples of NK-1 receptor antagonists that can be used in the methods and pharmaceutical compositions of this invention are compounds of the formula 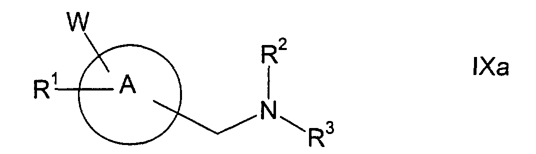 or 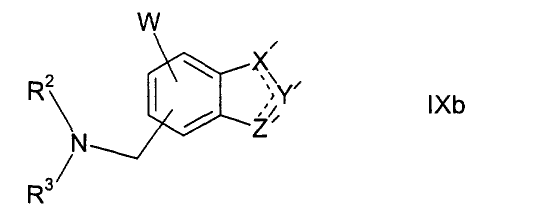 and their pharmaceutically acceptable salts, wherein A is a ring system selected from phenyl, naphthyl, thienyl, quinolinyl and indolinyl, and wherein the side chain containing NR²R³ is attached to a carbon atom of ring system A;
W is hydrogen, (C₁-C₆)alkyl optionally substituted with from one to three fluorine atoms,-S(O)ᵥ-(C₁-C₆) alkyl wherein v is zero, one or two, halo, benzyloxy or (C₁-C₆)alkoxy optionally substituted with from one to three fluorine atoms;
R¹ is a 4, 5 or 6 membered heterocyclic ring containing from one to three heteroatoms selected from oxygen, nitrogen and sulfur (e.g., thiazolyl, azetidinyl, pyrrolyl, pyrazolyl, 1,2,3-triazolyl, 1,2,4-triazolyl, isothiazolyl, imidazolyl, isoxazolyl, oxazolyl, pyridyl, pyrimidinyl, pyrazolyl or thiophenyl), wherein said heterocyclic ring may contain from zero to three double bonds and may optionally be substituted with one or more substituents, preferably one or two substituents, independently selected from (C₁-C₆) alkyl optionally substituted with from one to three fluorine atoms and (C₁-C₆) alkoxy optionally substituted with from one to three fluorine atoms;
the dotted lines in formula lb indicate that one of the X'-Y' and Y'-Z' bonds may optionally be a double bond;
X' is selected from =CH-, -CH₂-, -O-, -S-, -SO-, -SO₂-, -N(R⁴)-, -NH-, =N-, -CH[(C₁-C₆)alkyl]-, =C[(C₁-C₆)alkyl]-, -CH(C₆H₅)- and =C(C₆H₅)-;
Y' is selected from C=O, C=NR⁴, C=S, =CH-, -CH₂-, =C[(C₁-C₆)alkyl]-, -CH[(C₁-C₆)alkyl]-, =C(C₆H₅)-, -CH(C₆H₅)-, =N-, -NH-, -N(R⁴)-, =C(halo)-, =C(OR⁴)-, =C(SR⁴)-, =C(NR⁴)-, -O-, =C(CF₃)-, =C(CH₂C₆H₅)-, -S- and SO₂, wherein the phenyl moieties of said =C(C₆H₅)- and -CH(C₆H₅)- may optionally be substituted with from one to three substituents independently selected from trifluoromethyl and halo, and wherein the alkyl moieties of said =[(C₁-C₆)alkyl]- and -CH[C₁-C₆)alkyl]- may optionally be substituted with from one to three fluorine atoms;
Z' is selected from =CH-, -CH₂-, =N-, -NH-, -S-, -N(R⁴)-, =C(C₆H₅)-, -CH(C₆H₅)-, =C[(C₁-C₆) alkyl]- and -CH[(C₁-C₆)alkyl]-;
or X', Y' and Z', together with the two carbon atoms shared between the benzo ring and the X'Y'Z' ring, form a fused pyridine or pyrimidine ring;
R² is hydrogen or -CO₂(C₁-C₁₀)alkyl;
R³ is selected from 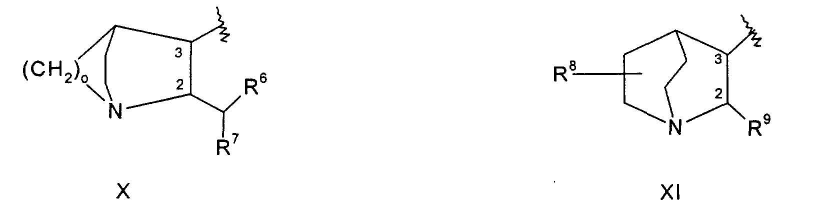 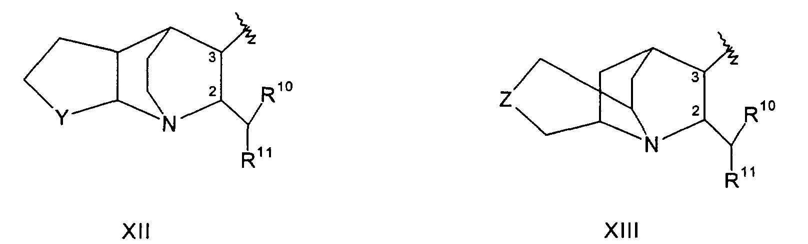 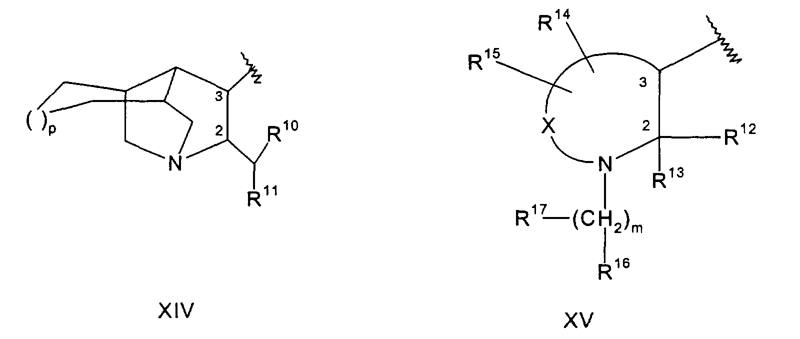 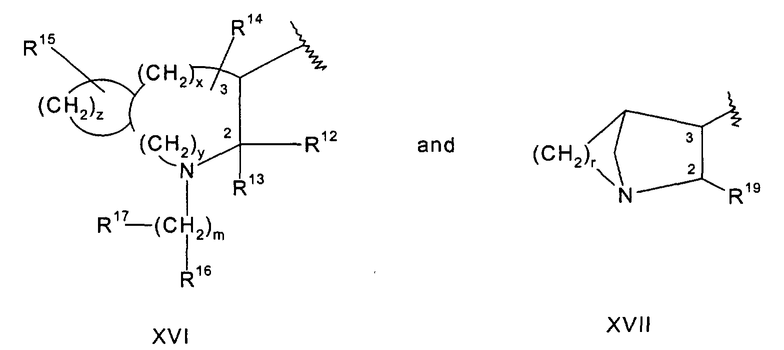
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R⁴ is (C₁-C₆) alkyl or phenyl;
R⁷ is selected from (C₃-C₄) branched alkyl, (C₅-C₆) branched alkenyl, (C₅-C₇) cycloalkyl, and the radicals named in the definition of R⁶;
R⁸ is hydrogen or (C₁-C₆) alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein I is an integer from one to three, or Y is a group of the formula 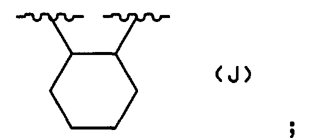
Z is oxygen, sulfur, amino, (C₁-C₃)alkylamino or (CH₂)ₙ wherein n is zero, one or two;
x is zero, one or two;
y is zero, one or two;
z is three, four or five;
o is two or three;
p is zero or one;
r is one, two or three;
the ring containing (CH₂)_{z} may contain from zero to three double bonds, and one of the carbon atoms of (CH₂)_{z} may optionally be replaced by oxygen, sulfur or nitrogen;
R¹¹ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆) alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl-(C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-, (C₁-C₆)alkyl-, di-(C₁-C₆)alkylamino, -C(=O)-NH-(C₁-C₆)alkyl, (C₁-C₆)-alkyl-C(=O)-NH-(C₁-C₆)alkyl,-NHC(=O)H and -NHC(=O)-(C₁-C₆)alkyl; and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to such point of attachment may optionally be replaced by oxygen, nitrogen or sulfur;
R¹⁴ and R¹⁵ are each independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, -C(=O)-OH, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C-(C₁-C₆)alkyl-O-, (C₁-C₆)alkyl-C(=O)-, (C₁-C₆)alkyl-C(=O)-(C₁-C₆)alkyl-, and the radicals set forth in the definition of R¹²;
R¹⁶ is NHC(=O)R¹⁸, NHCH₂R¹⁸, SO₂R¹⁸, CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl (C₁-C₆)alkyl;
with the proviso that (a) when m is 0, one of R¹⁶ and R¹⁷ is absent and the other is hydrogen, (b) when R³ is a group of the formula XVI, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy-(C₁-C₆)alkyl and (C₁-C₆)alkoxy-(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ can not both be hydrogen, and (e) when R¹⁴ or R¹⁵ is attached to a carbon atom of X or (CH₂)_{y} that is adjacent to the ring nitrogen, then R¹⁴ or R¹⁵, respectively, must be a substituent wherein the point of attachment is a carbon atom.

The fused bicyclic nucleus of compounds of the formula IXb to which W and the -CH₂NR²R³ sidechain are attached may be, but is not limited to, one of the following groups: benzoxazolyl, benzthiazolyl, benzimidazolyl, benzisoxazolyl, benzoisothiazolyl, indazolyl, indolyl, isoquinolinyl, benzofuryl, benzothienyl, oxindolyl, benzoxazolinonyl, benzthiazolinonyl, benzimidazolinonyl, benzimidazoliniminyl, dihydrobenzothienyl-S ,S-dioxide, benztriazolyl, benzthiadiazolyl, benzoxadiazolyl, and quinazolinyl.

Examples of acids that can be used to prepare pharmaceutically acceptable acid addition salts of basic NK-1 antagonists for use in this invention are those which form non-toxic acid addition salts, *i.e*., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i.e.*, 1,1□-methylene-bis-(2-hydroxy-3-naphthoate)]salts. The chemical bases that can be used as reagents to prepare the pharmaceutically acceptable base salts of acidic NK-1 antagonists and acidic compounds exhibiting antidepressant or anxiolytic properties for use in this invention are those which form non-toxic base salts with such compounds. Such non-toxic base salts include those derived from such pharmacologically acceptable cations as sodium, potassium calcium and magnesium, etc.

Other examples of NK-1 receptor antagonists that can be used in the method and pharmaceutical compositions of this invention are compounds of the formula 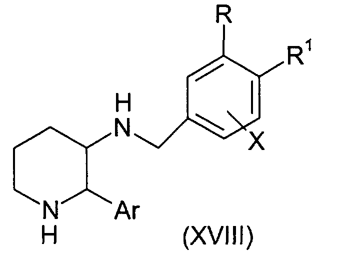 and their pharmaceutically acceptable salts, wherein
R is halo (C₁-C₈)alkyl, halo (C₂-C₈)alkenyl, halo (C₂-C₈)alkynyl or halo (C₁-C₈)alkyl substituted by hydroxy or (C₁-C₈)alkoxy; R¹ is hydrogen, halo or (C₁-C₆)alkoxy; or
R and R¹, together with the two carbon atoms shared between the benzene ring and the R and R¹, complete a fused (C₄-C₆)cycloalkyl wherein one carbon atom is optionally replaced by oxygen and wherein one or two of the carbon atoms are optionally substituted by up to five subtituents selected from halo, (C₁-C₆)alkyl and halo (C₁-C₆)alkyl;
X is (C₁-C₆)alkoxy, halo (C₁-C₆)alkoxy, phenoxy or halo; and
Ar is phenyl optionally substituents by halo.

Other examples of NK-1 receptor antagonists that can be used in the methods and pharmaceutical compositions of this invention are compounds of the formula 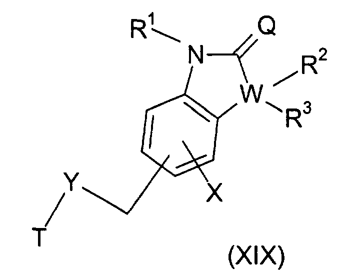 and their pharmaceutically acceptable salts, wherein
W is methylene, ethylene, propylene, vinylene, -CH₂-O-,-O-CH₂-, -CH₂-S- or -S-CH₂-;
R¹, R² and R³ are independently hydrogen, (C₁-C₃) alkyl, (C₁-C₃) alkoxy or halo (C₁-C₃) alkyl, provided that when W is methylene, both R² and R³ are not hydrogen;
X is halo, (C₁-C₃) alkoxy, (C₁-C₃) alkoxy or (C₁-C₃) alkenyl;
Y is imino or oxy;
Q is oxygen or sulfur; and
T is (2S,3S)-2-diphenylmethylquinuclidin-3-yl,(2S,3S)-2-phenylpiperdin-3-yl or (2S,3S)-2-diphenylmethyl-1-azanorbornan-3-yl.

Other examples of NK-1 antagonists that can be used in the pharmaceutical compositions and methods of this invention are the following compounds and their pharmaceutically acceptable salts: 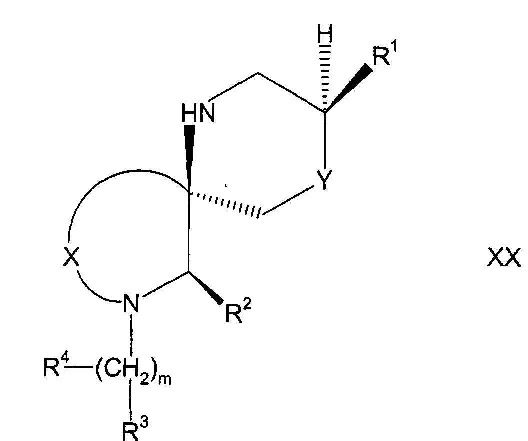 wherein R¹ is phenyl optionally substituted with one or more substituents, preferably with from one to three substituents, independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆)alkyl-C(=O)-NH-(C₁-C₆) alkyl, hydroxy(C₁-C₄)alkyl,-NHC(=O)H, -NHC(=O)-(C₁-C₆) alkyl, (C₁-C₄)alkoxy(C₁-C₄)alkyl, -S(O)ᵥ-(C₁-C₁₀)-alkyl wherein v is zero, one or two, -S(O)ᵥ-aryl wherein v is zero, one or two, -O-aryl,-SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, (SO₂-(C₁-C₁₀)alkyl) ((C₁-C₁₀)alkyl)N wherein one or both of the alkyl moieties may optionally be substituted with from one to three fluorine atoms, -N(SO₂-(C₁-C₁₀)alkyl)₂ and (SO₂-aryl) ((C₁-C₁₀)alkyl)N; and wherein the aryl moieties of said -S(O)ᵥ-aryl, -O-aryl and (SO₂-aryl) ((C₁-C₁₀)alkyl)N are independently selected from phenyl and benzyl and may optionally be substituted with from one to three substituents independently selected from (C₁-C₄)alkyl, (C₁-C₄)alkoxy and halo;
or R¹ is phenyl substituted with a group having the formula 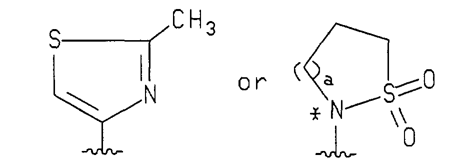 wherein a is 0, 1 or 2 and the asterisk represents a position meta to the point of attachment of R¹;
R² is selected from (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents, preferably with from one to three substituents, independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)-(C₁-C₆)alkyl, (C₁-C₆)alkyl-C(=O)-O-, (C₁-C₆)alkyl-C-(C₁-C₆)alkyl-O-, (C₁-C₆)alkyl-C(=O)-, (C₁-C₆)alkyl-C-(C₁-C₆)alkyl-, di-(C₁-C₆)alkylamino, -C(=O)NH-(C₁-C₆)alkyl, (C₁-C₆)-alkyl-C(=O)-NH-(C₁-C₆)alkyl, -NHC(=O)H and -NHC(=O)-(C₁-C₆) alkyl; and wherein one of the phenyl moieties of said benzhydryl may optionally be replaced by naphthyl, thienyl, furyl or pyridyl;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
R³ is selected from NHC(=O)R⁸, NHCH₂R⁸, SO₂R⁸, AR⁵, CO₂H and the radicals set forth in the definitions of R², R⁶ and R⁷;
A is CH₂, nitrogen, oxygen, sulfur or carbonyl;
R⁸ is (C₁-C₆)alkyl, hydrogen, phenyl or phenyl (C₁-C₆)alkyl;
R⁴ is selected from oximino (=NOH) and the radicals set forth in the definitions of R², R⁶ and R⁷;
R⁵ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 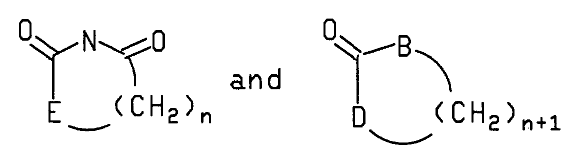 wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆) spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
R⁶ and R⁷ are independently selected from hydrogen, hydroxy, halo, amino, oxo (=O), cyano, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, -C(=O)-OH, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C-, (C₁-C₆)alkyl-C(=O)-(C₁-C₆)alkyl- and the radicals set forth in the definition of R²; and
Y is (CH₂)_{z} wherein z is zero or one;
with the proviso that: (a) when A is -(CH₂)- or carbonyl, R⁵ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; (c) when R⁶ or R⁷ is attached to a carbon atom of X that is adjacent to the ring nitrogen, then R⁶ or R⁷, respectively, must be a substituent wherein the point of attachment is a carbon atom;

Other examples of NK-1 receptor antagonists that can be used in the methods and pharmaceutical compositions of this invention include the following compounds and their pharmaceutically acceptable salts: 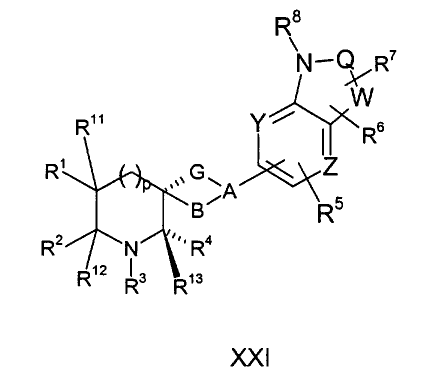 wherein
Q is C=NH, C=CH₂, C=S, C=O, SO or SO₂;
A is CH, CH₂, C(C₁-C₆)alkyl, CH(C₁-C₆)alkyl, C(CF₃) or CH(CF₃), with the proviso that when B is present, A must be either CH, C(C₁-C₆)alkyl or C(CF₃);
B is absent or is methylene or ethylene;
each of Y and Z is N or CH, with the proviso that Y and Z can not both be N;
G is NH(CH₂)_{q}, S(CH₂)_{q} or O(CH₂)_{q}, wherein q is zero or one;
W is a one carbon linking group (i.e., methylene) or a saturated or unsaturated two or three carbon linking group, wherein each of the foregoing W groups can optionally be substituted with one substituent R⁷ or two substituents R⁷ and R⁶, or W is a one carbon linking group that forms, together with a 2, 3, 4 or 5 carbon chain, a 3, 4, 5 or 6 membered spiro ring, respectively;
or W is a saturated two carbon chain linking group that forms, together with a separate 1, 2 or 3 carbon chain, a fused 3, 4 or 5 membered ring, respectively;
or W is a saturated two carbon chain linking group, wherein one of the two carbons in the chain forms, together with a separate 2, 3, 4 or 5 carbon chain, a 3, 4, 5 or 6 membered spiro ring, respectively;
p is zero, one or two;
R³ is selected from hydrogen, COR⁹, CO₂R⁹, optionally substituted phenyl, optionally substituted heterocyclic rings, and optionally substituted (C₁-C₈)alkyl wherein one of the CH₂ groups of said (C₁-C₈) alkyl may optionally be replaced with a sulfur, oxygen or carbonyl group and wherein said (C₁-C₈)alkyl can optionally be substituted with from one to three substituents, preferably with zero substituents or one substituent, independently selected from hydroxy, oxo, phenyl-(C₁-C₃)alkoxy, phenyl, cyano, halo, optionally substituted heterocyclic rings, NR⁹COR¹⁰, NR⁹CO₂R¹⁰, CONR⁹R¹⁰, COR⁹, CO₂R⁹, NR⁹R¹⁰, and (C₁-C₆)alkoxy optionally substituted with from one to seven fluorine atoms, preferably with from zero to three fluorine atoms;
and wherein the heterocyclic rings of R³ and the heterocyclic ring substituents on the alkyl groups of R³ are selected, independently, from 3 to 7 membered saturated or unsaturated monocyclic rings containing from 1 to 4 ring heteroatoms, and 8 to 12 membered saturated or unsaturated bicyclic rings containing from 1 to 4 ring heteroatoms, wherein said heteroatoms are selected, independently, from oxygen, nitrogen and sulfur, with the proviso that there can not be two adjacent ring oxygen atoms or two adjacent ring sulfur atoms in either the monocyclic or bicyclic heterocyclic rings, and with the proviso that heterocyclic rings formed from NR⁹R¹⁰ or CONR⁹R¹⁰ must contain at least one nitrogen atom;
and wherein the heterocyclic rings of R³ and the heterocyclic ring substituents on the alkyl groups of R³ can optionally be substituted with one or more substituents, preferably with zero, one or two substituents, independently selected from oxo, hydroxy, thioxo, halo, cyano, phenyl, (CH₂)ₘNR⁹R¹⁰, NR⁹COR¹⁰, (CH₂)ₘOR⁹, wherein m is zero, one or two, and (C₁-C₆)alkyl optionally substituted with one or more substituents, preferably with from zero to two substituents, independently selected from halo, CF₃, methoxy and phenyl;
and wherein the phenyl groups of R³ and the phenyl substituents in the alkyl groups of R³ can optionally be substituted with one or more substitutents, preferably with from zero to two substituents, independently selected from the group consisting of halo, cyano, nitro, CF₃, (CH₂)ₘNR⁹R¹⁰, wherein m is zero, one or two, NR⁹COR¹⁰, NR⁹CO₂R¹⁰, CONR⁹R¹⁰, CO₂NR⁹R¹⁰, COR⁹, CO₂R⁹, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆)alkenyl optionally substituted with from one to seven fluorine atoms, preferably with from zero to three fluorine atoms;
each of R¹, R², R¹¹, R¹² and R¹³ are selected, independently, from hydrogen and (C₁-C₆)alkyl optionally substituted with one or more substituents, preferably with zero, one or two substituents, that are selected, independently, from hydroxy, oxo, (C₁-C₆)alkoxy and cyano;
or R¹ and R², together with the carbon atoms to which they are attached, or R² and R³, together with the carbon and nitrogen to which they are attached, respectively, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms; or R¹ and R², together with the carbons to which they are attached, form a 5 or 6 membered, saturated or unsaturated carbocyclic ring, and wherein said heterocyclic and carbocyclic rings formed by R¹ and R² or by R² and R³ can be substituted with one or more substituents, preferably with zero substituents or one substituent, independently selected from halo, oxo, NR⁹R¹⁰, (C₁-C₆)alkyl optionally substituted with from one to seven fluorine atoms, preferably with from zero to three fluorine atoms, and (C₁-C₆)alkoxy optionally substituted with from one to seven fluorine atoms, preferably with from zero to three fluorine atoms;
or R¹² and R¹³, together with the carbon atoms to which they are attached, form a 5 or 6 membered saturated heterocyclic ring containing one or two heteroatoms that are selected, independently, from nitrogen, oxygen and sulfur, with the proviso that said ring can not contain two adjacent oxygen atoms or two adjacent sulfur atoms, or R¹² and R¹³, together with the carbons to which they are attached, form a 5 or 6 membered, saturated or unsaturated carbocyclic ring, and wherein said heterocyclic and carbocyclic rings formed by R¹² and R¹³ can be substituted with one or more substituents, preferably with zero substituents or one substituent, independently selected from NR⁹R¹⁰, halo, phenyl-S-, phenyl-SO-, phenyl-SO₂-,oxo, (C₁₋C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms:

with the proviso that no more than one of R¹ and R², R² and R³, and R¹² and R¹³ can form a ring;
R⁴ is selected from phenyl, 2-, 3- or 4-pyridyl, 2- or 3-thienyl, and pyrimidyl, wherein R⁴ can be optionally substituted with one or more substituents, preferably with zero or one substituent, selected, independently, from halo, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆) alkenyl optionally substituted with from one to seven fluorine atoms, preferably with from zero to three fluorine atoms;
R⁵ and R⁸ are selected, independently, from hydrogen, -SO(C₁-C₆)alkyl, -SO₂-(C₁-C₆)alkyl, -SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected, independently, from -SO(C₁-C₆)alkyl, -SO₂-(C₁-C ₆)alkyl,-SO-aryl, -SO₂-aryl, CF₃, halo, phenyl, phenyl-(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected, independently, from hydroxy, oxo, (C₁-C₆)alkoxy, phenyl-(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
each R⁹ and each R¹⁰ is selected, independently, from hydrogen, (C₁-C₆)alkyl, hydroxy(C₁-C₆)alkyl, phenyl and CF₃;
or R⁹ and R¹⁰, when R³ is NR⁹R¹⁰or CONR⁹R¹⁰, can form, together with the nitrogen to which they are attached, an optionally substituted heterocyclic ring that contains at least one nitrogen atom;
and wherein the phenyl groups in the definition of R⁵, R⁶, R⁷ and R⁸ and the phenyl moiety of phenyl (C₁-C₂)alkyl in the definition of R⁵, R⁶, R⁷ and R⁸ can optionally be substituted with one or more substituents, preferably with from zero to two substituents, that are selected, independently, from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms;
with the proviso that: (a) R⁸ can not be halo, hydroxy, cyano, aryloxy, heteroaryloxy, substituted or unsubstituted (C₁-C₆)alkoxy or methyl substituted with from 1-3 fluorine atoms; and (b) when Q is C=O or C=S, and Y and Z are both carbon, and W is a methylene, ethylene or propylene group that is optionally substituted with (C₁-C₆)alkyl or fluoro substituted (C₁-C₆)alkyl, and all of R¹, R², R¹¹, R¹² and R¹³ are hydrogen, and R⁵, R⁶, R⁷, and R⁸ are selected from hydrogen, halo, (C₁-C₆) alkyl optionally substituted with from 1 to 7 fluorine atoms, (C₁-C₆) alkoxy optionally substituted with from 1 to 7 fluorine atoms, then R³ can not be hydrogen;
and the pharmaceutically acceptable salts of such compounds.

Examples of the optionally substituted heterocyclic rings of R³ and the optionally substituted heterocyclic ring substitutents on the alkyl groups of R³ are the following: pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, and thienyl, and groups of the formulas 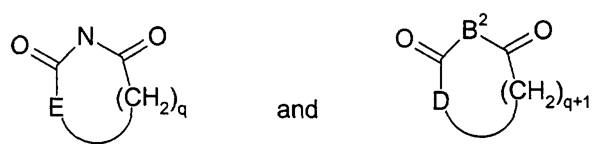 wherein B² and D are selected from carbon, oxygen and nitrogen, and at least one of B² and D is other than carbon; E is carbon or nitrogen; q is an integer from 1 to 5; any one of the carbon atoms of said (CH₂)_{q} and (CH₂)_{q+1} may be optionally substituted with (C₁-C₆)alkyl or (C₁-C₆) spiroalkyl; and either any one pair of the carbon atoms of said (CH₂)_{q} and (CH₂)_{q+1} may be bridged by a one or two carbon atom linkage, or any one pair of adjacent carbon atoms of said (CH₂)_{q} and (CH₂)_{q+1} may form, together with from one to three carbon atoms that are not members of the carbonyl containing ring, a (C₃-C₅) fused carbocyclic ring.

Compounds of formula XXI may contain chiral centers and therefore may exist in different enantiomeric and diastereomeric forms. This invention relates to all optical isomers and all stereoisomers of compounds of the formula XXI, both as racemic mixtures and as individual enantiomers and diastereoismers of such compounds, and mixtures thereof, and to all pharmaceutical compositions and methods of treatment defined above that contain or employ them, respectively.

As the compounds of formula XXI possess at least two asymmetric centers, they are capable of occurring in various stereoisomeric forms or configurations. Hence, the compounds can exist in separated (+)- and (-)-optically active forms, as well as mixtures thereof. The present invention includes pharmaceutical composition comprising and method of treatment employing all such forms within its scope. Individual isomers can be obtained by known methods, such as optical resolution, optically selective reaction, or chromatographic separation in the preparation of the final product or its intermediate.

In so far as the compounds of formula XXI of this invention are basic compounds, they are all capable of forming a wide variety of different salts with various inorganic and organic acids. Although such salts must be pharmaceutically acceptable for administration to animals, it is often desirable in practice to initially isolate the base compound from the reaction mixture as a pharmaceutically unacceptable salt and then simply convert to the free base compound by treatment with an alkaline reagent and thereafter convert the free base to a pharmaceutically acceptable acid addition salt. The acid addition salts of the base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained. The acids which are used to prepare the pharmaceutically acceptable acid addition salts of the aforementioned base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, p-toluenesulfonate and pamoate (i.e., 1,1'-methylene-bis-(2-hydroxy-3-naphthoate))salts.

Individual enantiomers of the compounds of formula XXI may have advantages, as compared with the racemic mixtures of these compounds, in the treatment of various disorders or conditions. For example, the compounds prepared from the 2S-phenyl-piperidin-3S-ylamino template are preferred.

More specific embodiments of the invention include compounds of the formula XXI are those wherein B is absent and A is CH₂.

Other more specific embodiments of compounds of the formula XXI are those wherein Q is a carbonyl group.

Other more specific embodiments of compounds of the formula XXI are those wherein Y and Z are both CH.

Other more specific embodiments of compounds of the formula XXI are those wherein B is ethylene, A is CH and G is NHCH₂.

Other more specific embodiments of compounds of the formula XXI are those wherein B is ethylene, A is CH and G is SCH₂.

Other more specific embodiments of compounds of the formula XXI are those wherein R³ is hydrogen.

Other more specific embodiments of compounds of the formula XXI are those wherein B is ethylene, A is CH and G is NHCH₂.

Other more specific embodiments of compounds of the formula XXI are those wherein R³ is CO₂R⁹.

Other more specific embodiments of compounds of the formula XXI are those wherein B is absent, G is NH and A is CH₂.

Other more specific embodiments of compounds of the formula XXI are those wherein W is ethylene.

Other more specific embodiments of compounds of the formula XXI are those wherein R⁴ is phenyl.

Other more specific embodiments of compounds of the formula XXI are those wherein R⁴ is phenyl and R⁸ is hydrogen.

Other more specific embodiments of compounds of the formula XXI are those wherein R⁴ is phenyl and R⁸ is methyl.

Other more specific embodiments of compounds of the formula XXI are those wherein p is one.

Other more specific embodiments of compounds of the formula XXI are those wherein R² is (C₁-C₆)alkyl.

Other more specific embodiments of compounds of the formula XXI are those wherein R² is (C₁-C₆)alkyl wherein the stereochemical configuration at the chiral carbon to which R² is attached is "S".

Other more specific embodiments of compounds of the formula XXI are those wherein R⁴ is 2-, 3- or 4-pyridyl.

Other more specific embodiments of compounds of the formula XXI are those wherein R² and R¹² are selected, independently, from hydrogen, methyl, ethyl and propyl.

Other more specific embodiments of compounds of the formula XXI are those wherein both R² and R¹² are other than hydrogen.

Other more specific embodiments of compounds of the formula XXI are those wherein Y is CH.

Other more specific embodiments of compounds of the formula XXI are those wherein Y is CH and Z is CH.

Other more specific embodiments of compounds of the formula XXI are those wherein Y is CH and Z is nitrogen.

Other more specific embodiments of compounds of the formula XXI are those wherein Q is C=O and W is methylene optionally substituted with one or two substituents independently selected from (C₁-C₆)alkyl and CF₃.

Other more specific embodiments of compounds of the formula XXI are those wherein Q is C=O and W is ethylene optionally substituted with one or two substituents independently selected from (C₁-C₆)alkyl and CF₃.

Other more specific embodiments of compounds of the formula XXI are those wherein Q is SO.

Other more specific embodiments of compounds of the formula XXI are those wherein Q is SO₂.

Other more specific embodiments of compounds of the formula XXI are those wherein Y is nitrogen and Z is CH.

Other more specific embodiments of compounds of the formula XXI are those wherein Q is C=S.

Other more specific embodiments of compounds of the formula XXI are those wherein R⁸ is hydrogen.

Other more specific embodiments of compounds of the formula XXI are those wherein R⁸ is methyl.

Other more specific embodiments of compounds of the formula XXI are those wherein R³ is a heterocyclic ring.

Other more specific embodiments of compounds of the formula XXI are those wherein R³ is an alkyl group substituted with a heterocyclic ring.

Other more specific embodiments of the invention include compounds of the formula XXI wherein R³ is an alkyl group substituted with a heterocyclic ring selected from imidazolyl, 5-oxo-4,5-dihydro-1H-[1,2,4]triazol-3-yl, benzoxazol-2-yl, and 5-oxo-pyrrolidin-2-yl.

Other more specific embodiments of compounds of the formula XXI are those wherein R⁸ is a cycloalkyl group.

Other more specific embodiments of compounds of the formula XXI are those wherein R⁸ is a cyclopropyl group.

Preferred compounds of the invention include compounds of the formula XXI wherein R⁴ is optionally substituted pyridyl.

Other preferred compounds of compounds of the formula XXI wherein R² and R¹² are selected from (C₁-C₃)alkyl.

Examples of preferred compounds of this invention are the isomers of the following compounds that have the sterochemistry depicted in structural formula XXI:
7-[(1-Dimethylaminoacetyl-2-phenyl-piperidin-3-ylamino)-methyl]-6-methoxy-1-methyl-3,4-dihydro-1H-quinolin-2-one;
6-Methoxy-1-methyl-7-{[2-phenyl-1-(pyridin-2-yl-acetyl)-piperidin-3-ylamino]-methyl}-3,4-dihydro-1H-quinolin-2-one;
6-Methoxy-1-methyl-7-{[2-phenyl-1-(pyridin-3-yl-acetyl)-piperidin-3-ylamino]-methyl}-3,4-dihydro-1H-quinolin-2-one;
6-Methoxy-1-methyl-7-{[2-phenyl-1-(pyridin-4-yl-acetyl)-piperidin-3-ylamino]-methyl}-3,4-dihydro-1H-quinolin-2-one;
6-Cyclopropoxy-1-methyl-7-[(2-phenyl-piperidin-3-ylamino)-methyl]-3,4-dihydro-1H-quinolin-2-one;
(5-Chloro-2-methoxy-benzyl)-(2-phenyl-octahydro-cyclopenta[b]pyrrol-3-yl)-amine;
6-Methoxy-1-methyl-7-[(1-[1,2,4]oxadiazol-3-ylmethyl-2-phenyl-piperidin-3-ylamino)-methyl]-3,4-dihydro-1H-quinolin-2-one;
7-{[1-(Imidazol-1-yl-acetyl)-2-phenyl-piperidin-3-ylamino]-methyl}-6-methoxy-1-methyl-3,4-dihydro-1H-quinolin-2-one;
1-[3-(2-Methoxy-5-trifluoromethoxy-benzylamino)-2-phenyl-piperidin-1-yl]-2-pyridin-2-yl-ethanone;
1-[3-(2-Methoxy-5-trifluoromethoxy-benzylamino)-2-phenyl- piperidin-1-yl]-2-pyridin-3-yl-ethanone;
1-[3-(2-Methoxy-5-trifluoromethoxy-benzylamino)-2-phenyl-piperidin-1-yl]-2-pyridin-4-yl-ethanone;
2-lmidazol-1-yl-1-[3-(2-methoxy-5-trifluoromethoxy-benzylamino)-2-phenyl-piperidin-1-yl]-ethanone;
2-Dimethylamino-1-[3-(2-methoxy-5-trifluoromethoxy-benzylamino)-2-phenyl-piperidin-1-yl]-ethanone
3-(2-Benzyloxy-5-trifluoromethoxy-phenyl)-6-phenyl-1-oxa-7-aza-spiro[4.5]decane;
1-[3-(2-Methoxy-5-trifluoromethoxy-benzylamino)-2-phenyl-piperidin-1-yl]-2-pyrrolidin-1-yl-ethanone;
(2-Methoxy-5-trifluoromethoxy-benzyl)-(1-[1,2,4]oxadiazol-3-ylmethyl-2-phenyl-piperidin-3-yl)-amine;
7-{[2-(4-Fluoro-phenyl)-piperidin-3-ylamino]-methyl}-6-methoxy-1- methyl-3,4-dihydro-1H-quinolin-2-one;
[1-(2-lmidazol-1-yl-ethyl)-2-phenyl-piperidin-3-yl]-(2-methoxy-5-trifluoromethoxy-benzyl)-amine;
7-{[1-(2-Dimethylamino-ethyl)-2-phenyl-piperidin-3-ylamino]-methyl}-6-methoxy-1-methyl-3,4-dihydro-1H-quinolin-2-one;
(5-Chloro-2-ethoxy-pyridin-3-ylmethyl)-(2-phenyl-piperidin-3-yl)-amine;
(5-Chloro-2-methoxy-pyridin-3-ylmethyl)-(2-phenyl-piperidin-3-yl)-amine;
Dibenzofuran-2-ylmethyl-(2-phenyl-piperidin-3-yl)-amine;
[3-(lndan-2-yloxy)-4-methoxy-benzyl]-(2-phenyl-piperidin-3-yl)-amine;
6-[(2-Phenyl-piperidin-3-ylamino)-methyl]-chroman-4-one;
(5-Methyl-benzo[b]thiophen-3-ylmethyl)-(2-phenyl-piperidin-3-yl)-amine;
(2,2-Dimethyl-chroman-6-ylmethyl)-(2-phenyl-piperidin-3-yl)-amine;
(1H-Benzoimidazol-5-ylmethyl)-(2-phenyl-piperidin-3-yl)-amine;
1-{2-[(2-Phenyl-piperidin-3-ylamino)-methyl]-phenyl}-pyrrolidin-2-one;
(2-Phenyl-piperidin-3-yl)-[3-(pyridin-2-yloxy)-benzyl]-amine
[3-(4-Methoxy-phenoxy)-benzyl]-(2-phenyl-piperidin-3-yl)-amine;
(4-Phenoxy-benzyl)-(2-phenyl-piperidin-3-yl)-amine;
(2-Phenyl-piperidin-3-yl)-thiophen-2-ylmethyl-amine;
Furan-2-ylmethyl-(2-phenyl-piperidin-3-yl)-amine;
(5-Methyl-furan-2-ylmethyl)-(2-phenyl-piperidin-3-yl)-amine;
(3-Methyl-thiophen-2-ylmethyl)-(2-phenyl-piperidin-3-yl)-amine;
(2-Phenyl-piperidin-3-yl)-thiophen-3-ylmethyl-amine;
(3-Methyl-benzo[b]thiophen-2-ylmethyl)-(2-phenyl-piperidin-3-yl)-amine;
Benzofuran-2-ylmethyl-(2-phenyl-piperidin-3-yl)-amine;
(5-Ethyl-furan-2-ylmethyl)-(2-phenyl-piperidin-3-yl)-amine;
(5-Chloro-3-methyl-1-phenyl-1H-pyrazol-4-ylmethyl)-(2-phenyl-piperidin-3-yl)-amine;
6-Methoxy-7-{[1-(2-methoxy-ethyl)-2-phenyl-piperidin-3-ylamino]-methyl}-1-methyl-3,4-dihydro-1H-quinolin-2-one;
(5-Methyl-3-phenyl-isoxazol-4-ylmethyl)-(2-phenyl-piperidin-3-yl)-amine;
(3-Phenoxy-benzyl)-(2-phenyl-piperidin-3-yl)-amine;
Furan-3-ylmethyl-(2-phenyl-piperidin-3-yl)-amine;
(3,5-Dimethyl-1-phenyl-1H-pyrazol-4-ylmethyl)-(2-phenyl-piperidin-3-yl)-amine;
(5,7-Dimethoxy-1H-indol-4-ylmethyl)-(2-phenyl-piperidin-3-yl)-amine;
(5-Methoxy-1H-indol-3-ylmethyl)-(2-phenyl-piperidin-3-yl)-amine;
(4-Oxy-quinoxalin-2-ylmethyl)-(2-phenyl-piperidin-3-yl)-amine;
(2-Phenyl-piperidin-3-yl)-quinoxalin-2-ylmethyl-amine;
7-{[1-(2,3-Dihydroxy-propyl)-2-phenyl-piperidin-3-ylamino]-methyl}-6-methoxy-1-methyl-3,4-dihydro-1H-quinolin-2-one;
(2-Methoxy-5-trifluoromethoxy-benzyl)-[2-phenyl-1-(2-pyrrolidin-1-yl-ethyl)-piperidin-3-yl]-amine;
6-Ethoxy-1-methyl-7-[(2-phenyl-piperidin-3-ylamino)-methyl]-3,4-dihydro-1H-quinolin-2-one;
[1-(2-Dimethylamino-ethyl)-2-phenyl-piperidin-3-yl]-(2-methoxy-5-trifluoromethoxy-benzyl)-amine;
3-(2-Cyclopropoxy-5-trifluoromethoxy-phenyl)-6-phenyl-1-oxa-7-aza-spiro[4.5]decane;
[1-(2-Methoxy-ethyl)-2-phenyl-piperidin-3-yl]-(2-methoxy-5-trifluoromethoxy-benzyl)-amine;
6-Hydroxy-1-methyl-7-[(2-phenyl-piperidin-3-ylamino)-methyl]-3,4-dihydro-1H-quinolin-2-one;
6-Methoxy-1-methyl-7-[(2-phenyl-octahydro-cyclopenta[b]pyrrol-3-ylamino)-methyl]-3,4-dihydro-1H-quinolin-2-one;
7-{[2-(4-Fluoro-phenyl)-piperidin-3-ylamino]-methyl}-6-methoxy-3,4-dihydro-1H-quinolin-2-one;
6-Methoxy-1-methyl-7-(6-phenyl-1-oxa-7-aza-spiro[4.5]dec-3-yl)-3,4-dihydro-1H-quinolin-2-one;
6-Methoxy-1,3,3-trimethyl-5-[(2-phenyl-octahydro-cyclopenta[b]pyrrol-3-ylamino)-methyl]-1,3-dihydro-indol-2-one;
[3-Chloro-2-(4-fluoro-phenoxy)-pyridin-4-ylmethyl]-(2-phenyl-piperidin-3-yl)-amine;
6-Ethoxy-1,3,3-trimethyl-5-[(2-phenyl-piperidin-3-ylamino)-methyl]-1,3-dihydro-indol-2-one;
6-Ethoxy-1,3,3-trimethyl-5-[(2-phenyl-octahydro-cyclopenta[b]pyrrol-3-ylamino)-methyl]-1,3-dihydro-indol-2-one;
6-lsopropoxy-1,3,3-trimethyl-5-[(2-phenyl-piperidin-3-ylamino)-methyl]-1,3-dihydro-indol-2-one;
6-lsopropoxy-1,3,3-trimethyl-5-[(2-phenyl-octahydro-cyclopenta[b]pyrrol-3-ylamino)-methyl]-1,3-dihydro-indol-2-one;
6-Ethoxy-1,3,3-trimethyl-5-[(2-phenyl-octahydro-cyclopenta[b]pyrrol-3-ylamino)-methyl]-1,3-dihydro-indol-2-one;
6-lsopropoxy-1,3,3-trimethyl-5-[(2-phenyl-octahydro-cyclopenta[b]pyrrol-3-ylamino)-methyl]-1,3-dihydro-indol-2-one;
7-lsopropoxy-1-methyl-6-[(2-phenyl-piperidin-3-ylamino)-methyl]-3,4-dihydro-1H-quinolin-2-one;
6-Methoxy-1-methyl-7-[(6-methyl-2-phenyl-piperidin-3-ylamino)-methyl]-3,4-dihydro-1H-quinolin-2-one;
6-Methoxy-1,3,3-trimethyl-7-[(2-phenyl-piperidin-3-ylamino)-methyl]-3,4-dihydro-1H-quinolin-2-one;
6-Methoxy-1,3-dimethyl-7-[(2-phenyl-piperidin-3-ylamino)-methyl]-3,4-dihydro-1H-quinolin-2-one;
6-Methoxy-1,3-dimethyl-5-[(2-phenyl-piperidin-3-ylamino)-methyl]-1,3-dihydro-indol-2-one;
6-Methoxy-1-methyl-5-[(2-phenyl-piperidin-3-ylamino)-methyl]-1,3-dihydro-indol-2-one;
5-[(1-Isopropyl-2-phenyl-piperidin-3-ylamino)-methyl]-6-methoxy-1,3,3-trimethyl-1,3-dihydro-indol-2-one;
6-Methoxy-1-methyl-7-[(2-phenyl-1-propyl-piperidin-3-ylamino)-methyl]-3,4-dihydro-1H-quinolin-2-one;
6-Methoxy-1-methyl-7-{[1-(5-methyl-3H-imidazol-4-ylmethyl)-2-phenyl-piperidin-3-ylamino]-methyl}-3,4-dihydro-1H-quinolin-2-one;
7-{[1-(1H-Imidazol-4-ylmethyl)-2-phenyl-piperidin-3-ylamino]-methyl}-6-methoxy-1-methyl-3,4-dihydro-1H-quinolin-2-one;
7-[(1-Isopropyl-2-phenyl-piperidin-3-ylamino)-methyl]-6-methoxy-1-methyl-3,4-dihydro-1H-quinolin-2-one;
6-Methoxy-1,3-dimethyl-7-[(1-methyl-2-phenyl-piperidin-3-ylamino)-methyl]-3,4-dihydro-1H-quinolin-2-one;
5-[(1-lsopropyl-2-phenyl-piperidin-3-ylamino)-methyl]-6-methoxy-1,3,3-trimethyl-1,3-dihydro-indol-2-one
6-Methoxy-1-methyl-7-{[1-(5-oxo-2,5-dihydro-1H-[1,2,4]triazol-3-ylmethyl)-2-phenyl-piperidin-3-ylamino]-methyl}-3,4-dihydro-1H-quinolin-2-one;
6-Methoxy-7-[(2-phenyl-piperidin-3-ylamino)-methyl]-3,4-dihydro-1H-quinolin-2-one;
1-Ethyl-6-methoxy-7-[(2-phenyl-piperidin-3-ylamino)-methyl]-3,4-dihydro-1H-quinolin 2-one;
1-Methanesulfonyl-6-methoxy-7-[(2-phenyl-piperidin-3-ylamino)-methyl]-3,4-dihydro-1H-quinolin-2-one;
6-Methoxy-1,4,4-trimethyl-7-[(2-phenyl-piperidin-3-ylamino)-methyl]-3,4-dihydro-1H-quinolin-2-one;
8-Fluoro-6-methoxy-1,4,4-trimethyl-7-[(2-phenyl-piperidin-3-ylamino)-methyl]-3,4-dihydro-1H-quinolin-2-one;
6-Methoxy-1-methyl-7-[(2-phenyl-piperidin-3-ylamino)-methyl]-3,4-dihydro-1H-quinolin-2-one;
6-Methoxy-1,4-dimethyl-7-[(2-phenyl-piperidin-3-ylamino)-methyl]-3,4-dihydro-1H-quinolin-2-one;
6-Methoxy-2-methyl-7-[(2-phenyl-piperidin-3-ylamino)-methyl]-3,4-dihydro-2H-isoquinolin-1-one;
6-Methoxy-3-methyl-5-[(2-phenyl-piperidin-3-ylamino)-methyl]-1,1a,3,7b-tetrahydro-3-aza-cyclopropa[a]naphthalen-2-one;
6-Methoxy-1-methyl-,3,3-cyclopropyl-5-[(2-phenyl-piperidin-3-ylamino)-methyl]-1,3-dihydro-indol-2-one;
5-Methoxy-1-methyl-,3,3-cyclopropyl-6-[(2-phenyl-piperidin-3-ylamino)-methyl]-1,3-dihydro-indol-2-one;
6-Methoxy-1-methyl-(6-phenyl-1,7-diaza-spiro[4.5]dec-3-yl)-3,4-dihydro-1H-quinolin-2-one;
6-Methoxy-1-methyl-7-phenyl-1,7-diaza-spiro[4.5]dec-3-yl)-3,4-dihydro-1H-quinolin-2-one;
6-Methoxy-3-methyl-5-[(1-phenyl-8-aza-bicyclo[3.2.1]oct-2-ylamino)-methyl]-1,1a,3,7b- tetrahydro-3-aza-cyclopropa[a]naphthalen-2-one;
6-Methoxy-3-methyl-5-[(6-methyl-2-phenyl-piperidin-3-ylamino)-methyl]-1,1a,3,7b-tetrahydro-3-aza-cyclopropa[a]naphthalen-2-one;
6-Methoxy-1-methyl-7-(6-phenyl-1,7-diaza-spiro[4.5]dec-3-yl)-3,4-dihydro-1H-quinolin-2-one;
6-Methoxy-1,3,3-trimethyl-5-[(2-phenyl-piperidin-3-ylamino)-methyl]-1,3-dihydro-pyrrolo[2,3-b]pyridin-2-one;
5-Methoxy-1,3,3-trimethyl-6-[(2-phenyl-piperidin-3-ylamino)-methyl]-1,3-dihydro-pyrrolo[3,2-b]pyridin-2-one;
6-Methoxy-1-methyl-7-[(2-phenyl-piperidin-3-ylamino)-methyl]-3,4-dihydro-1H-[1,5]naphthyridin-2-one;
7-[(6-Ethyl-2-phenyl-piperidin-3-ylamino)-methyl]-6-methoxy-1-methyl-3,4-dihydro-1H-quinolin-2-one;
5-[(6-Ethyl-2-phenyl-piperidin-3-ylamino)-methyl]-6-methoxy-1,3,3-trimethyl-1,3,-dihydro-indol-2-one;
6-Methoxy-1,3,3-trimethyl-5-[(2-phenyl-piperidin-3-ylamino)-methyl]-1,3-dihydro-pyrrolo[2,3-b]pyridin-2-one;
5-Methoxy-1,3,3-trimethyl-6-[(2-phenyl-piperidin-3-ylamino)-methyl]-1,3-dihydro-pyrrolo[3,2-b]pyridin-2-one;
6-Methoxy-1-methyl-7-[(2-phenyl-piperidin-3-ylamino)-methyl]-3,4-dihydro-1H-[1,5]naphthyridin-2-one;
6-Methoxy-3-methyl-5-[(6-methyl-2-phenyl-piperidin-3-ylamino)-methyl]-1,1a,3,7b-tetrahydro-3-aza-cyclopropa[a]naphthalen-2-one; and
6-Methoxy-1-methyl-7-(6-phenyl-1,7-diaza-spiro[4.5]dec-3-yl)-3,4-dihydro-1H-quinolin-2-one.

Other more specific examples of compounds of Formula XXI of this invention are:
6-Methoxy-1-methyl-7-[(2-phenyl-6-propyl-piperidin-3-ylamino)-methyl]-3,4-dihydro-1H-quinolin-2-one;
7-[(6-lsopropyl-2-phenyl-piperidin-3-ylamino)-methyl]-6-methoxy-1-methyl-3,4-dihydro-1H-quinolin-2-one;
7-[(6-Tert-butyl-2-phenyl-piperidin-3-ylamino)-methyl]-6-methoxy-1-methyl-3,4-dihydro-1H-quinolin-2-one;
7-[(6-Isobutyl-2-phenyl-piperidin-3-ylamino)-methyl]-6-methoxy-1-methyl-3,4-dihydro-1H-quinolin-2-one;
7-[(1,2,3,4,5,6-Hexahydro-[2,3']bipyridinyl-3-ylamino)-methyl]-6-methoxy-1-methyl-3,4-dihydro-1H-quinolin-2-one;
7-[(1,2,3,4,5,6-Hexahydro-[2,4']bipyridinyl-3-ylamino)-methyl]-6-methoxy-1-methyl-3,4-dihydro-1H-quinolin-2-one;
6-Methoxy-3-methyl-5-[(6-methyl-2-phenyl-piperidin-3-ylamino)-methyl]-1,1a,3,7b-tetrahydro-3-aza-cyclopropa[a]naphthalen-2-one;
6-Methoxy-1-methyl-,3,3-cyclopropyl-5-[(6-methyl-2-phenyl-piperidin-3-ylamino)-methyl]-1,3-dihydro-indol-2-one;
5-Methoxy-1-methyl-,3,3-cyclopropyl-6-[(1-phenyl-8-aza-bicyclo[3.2.1]oct-2-ylamino)-methyl]-1,3-dihydro-indol-2-one;
6-Methoxy-1-methyl-,3,3-cyclohexane-5-[(2-phenyl-piperidin-3-ylamino)-methyl]-1,3-dihydro-indol-2-one;
6-Methoxy-1-methyl-,3,3-cyclopentyl-5-[(2-phenyl-piperidin-3-ylamino)-methyl]-1,3-dihydro-indol-2-one;
6-Methoxy-1-methyl-,3,3-cyclopropyl-5-[(2-(-4-fluorophenyl)-piperidin-3-ylamino)-methyl]-1,3-dihydro-indol-2-one;
6-Methoxy-1-methyl-,3,3-cyclobutyl-5-[(2-phenyl-piperidin-3-ylamino)-methyl]-1,3-dihydro-indol-2-one;
5-Methoxy-1-methyl-,3,3-cyclobutyl-6-[(2-phenyl-piperidin-3-ylamino)-methyl]-1,3-dihydro-indol-2-one;
5-Methoxy-1-methyl-,3,3-cyclopropyl-6-[(6-methyl-2-phenyl-piperidin-3-ylamino)-methyl]-1,3-dihydro-indol-2-one;
6-Methoxy-1,3-dimethyl-5-[(2-phenyl-piperidin-3-ylamino)-methyl]-1,1a,3,7b-tetrahydro-3-aza-cyclopropa[a]naphthalen-2-one;
7-[(1,2,3,4,5,6-Hexahydro-[2,2']bipyridinyl-3-ylamino)-methyl]-6-methoxy-1-methyl-3,4-dihydro-1H-quinolin-2-one; and
6-[(6-Ethyl-2-phenyl-piperidin-3-ylamino)-methyl]-5-methoxy-1,1-dimethyl-indan-2-one.

Other examples of specific NK-1 receptor antagonists of formula 1-XX that can be used in the methods and pharmaceutical compositions of this invention are the following compounds and their pharmaceutically acceptable salts:
(*2S,3S*)-3-[2-methoxy-5-(2-thiazolyl)benzyl]amino-2-phenylpiperidine;
(*2S,3S*)-3-(5-(2-imidazolyl)-2-methoxybenzyl]amino-2-phenylpiperidine;
(*2S,3S*)-3-(2-methoxy-5-(2-oxopyrrolidinyl)benzyl]amino-2-phenylpiperidine;
(*2S,3S*)-3-[2-methoxy-5-(4-methyl-2-thiazolyl)benzyl]-amino-2-phenylpiperidine;
(*2S,3S*)-3-(2-methoxy-5-(1,2,3-thiadiazol-4-yl)benzyl]amino-2-phenylpiperidine;
(*2S,3S*)-(6-methoxy-2-methyl-benzothiazol-5-ylmethyl)-(2-phenylpiperidin-3-yl)amine;
(*2S,3S*)-(5-(2,5-dimethyl-pyrrol-1-yl)-2-methoxybenzyl]-(2-phenylpiperidin-3-yl)amine;
(*2S,3S*)-3-[2-methoxy-5-(5-oxazolyl)benzyl]amino-2-phenylpiperidine;
(*2S,3S*)-(6-methoxy-2-phenyl-benzothiazol-5-ylmethyl)-(2-phenylpiperidin-3-yl)-amine;
(*2S,3S*)-(6-methoxy-2-cyclopropyl-benzothiazol-5-ylmethyl)-(2-phenylpiperidin-3-yl)amine;
(*2S,3S*)-(6-methoxy-2-tert-butyl-benzothiazol-5-ylmethyl)-(2-phenylpiperidin-3-yl)amine;
(*2S,3S*)-(6-isopropoxyoxy-2-phenyl-benzothiazol-5-ylmethyl)-(2-phenylpiperidin-3-yl)amine;
(*2S,3S*)-(6-isopropoxyoxy-2-methyl-benzothiazol-5-ylmethyl)-(2-phenylpiperidin-3-yl)amine;
(*2S,3S*)-(6-trifluoromethoxy-2-methyl-benzothiazol-5-ylmethyl)-(2-phenylpiperidin-3-yl)amine;
(*2S,3S*)-(6-methoxy-2-methyl-benzoxazol-5-ylmethyl)-(2-phenylpiperidin-3-yl)amine;
(*1SR-2SR, 3SR, 4RS*)-3-(6-methoxy-3-methylbenzisoxazol-5-yl]methylamino-2-benzhydrylazanorbornane;
(*2S,3S*)-(2-methoxy-5-pyridin-2-ylbenzyl)-(2-phenylpiperidin-3-yl)amine;
(*2S,3S*)-(2-methoxy-5-pyrimidin-2-ylbenzyl)-(2-phenylpiperidin-3-yl)amine;
(*2S,3S*)-(2-methoxy-5-pyridin-3-ylbenzyl)-(2-phenylpiperidin-3-yl)amine;
(*2S,3S*)-(2-methoxy-5-(6-methylpyridin-2-yl)benzyl]-(2-phenylpiperidin-3-yl)amine;
(*2S,3S*)-[5-(3,5-dimethylpyrazol-1-yl)-2-methoxybenzyl]-(2-phenylpiperidin-3-yl)amine;
(*2S,3S*)-[2-methoxy-5-(3,4,5-trimethylpyrazol-1-yl)benzyl]-(2-phenylpiperidin-3-yl)amine;
(*2S,3S*)-(2-isopropoxy-5-(3,4,5-trimethylpyrazol-1-yl)benzyl)-(2-phenylpiperidin-3-yl)amine;
(*2S,3S*)-[5-(3,5-diisopropylpyrazol-1-yl)-2-methoxybenzyl]-(2-phenylpiperidin-3-yl)amine;
(*2S,3S*)-[5-(3,5-dimethylthiophen-2-yl)-2-methoxybenzyl]-(2-phenylpiperidin-3-yl)amine;
(*2S,3S*)-(6-methoxy-2,3-d imethyl-benzo[b]thiophen-7-ylmethyl)-(2-phenylpiperidin-3-yl)amine.
(*2S,3S*)-(6-methoxy-3-methyl-benzo(d]isoxazol-5-ylmethyl)-(2-phenylpiperidin-3-yl)-amine;
(*1SR,2SR,3SR,4RS*)-(2-benzhydryl-1-aza-bicyclo[2.2.1)hept-3-yl)-6-methoxy-2-methyl-benzothiazol-5-ylmethyl)-amine;
(*2S,3S*)-(6-methoxy-benzoxazol-5-ylmethyl)-(2-phenyl-piperidin-3-yl)-amine;
(*2S,3S*)-(6-methoxy-benzothiazol-5-ylmethyl)-(2-phenyl-piperidin-3-yl)-amine;
(*2S,3S*)-5-methoxy-1-methyl-6-(2-phenylpiperidin-3-ylaminomethyl)-1,3-dihydro-indol-2-one;
(*2S,3S*)-6-methoxy-3-methyl-5-(2-phenylpiperidin-3-ylaminomethyl)-3H-benzoxazol-2-one;
(*2S,3S*)-6-methoxy-3-methyl-5-(2-phenylpiperidin-3-ylaminomethyl)-3H-benzothiazol-2-one;
(*2S,3S*)-5-methoxy-1,3-dimethyl-6-(2-phenylpiperidin-3-ylaminomethyl)-1,3-dihydro-benzoimidazol-2-one;
(*2S,3S*)-(6-methoxy-3-methyl-3H-benzotriazol-5-ylmethyl)-(2-phenylpiperidin-3-yl)amine;
(*2S,3S*)-(2-methoxy-5-[1,2,3]thiadiazol-4-yl-benzyl)-(2-phenyl-1-azabicyclo[2.2.2]oct-3-yl)amine;
(*2S,3S*)-(2-methoxy-5-[1,2,3]thiadiazol-4-yl-benzyl)-(2-benzhydryl-1-azabicyclo-[2.2.2)oct-3-yl)amine;
(*2S,3S*)-(6-methoxy-2-methyl-benzothiazol-5-ylmethyl)-(2-phenyl-1-azabicyclo-[2.2.2]oct-3-yl)amine;
(*2S,3S*)-(6-methoxy-2-methyl-benzothiazol-5-ylmethyl)-(2-benzhydryl-1-azabicyclo-[2.2.2)oct-3-yl)amine;
(*2S,3S*)-(2-methoxy-5-thiazol-2-yl-benzyl)-(2-benzhydryl-1-azabicyclo(2.2.2)oct-3-yl)amine;
(*2S,3S*)-(6-methoxy-2-methyl-benzothiazol-5-ylmethyl)-(2-phenyl-1-azabicyclo-[2.2.1]hept-3-yl)amine;
(*2S,3S*)-(6-methoxy-2-methyl-benzothiazol-5-ylmethyl)-(2-benzhydryl-l-azabicyclo-[2.2.1]hept-3-yl)amine;
(*2S,3S*)-(2-methoxy-5-[1,2,4]triazol-4-yl-benzyl)-(2-phenylpiperidin-3-yl)amine;
(*2S,3S*)-(2-methoxy-5-(1,2,4)triazol-1-yl-benzyl)-(2-phenylpiperidin-3-yl)amine;
(*2S*,*3S*)-(2-methoxy-5-thiazol-2-ylbenzyl)-(2-phenyl-decahydroquinolin-3-yl)amine;
(*2S*,*3S*)-(2-methoxy-5-thiazol-2-ylbenzyl)-(2-phenyl-octahydro-indol-3-yl)amine;
(*2S*,*3S*)-(2-methoxy-5-oxazol-4-ylbenzyl)-(2-phenylpiperidin-3-yl)amine;
(*2S*,*3S*)-(6-methoxy-2-(2-propyl)-benzothiazol-5-ylmethyl)-(2-phenylpiperidin-3-yl)-amine;
(*2S*,*3S*)-N-[(5-oxo-1H,4H-1,2,4-triazolo)methyl]-2-(4-fluorophenyl)-3-(3,5-ditrifluoromethyl )benzyloxymorpholine;
(*1SR,2SR,3SR,4RS*)-(2-benzhydryl-1-azabicyclo[2.2.1]hept-3-yl)-(6-methoxy-2-phenylbenzothiazol-5-ylmethyl)amine;
(*1SR,2SR,3SR,4RS*)-(2-benzhydryl-1-azabicyclo[2.2.1]hept-3-yl)-(6-methoxy-2-cyclopropylbenzothiazol-5-ylmethyl)amine;
(*1SR,2SR,3SR,4RS*)-(2-benzhydryl-1-azabicyclo[2.2.1]hept-3-yl)-(6-methoxy-2-tert-butylbenzothiazol-5-ylmethyl)amine;
(*1SR,2SR,3SR,4RS*)-(2-benzhydryl-1-azabicyclo[2.2.1)hept-3-yl)-(6-methoxy-2-(2-propyl)benzothiazol-5-ylmethyl)amine;
(*1SR,2SR,3SR,4RS*)-(2-benzhydryl-1-azabicyclo[2.2.1]hept-3-yl)-(6-isopropoxyoxy-2--phenyl-benzothiazol-5-ylmethyl )amine;
(*1SR,2SR,3SR,4RS*)-(2-benzhydryl-1-azabicyclo[2.2.1]hept-3-yl)-(6-isopropoxyoxy-methyl-benzothiazol-5-ylmethyl)amine;
(*1SR,2SR,-3SR,4RS*)-(2-benzhydryl-1-azabicyclo[2.2.1]hept-3-yl)-(6-trifluoromethoxy-2-methyl-benzothiazol-5-ylmethyl)amine;
(6-methoxy-1-oxa-2,3-diazainden-5-ylmethyl)-(2-phenyl-piperidin-3-yl)amine; and
(6-methoxy-2-methyl-1H-benzoimidazol-5-ylmethyl)-(2-phenylpiperidine-3-yl)amine.
(±)-[3R-[3α, 6α (R*)]]-3-phenyl-7-phenyl-1,8-diazaspiro[5.5]undecane;
(±)-[3R-[3α,6α (R*)]]-3-(2-methoxyphenyl)-7-phenyl-1,8-diazaspiro[5.5]undecane;
(±)-[3R-[3α,6α (R*)]]-3-(2-methoxy-5-trifluoromethoxy-phenyl)-7-phenyl-1,8-diazaspiro-[5.5]undecane;
(±)-[3R-[3α,6α(R*)]]-3-(5-chloro-2-methoxyphenyl)-7-phenyl-1,8-diazaspiro-[5.5]undecane;
(±)-[3R-[3α,6α(R*)]]-3-(5-isopropyl-2-methoxyphenyl)-7-phenyl-1,8-diazaspiro[5.5]-undecane;
(±)-[3R-[3α,6α(R*)]]-3-(5-tert.butyl-2-methoxyphenyl)-7-phenyl-1,8-diazaspiro[5.5]-undecane;
(±)-[3R-[3α,6α(R*)]]-3-(2-methoxy-5-(N-methyl-N-methylsulfonylaminophenyl)-7-phenyl-1,8-diazaspiro[5.5]-undecane;
(±)-[3R-[3α,6α(R*)]]-3-(2-iodophenyl)-7-phenyl-1,8-diazaspiro[5.5]undecane;
(±)-[3R-[3α,6α(R*)]]-3-(2-methoxy-4-methylphenyl)-7-phenyl-1,8-diazaspiro[5.5]-undecane;
(±)-[3R-[3α,6α(R*)]]-3-(2-isopropoxyphenyl)-7-phenyl-1,8-diazaspiro[5.5]-undecane;
(±)-[3R-[3α,6α(R*)]]-3-(2-difluoromethoxy-5-trifluoromethoxyphenyl)-7-phenyl-1,8-diazaspiro[5.5]undecane;
(±)-[3R-[3α,5α(R*)]]-3-(2-methoxyphenyl)-6-phenyl-1,7-diazaspiro[4.5]decane;
(±)-[3R-(3α,5α(R*)]]-3-(2-methoxy-5-trifluoromethoxyphenyl)-6-phenyl-1,7-diazaspiro-[4.5]decane;
(±)-[3R-[3α,5α(R*)]]-3-(5-chloro-2-methoxyphenyl)-6-phenyl-1,7-diazaspiro[4.5]decane;
(±)-[3R-[3α,5α(R*)]]-3-(5-isopropyl-2-methoxyphenyl)-6-phenyl-1,7-diazaspiro[4.5]decane;
(±)-[3R-[3α,5α(R*)]]-3-(5-tert.butyl-2-methoxyphenyl)-6-phenyl-1,7-diazaspiro[4.5]decane;
(*2S,3S*)-3-(2-Fluoro-5-(trifluoromethyl)benzyl)amino-2-phenylpiperidine;
(*2S,3S*)-3-(2-Chloro-5-(trifluoromethyl)benzyl)amino-2-phenylpipendine;
(*2S,3S*)-3-(2-Methoxy-5-(trifluoromethyl)benzyl)amino-2-phenylpiperidine;
(*2S,3S*)-3-(2-Phenoxy-5-(trifluoromethyl)benzyl)amino-2-phenylpiperidine;
(*2S,3S*)-3-(5-(1,1-Difluoroethyl)-2-(trifluoromethoxy)benzyl)amino-2-phenylpiperidine;
(*2S,3S*)-3-(5-(1,1-Difluoroethyl)-2-methoxybenzyl)amino-2-phenylpiperidine;
(*2S,3S*)-3-(2-Methoxy-5-(2,2,2-trifluoroethyl)benzyl)amino-2-phenylpiperidine;
(*2S,3S*)-3-(2-Methoxy-5-(1-(trifluoromethyl)ethyl)benzyl)amino-2-phenylpiperidine;
(*2S,3S*)-3-[5-(1,1-dimethyl-4,4,4-trifluoro-2-butynyl)-2-methoxybenzyl)amino-2-phenylpiperidine;
(*2S,3S*)-3-[5-(1,1-Dimethyl-2,2,2-trifluoroethyl )-2-methoxybenzylamino]-2-phenylpiperidine;
(*2S,3S*)-3-(2,4-Dimethoxy-5-(2,2,2-trifluoroethyl)benzyl)amino-2-phenylpiperidine;
(*2S,3S*)-3-[5-[(1-Chloro-1-(trifluoromethyl)ethyl]-2-methoxybenzylamino]-2-phenylpiperidine;
(*2S,3S*)-2-Phenyl-3-(5-(2,2,2-trifluoro-1-(trifluoromethyl)ethyl)-2-methoxybenzyl)aminopiperidine;
(*2S,3S*)-2-Phenyl-3-(5-(2,2,2-trifluoro-1-(trifluoromethyl)ethyl)-2-methoxybenzyl)aminopiperidine;
(*2S,3S*)-2-Phenyl-3-(5-(1,2,2,2-tetrafluoro-1-(trifluoromethyl)ethyl)-2-methoxybenzyl)aminopiperidine;
(*2S,3S*)-3-(2-Methoxy-5-(1,1,2,2,2-pentafluoroethyl)benzyl)amino-2-phenylpiperidine;
(*2S,3S*)-2-Phenyl-3-(5-(2,2,2-trifluoro-1-methyl-1-(trifluoromethyl)ethyl)-2-methoxy-benzyl)aminopiperidine;
(*2S,3S*)-3-[5-[2,2-Difluoro-1-(trifluoromethyl)ethenyl]-2-methoxybenzyl]amino-2-phenylpiperidine;
(*2S,3S*)-3-(2-Methoxy-5-(2,2,2-trifluoro-1-hydroxy-1-(trifluoromethyl)ethyl)benzyl)-amino-2-pheriylpiperidine;
(*2S,3S*)-3-[5-Methoxy-1-(trifluoromethyl)indan-6-yl)methylamino]-2-phenylpiperidine;
(*2S,3S*)-3-((6-Methoxy-1-(trifluoromethyl)-1,2,3,4-tetrahydronaphthalen-7-yl)methyl)amino-2-phenylpiperidine;
(*2S,3S*)-3-((2,2-Difluoro-6-methoxy-1,2,3,4-tetrahydronaphthalen-7-yl)methyl)amino-2-phenylpiperidine;
(*2S,3S*)-3-(6-methoxy-1,3,3-trimethyloxindol-5-yl)methylamino-2-phenylpiperidine;
(2S,3S)-3-(6-methoxy-1-methyl-2-oxo-1,2,3,4-tetrahydroquinolin-7-yl)methylamino-2-phenylpiperidine;
(2S,3S)-3-(6-isopropoxy-1-methyl-2-oxo-1,2,3,4-tetrahydroquinolin-7-yl)methylamino-2-phenylpiperidine;
(2S,3S)-3-(1-isopropyl-6-methoxy-2-oxo-1,2,3,4-tetrahydroquinolin-7-yl)methylamino-2-phenylpiperidine;
(2S,3S)-3-[(6-methoxy-1-methyl-2-thioxo-1,2,3,4-tetrahydroquinolin-7-yl)methyl]amino-2-phenylpiperidine dihydrochloride;
(2S,3S)-3-[(7-methoxy-1-methyl-2-oxo-1,2,3,4-tetrahydroquinolin-6-yl)methyl]amino-2-phenylpiperidine dihydrochloride;
(2S,3S)-3-[(6-methoxy-1-methyl-2-oxo-4H-3,1-benzothiazin-7-yl)methyl]amino-2-phenylpiperidine dihydrochloride.
(2S,3S)-3-[(6-methoxy-1-methyl-2-oxo-4H-3,1-benzothiazin-7-yl)methyl]amino-2-phenylpiperidine dihydrochloride;
(2S,3S,4R)-2-diphenylmethyl-3-[(2-methoxy-4,5-dimethylphenyl)methylamino]-4-(2-hydroxyethyl)pyrrolidine;
(2SR,3SR,4RS)-2-diphenylmethyl-3-[(2-methoxy-4,5-dimethylphenyl)methylamino]-4-(2-hydroxyethyl)pyrrolidine;
(2SR,3SR,4RS)-2-diphenylmethyl-3-[(2-methoxy-5-(methylethyl)phenyl)methylamino]-4-(carbomethoxymethyl)- pyrrolidine;
(2SR,3SR,4RS)-2-diphenylmethyl-3-[(2-methoxy-5-(methylethyl)phenyl)methylamino]-4-(carboxymethyl)-pyrrolidine;
(2SR,3SR,4RS)-2-diphenylmethyl-3-[(2-methoxy-5-(methylethyl)phenyl)methylamino]-4-(2-dimethylamino-carbamoylethyl)pyrrolidine;
(2SR,3SR,4RS)-2-diphenylmethyl-3-[(2-trifluoromethoxyphenyl)methylamino]-4-(2-hydroxyethyl)-pyrrolidine;
(2S, 3S, 4R)-2-diphenylmethyl-3-[(2-methoxy-5-(1,1-dimethylethyl)phenyl)methylamino]-4-(2-hydroxyethyl)- pyrrolidine;
(2SR,3SR,4RS)-2-diphenylmethyl-3-[(2-methoxy-5-(1,1-dimethylethyl)phenyl)methylamino]-4-(2-methoxyethyl)- pyrrolidine;
(2S,3S,4R)-2-diphenylmethyl-3-[(2-methoxy-5-methylethyl)phenyl)methylamino]-4-(2-hydroxyethyl)- pyrrolidine;
(2SR,3SR,4RS)-2-diphenylmethyl-3-[(2-methoxy-5-methylethyl)phenyl)methylamino]-4-(2-methoxyethyl)- pyrrolidine;
(2SR,3SR,4RS)-2-diphenylmethyl-3-[(2-methyl-5-(1,1-dimethylethyl)phenyl)methylamino]-4-(2-hydroxyethyl)- pyrrolidine;
(1SR,2SR,3SR,4RS)-1-aza-2-diphenylmethyl-3-[(2-methoxy-4,5-dimethylphenyl)methylamino]-bicyclo[2.2.1]- heptane;
(1SR,2SR,3SR,4RS)-1-aza-2-diphenylmethyl-3-[(2-methoxyphenyl)methyamino]bicyclo[2.2.1]heptane;
(1SR,2SR,3SR,4RS)-1-aza-2-diphenylmethyl-3-[(2-methoxy-5-(1,1-dimethylethyl)phenyl)methylamino]bicyclo-[2.2.1]heptane;
(1SR,2SR,3SR,4RS)-1-aza-2-diphenylmethyl-3-[(2-methoxy-5-trifluoromethoxphenyl)methylamino]bicyclo- [2.2.1]heptane;
(1SR,2SR,3SR,4RS)-1-aza-2-diphenylmethyl-3-((2-methoxy-5-(1-methylethyl)phenyl)methylamino]bicyclo- [2.2.1]heptane;
(1SR,2SR,3SR,4RS)-1-aza-2-diphenylmethyl-3-[(2-methoxy-5-propylphenyl)methyamino]bicyclo[2.2.1]heptane;
(1SR,2SR,3SR,4RS)-1-aza-2-diphenylmethyl-3-[(2-methoxy-5-(1-methylpropyl)phenyl)methylamino]bicyclo- [2.2.1]heptane;
(1SR,2SR,3SR,4RS)-1-aza-2-phenyl-3-[(2-methoxyphenyl)methylamino]bicyclo[2.2.1]heptane;
(1SR,2SR,3RS,4RS)-1-aza-2-phenyl-3-[(2-methoxy-5-trifluoromethoxyphenyl)methylamino]bicyclo[2.2.1]heptane;
(2SR,3SR,4RS)-N-1-phenylmethyl-2-diphenylmethyl-3-[(2-methoxyphenyl)methylamino]-4-(2-hydroxyethyl)- pyrrolidine;
(2SR,3SR,4RS)-2-diphenylmethyl-3-[(2-methoxy-phenyl)methylamino]-4-(2-hydroxyethyl)pyrrolidine;
(2SR,3SR,4RS)-2-diphenylmethyl-3-[(2-methoxy-5-(1,1-dimethylethyl)phenyl)methylamino]-4-(2-hydroxyethyl)- pyrrolidine;
(2SR,3SR,4RS)-2-diphenylmethyl-3-[(2-methoxy-5-trifluoromethoxyphenyl)methylamino]-4-(2-hydroxyethyl)- pyrrolidine;
(2SR,3SR,4RS)-2-diphenylmethyl-3-[(2-methoxy-5-(1-methylethyl)phenyl)methylamino]-4-(2-hydroxyethyl)- pyrrolidine;
(2SR,3SR,4RS)-2-diphenylmethyl-3-[(2-methoxy-5-propylphenyl)methylamino]-4-(2-hydroxyethyl)pyrrolidine;
(2SR,3SR,4RS)-2-diphenylmethyl-3-[(2-methoxy-5-(1-methyl-1-propyl)phenyl)methylamino]-4-(2-hydroxy-ethyl)pyrrolidine;
(2SR,3SR,4RS)-2-diphenylmethyl-3-[(2-trifluoro methoxy-5-(1,1-dimethylethyl)phenyl)methylamino]-4-(2-hydroxyethyl)pyrrolidine;
(2SR,3SR,4RS)-2-diphenylmethyl-3-[(2-methoxy-5-chlorophenyl)methylamino]-4-(2-hydroxyethyl)pyrrolidine;
(2SR,3SR,4RS)-2-phenyl-3-[(2-methoxyphenyl)methyl-amino]-4-(2-hydroxyethyl)pyrrolidine;
(2SR,3SR,4RS)-2-phenyl-3-[(2-methoxy-5-(1,1-dimethylethyl)phenyl)methylamino]-4-(2-hydroxy-ethyl)pyrrolidine;
(2SR,3SR,4RS)-2-phenyl-3-[(2-methoxy-5-trifluoromethoxyphenyl)methylamino]-4-(2-hydroxy-ethyl)pyrrolidine;
6-Methoxy-1,3,3-trimethyl-5-[(2-phenyl-piperidin-3-ylamino)-methyl]-3-dihydro-indol-2-one;
6-Methoxy-1,methyl-7-[(2-phenyl-piperidin-3-ylamino)-methyl]-,3-4-dihydro-1H-quindol-2-one;
6-Methoxy-1,2-dimethyl-1,2,3,4-terrahydro-quinolin-7-ylmethyl)-(2-phenyl-piperidin-3-yl)-amine;
6-Isopopoxy-1-methyl-7-[(2-phenyl-piperidin-3-ylamino)-methyl]-3,4-dihydro-1H-quinolin-2-one;
7-Methoxy-1-methyl-6-[(2-phenyl-piperidin-3-ylamino)-methyl]-3,4-dihydro-1H-quinolin-2-one;
6-Methoxy-1-methyl-7-[(2-phenyl-piperidin-3-ylamino)-methyl]-1,4-dihydro-benzo-[d][1,3]thiazin-2-one;
6-Methoxy-7-[(2-phenyl-piperidin-3-ylamino)-methyl]1-(2,2,2-trifluoroethyl)-3,4-dihydro-1H-quinolin-2-one;
6-Isopopoxy-1-methyl-7-[(2-phenyl-piperidin-3-ylamino)-methyl]-3,4-dihydro-1H-quinolin-2-thione; and
6-Methoxy-1,3-dimethyl-7-[(2-phenyl-piperidin-3-ylamino)-methyl]-3,3-dihydro-1H-quinolin-2-one.

Other preferred embodiments of this invention relate to the above pharmaceutical compositions for the treatment of anxiety or depression, and the above methods of treating anxiety or depression, wherein the NK-1 receptor antagonist, or pharmaceutically acceptable salt thereof of Formula I-XX is selected from the following compounds and their pharmaceutically acceptable salts:
(6-Methoxy-3-trifluoromethyl-benzo[d]isoxazol-5-ylmethyl)-(2-phenyl-piperidin-3-yl)-amine;
6-Methoxy-1-methyl-7-[(2-phenyl-1-propyl-piperidin-3-ylamino)-methyl]-3,4-dihydro-1H-quinolin-2-one;
6-Methoxy-1-methyl-7-{[1-(5-oxo-2,5-dihydro-1H-[1,2,4]triazol-3-ylmethyl)-2-phenyl-piperidin-3-ylamino]-methyl}-3,4-dihydro-1H-quinolin-2-one;
3-(2-Methoxy-5-trifluoromethoxy-phenyl)-6-phenyl-1,7-diaza-spiro[4.5]decane;
6-Methoxy-1-methyl-7-[(2-phenyl-piperidin-3-ylamino)-methyl]-3,4-dihydro-1H-quinolin-2-one;
[2-Methoxy-5-(2,2,2-trifluoro-1-trifluoromethyl-ethyl)-benzyl]-(2-phenyl-piperidin-3-yl)-amine;
(*2S,3S*)-N-[(5-oxo-1H,4H-1,2,4-triazolo)methyl]-2-(4-fluorophenyl)-3-(3,5-ditrifluoromethyl)benzyloxymorpholine;
[5-(1,1-Dimethyl-prop-2-ynyl)-2-methoxy-benzyl]-(2-phenyl-piperidin-3-yl)-amine;
7-Methoxy-1-methyl-6-[(2-phenyl-piperidin-3-ylamino)-methyl]-3,4-dihydro-1H-quinolin-2-one;
[2-Methoxy-5-(2,2,2-trifluoro-1,1-dimethyl-ethyl)-benzyl]-(2-phenyl-piperidin-3-yl)-amine;
(7-Methoxy-4-methyl-3,4-dihydro-2H-benzo[1,4]oxazin-6-ylmethyl)-(2-phenyl-piperidin-3-yl)-amine;
[2-Methoxy-5-(1-methyl-1-trifluoromethyl-prop-2-ynyl)-benzyl]-(2-phenyl-piperidin-3-yl)-amine;
(6-Methoxy-1-methyl-1-trifluoromethyl-isochroman-7-ylmethyl)-(2-phenyl-piperidin-3-yl)-amine;
2-{3-[(2-Benzhydryl-1-aza-bicyclo[2.2.2]oct-3-ylamino)-methyl]-4- methoxy-phenyl}-2-methyl-propan-1-ol;
3-(3,5-Bis-trifluoromethyl-benzyloxy)-2-phenyl-piperidine;
5-[2-(3,5-Bis-trifluoromethyl-benzyloxy)-3-phenyl-morpholin-4-ylmethyl]-2,4-dihydro-[1,2,4]triazol-3-one;
(*2S,3S*)-3-(2-Methoxy-5-(trifluoromethoxy)benzyl)amino-2-phenylpiperidine;
(2S, 3S)-N-(5-isopropyl-2-methoxyphenyl)methyl-2-diphenylmethyl-1-azabicyclo[2.2.2]-octan-3-amine;
(*2S,3S*)-N-[(5-oxo-1H,4H-1,2,4-triazolo)methyl]-2-(4-fluorophenyl)-3-(3,5-ditrifluoromethyl)benzyloxymorpholine;
(2S,3S)-N-(5-tert-butyl-2-methoxyphenyl)methyl-2-diphenylmethyl-1-azabicyclo[2.2.2]-octane-3-amine;
(2S,3S)-N-(5-ethyl-2-methoxyphenyl)methyl-2-diphenylmethyl-1-azabicyclo[2.2.2]-octan-3-amine; and
(2S,3S)-N-(5-n-propyl-2-methoxyphenyl)methyl-2-diphenylmethyl-1-azabicyclo[2.2.2]-octane-3-amine.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "alkoxy", as used herein, includes O-alkyl groups wherein "alkyl" is defined as above.

The term "one or more substituents, as used herein, includes from one to the maximum number of substituents possible based on the number of available bonding sites.

The term "methylene", as used herein, means -CH₂-.

The term "ethylene", as used herein, means -CH₂CH₂-.

The term "propylene", as used herein, means -CH₂CH₂CH₂-.

The terms "anxiolytic effective amount" and "antianxiety effective amount", as used herein, refer to an amount that is effective in treating anxiety.

The term "antidepressant effective amount", as used herein, refers to an amount that is effective in treating depression.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The pharmaceutical compositions and methods of this invention comprise, or comprise administering NK-1 receptor antagonists of the formulas I through XX, which may have chiral centers and therefore exist in different enantiomeric forms. This invention includes methods and pharmaceutical compositions, as described above, wherein the NK-1 receptor antagonists that are employed are optical isomers, tautomers or stereoisomers of the compounds of formulas I through XX that are defined above, or mixtures thereof.

This present invention also relates to pharmaceutical compositions and methods comprising, or comprising administering, pharmaceutically acceptable acid addition salts of NK-1 receptor antagonists and of antidepressant and anxiolytic agents. The possible acids which are used to prepare the pharmaceutically acceptable acid addition salts of the basic active agents employed in the methods and pharmacuetical compositions of this invention are those which form non-toxic acid addition salts, *i.e*., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i.e*., 1,1'-methylene-bis-(2-hydroxy-3- naphthoate)]salts.

This invention also relates to pharmaceutical compositions and methods comprising, or comprising administering pharmaceutically acceptable base addition salts of NK-1 receptor antagonists and of NK-3 antagonists. The chemical bases that may be used as reagents to prepare pharmaceutically acceptable base salts of the acidic active agents that are employed in the methods of this invention are those that form non-toxic base salts with such compounds. Such non-toxic base salts include, but are not limited to those derived from such pharmacologically acceptable cations such as alkali metal cations (*e.g.*, potassium and sodium) and alkaline earth metal cations (*e.g.*, calcium and magnesium), ammonium or water-soluble amine addition salts such as N-methylglucamine (meglumine), and the lower alkanolammonium and other base salts of pharmaceutically acceptable organic amines.

The subject invention also relates to pharmaceutical compositions and methods of treatment that employ isotopically-labeled compounds that are identical to those recited in formulas I through XXI, or to other NK-1 receptor antagonists, but for the fact that one or more atoms are replaced by an atom having an atomic mass or mass number different from the atomic mass or mass number usually found in nature. Examples of isotopes that can be incorporated into the NK-1 receptor antagonists that are employed in the pharmaceutical compositions and methods of the present invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P ³²P, ³⁵S, ¹⁸F, and ³⁶Cl, respectively. The NK-1 receptor antagonists employed in the pharmaceutical compositions and methods of the present invention, prodrugs thereof, and pharmaceutically acceptable salts of said compounds or of said prodrugs which contain the aforementioned isotopes and/or other isotopes are within the scope of this invention. Certain isotopically-labeled NK-1 receptor antagonists, for example, those into which radioactive isotopes such as ³H and ¹⁴C are incorporated, are useful in drug and/or substrate tissue distribution assays. Tritiated, *i.e.*, ³H, and carbon-14, *i.e.*, ¹⁴C, isotopes are particularly preferred for their ease of preparation and detectability. Further, substitution with heavier isotopes such as deuterium, *i.e.*, ²H, can afford certain therapeutic advantages resulting from greater metabolic stability, for example increased *in vivo* half-life or reduced dosage requirements and, hence, may be preferred in some circumstances.

### Detailed Description Of The Invention

The following references refer, collectively, to quinuclidine, piperidine, ethylene diamine, pyrrolidine and azanorbornane derivatives and related compounds that exhibit activity as NK-1 receptor antagonists and that can be used, in combination with the NK-3 antagonists, in the pharmaceutical compositions and methods of this invention, and to methods of preparing the same: United States Patent 5,162,339, which issued on November 11, 1992; United States Patent 5,232,929, which issued on August 3, 1993; World Patent Application WO 92/20676, published November 26, 1992; World Patent Application WO 93/00331, published January 7, 1993; World Patent Application WO 92/21677, published December 10, 1992; World Patent Application WO 93/00330, published January 7, 1993; World Patent Application WO 93/06099, published April 1, 1993; World Patent Application WO 93/10073, published May 27, 1993; World Patent Application WO 92/06079, published April 16, 1992; World Patent Application WO 92/12151, published July 23, 1992; World Patent Application WO 92/15585, published September 17, 1992; World Patent Application WO 93/10073, published May 27, 1993; World Patent Application WO 93/19064, published September 30, 1993; World Patent Application WO 94/08997, published April 28, 1994; World Patent Application WO 94/04496, published March 3, 1994; World Patent Application WO 95/07908, published March 3, 1995; World Patent Application WO 94/20500, published September 15, 1994; World Patent Application WO 94/13663, published June 23, 1994; World Patent Application WO 95/16679, published June 22, 1995; World Patent Application WO 97/08144, published March 6, 1997; World Patent Application WO 97/03066, published January 30, 1997; World Patent Application WO 99/25714, published May 27, 1999; United States Patent Application 988,653, filed December 10, 1992; United States Patent Application 026,382, filed March 4, 1993; United States Patent Application 123,306, filed September 17, 1993, and United States Patent Application 072,629, filed June 4, 1993. All of the foregoing World Patent Applications designate the United States. The foregoing patents and patent applications are incorporated herein by reference in their entirety.

NK-1 receptor antagonists of the formula I can be prepared as described in the following patents and patent applications, all of which are referred to above and incorprated herein by reference in their entirety: WO 93/00331, WO 92/21677, WO 92/15585, WO 92/01688, WO 93/06099, WO 91/18899, United States Patent 5,162,339,and United States Patent 5,232,929. NK-1 receptor antagonists of the formula la (*i.e.*, compounds defined identically to compounds of the formula I, but having the further proviso that when neither X¹, X² nor X³ is a fluorinated alkoxy group, at least one of R¹, R³, R⁴, R⁵, R⁶, R⁷ and R¹³ is an aryl group substituted with a fluorinated alkoxy group) can be prepared as described in WO 93/00331.

NK-1 receptor antagonists of the formula IXa and IXb can be prepared as described in World Patent Application WO 94/13663, published June 23, 1994.

NK-1 receptor antagonists of the formula XVIII can be prepared as described in World Patent Application WO 97/08144, published March 6, 1997.

NK-1 receptor antagonists of the formula XIX can be prepared as described in World Patent Application WO 97/03066, published January 30, 1997 and World Patent Application WO 99/25714, published May 27, 1999.

NK-1 receptor antagonists of the formula XX can be prepared as described in World Patent Application WO 94/20500, published September 15, 1994.

NK-1 receptor antagonists of the formula XXI can be prepared as described in United States Prov. Application Serial No. 60/212,922 filed June 20, 2000.

Other NK-1 receptor antagonists that can be used, together with an anxiolytic or antidepressant agent in the pharmaceutical compositions and methods of this invention are those compounds and pharmaceutically acceptable salts described in the following references: European Patent Application EP 499,313, published August 19, 1992; European Patent Application EP 520,555, published December 30, 1992; European Patent Application EP 522,808, published January 13, 1993, European Patent Application EP 528,495, published February 24, 1993, PCT Patent Application WO 93/14084, published July 22, 1993, PCT Patent Application WO 93/01169, published January 21, 1993, PCT Patent Application WO 93/01165, published January 21, 1993, PCT Patent Application WO 93/01159, published January 21, 1993, PCT Patent Application WO 92/20661, published November 26, 1992, European Patent Application EP 517,589, published December 12, 1992, European Patent Application EP 428,434, published May 22, 1991, and European Patent Application EP 360,390, published March 28, 1990. All of the foregoing World Patent Applications designate the United States. The foregoing patents and patent applications are incorporated herein by reference in their entirety.

This invention relates both to methods of treating anxiety or depression in which the NK-1 receptor antagonist and the NK-3 antagonists, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the NK-1 receptor antagonist will be administered to an adult human in an amount ranging from about 0.05 to about 1500 mg per day, in single or divided doses, preferably from about 5 to about 200 mg/day. The compounds may be administered on a regimen of up to 6 times per day, preferably 1 to 4 times per day, especially 2 times per day and most especially once daily. A suitable dosage level for the NK-3 antagonists is about 0.005 to 1500 mg per day, preferably about 0.5 to 500 mg per day. The compounds may be administered on a regimen of up to 6 time per day, preferably 1 to 4 times per day, especially 2 time per day and most especially once daily. A suitable dosage level for the NK-3 antagonist is about 0.005. to 1500 mg per day, preferably about 0.5 to 500 mg per day. The compounds may be administered on a regimen of up to 6 times per day, preferably 1 to 4 times per day, especially 2 times per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

It will be appreciated that the amount of the NK-1 receptor antagonist and the NK-3 antagonists required for use in the treatment of depression or anxiety will vary not only with the particular compounds or compositions selected but also with the route of administration, the nature of the condition being treated, and the age and condition of the patient, and will ultimately be at the discretion of the patient's physician or pharmacist.

The NK-1 receptor antagonists, their pharmaceutically acceptable salts, and the antidepressant and anxiolytic agents and their pharmaceutically acceptable salts that are employed in the pharmaceutical compositions and methods of this invention are hereinafter also referred to as "therapeutic agents". The therapeutic agents can be administered via either the oral or parenteral route. Compositions containing both an NK-1 receptor antagonist and an NK-3 antagonist, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i.e.*, they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, *etc*. Moreover, oral pharmaceutical compositions can be suitably sweetened and/or flavored. In general, the therapeutic agents of this invention, when administered separately (*i.e.*, not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the NK-1 receptor antagonist and the NK-3 antagonist may be formulated in a single pharmaceutical composition or alternatively in individual pharmaceutical compositions for simultaneous, separate or sequential use in accordance with the present invention.

Preferably the compositions according to the present invention, which contain both an NK-1 receptor antagonist and a NK-3 antagonist as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.*, conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from 0.05 to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavoured syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of an NK-1 receptor antagonist or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g.*, Tween™ 20, 40, 60, 80 or 85) and other sorbitans (*e.g.*, Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™, Lipofundin ™ and Lipiphysan™. The therapeutic agent may be either dissolved in a pre-mixed emulsion composition or alternatively it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising an NK-1 receptor antagonist and an NK-3 antagonist or pharmaceutically acceptable salts of the same, which process comprises bringing an NK-1 receptor antagonist and the NK-3 antagonist or the pharmaceutically acceptable salts of one or both of these therapeutic agents into association with a pharmaceutically acceptable carrier or excipient.

The activity of the compounds of the present invention, as substance P antagonists, is determined by their ability to inhibit the binding of substance P at its receptor sites in CHO-cells which reveal NK-1 receptor or IM-9 cells employing radioactive ligands. The substance P antagonist activity of the herein described piperidine compounds is evaluated using the standard assay procedure described by M. A. Cascieri *et al*., as reported in The Journal of Immunology, 133, 3260 (1984). This method essentially involves determining the concentration of the individual compound required to reduce by 50% the amount of radiolabelled substance P ligands at their receptor sites in said isolated cow tissues or IM-9 cells, thereby affording characteristic IC₅₀ values for each compound tested. More specifically, inhibition of [³H]SP binding to human IM-9 cells by compounds is determined in assay buffer (50 mM Tris-HCI (ph 7.4), 1mM MnCl₂, 0.02% bovine serum albumin, bacitracin (40 µg/ml) leupeptin (4 µg/ml), chymostatin (2 µg/ml) and phosphoramidon (30 µg/ml). The reaction is initiated by the addition of cells to the assay buffer containing 0.56 nM [³H]SP and various concentrations of compounds (total volume; 0.5 ml) and allowed to incubate for 120 minutes at 4°C. Incubation is terminated by filtration onto GF/B filters (presoaked in 0.1% polyethylenimine for 2 hours). Nonspecific binding is defined as the radioactivity remaining in the presence of 1µM SP. The filters are placed into tubes and counted using a liquid scintillation counter.

When administered in combination, either as a single or as separate pharmaceutical composition(s), the CNS-penetrant NK-1 receptor antagonist and an NK-3 antagonist, are presented in a ratio which is consistent with the manifestation of the desired effect. In particular, the ratio by weight of the CNS-penetrant NK-1 receptor antagonist and the NK-3 antagonist will suitably be between 0.001 and 1 to 1000 to 1, and especially between 0.01 to 1 and 100 to 1.

As used herein the term "patient" includes animals of economic importance such as bovine, ovine, and porcine animals, especially those that produce meat, as well as domestic animals (*e.g.* cats and dogs), sports animals (*e.g.* horses), zoo animals, and humans, the latter being preferred.

As used herein, the term "CNS-penetrant" refers to NK-1 receptor antagonists which are able to inhibit NK-1 receptor agonist-induced foot-tapping in the gerbil as hereinafter defined.

Essentially, hind foot-tapping in the gerbil induced by infusion of the NK-1 receptor agonist, GR73632 (d Ala[L-Pro⁹,Me-Leu¹⁰] -substance P(7-1 1)), under anaesthesia, directly into the central ventricles is inhibited when a CNS-penetrant NK-I receptor antagonist is administered intravenously immediately prior to GR73632 challenge, wherein hind foot-tapping over a period of five minutes following recovery from the anaesthesia is inhibited with an ID₅₀≤3mg/kg, and preferably with an ID₅₀≤1mg/kg.

In an alternative method, the NK-1 receptor antagonist is administered orally, I hour prior to GR73632 challenge, wherein the foot-tapping over a period of five minutes following recovery from anaesthesia is inhibited with an ID₅₀≤30mg/kg, and preferably with an ID₅₀≤10mg/kg.

CNS-penetrant NK-1 receptor antagonists of use in the present invention are also effective in the attenuation of separation-induced vocalisations by guinea-pig pups as hereinafter defined.

Essentially, a vocalisation response in guinea-pig pups is induced by isolation from their mothers and littermates, which response is attenuated when a CNS-penetrant NK- I receptor antagonist is administered subcutaneously 30 minutes prior to isolation, wherein vocalisations during the first 15 minutes of isolation are attenuated with an ID₅₀≤20mg/kg, preferably with an ID₅₀≤10mg/kg, and especially with an ID₅₀≤5mg/kg.

In an alternative method, the NK-1 receptor antagonist is administered orally, 4 hours prior to isolation, wherein vocalisations during the first 15 minutes of isolation are attenuated with an ID₅₀≤20mg/kg, preferably with an ID₅₀≤10mg/kg, and especially with an ID₅₀≤5mg/kg.

## Claims

1. A pharmaceutical composition for the treatment of anxiety or depression in a mammal, comprising: (a) an NK-3 antagonist or a pharmaceutically acceptable salt thereof; (b) a CNS-penetrant NK-1 receptor antagonist or pharmaceutically acceptable salt thereof; and (c) a pharmaceutically acceptable carrier; wherein the active agents "a" and "b" above are present in amounts that render the composition effective in treating, respectively, anxiety or depression.

2. A pharmaceutical composition according to claim 1, wherein the NK-1 receptor antagonist or pharmaceutically acceptable salt thereof is selected from compounds of the formula I, as defined below, and their pharmaceutically acceptable salts: 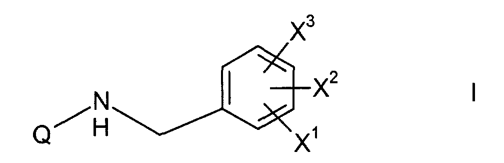 wherein
X¹ is hydrogen, (C₁-C₁₀)alkoxy optionally substituted with from one to three fluorine atoms or (C₁-C₁₀)alkyl optionally substituted with from one to three fluorine atoms;
X² and X³ are independently selected from hydrogen, halo, nitro, (C₁-C₁₀)alkyl optionally substituted with from one to three fluorine atoms, (C₁-C₁₀)alkoxy optionally substituted with from one to three fluorine atoms, trifluoromethyl, hydroxy, phenyl, cyano, amino, (C₁-C₆)alkylamino, di(C₁-C₆)alkylamino, -C(=O)NH(C₁-C₆)alkyl, (C₁-C₆)alkyl-C(=O)NH-(C₁-C₆)alkyl, hydroxy(C₁-C₄)alkyl, (C₁-C₄)alkoxy(C₁-C₄)alkyl, -NHC(=O)H and -NHC(=O)(C₁-C₆)alkyl; and
Q is a group of the formula 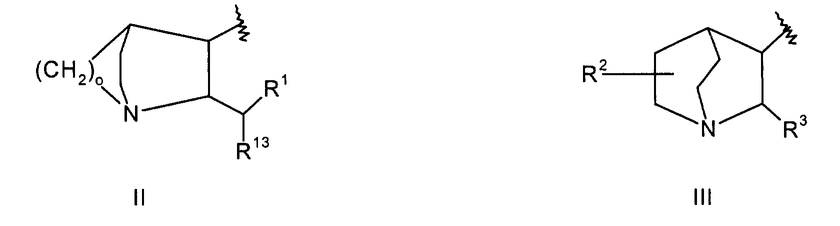 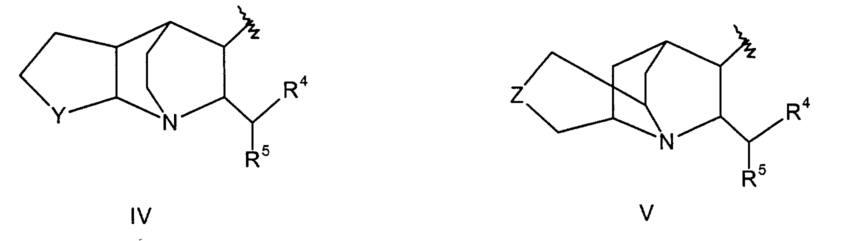 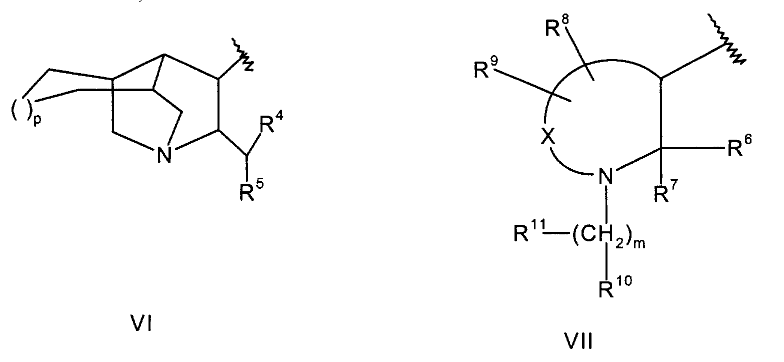 OR 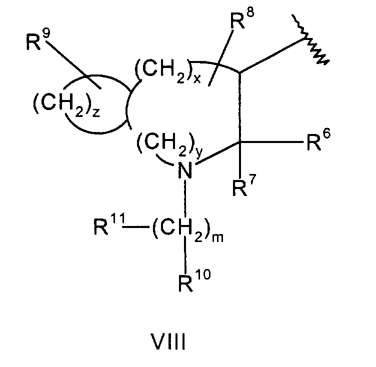 wherein
R¹ is a radical selected from furyl, thienyl, pyridyl, indolyl, biphenyl and phenyl optionally substituted with one or two substituents independently selected from halo, (C₁-C₁₀)alkyl optionally substituted with from one to three fluorine atoms, (C₁-C₁₀)alkoxy optionally substituted with from one to three fluorine atoms, carboxy, benzyloxycarbonyl and (C₁-C₃)alkoxycarbonyl;
R¹³ is selected from (C₃-C₄) branched alkyl, (C₅-C₆) branched alkenyl, (C₅-C₇)cycloalkyl, and the radicals named in the definition of R¹;
R² is hydrogen or (C₁-C₆)alkyl;
R³ is phenyl, biphenyl, naphthyl, pyridyl, benzhydryl, thienyl or furyl, and R³ may optionally be substituted with from one to three substituents independently selected from halo, (C₁-C₁₀)alkyl optionally substituted with from one to three fluorine atoms and (C₁-C₁₀)alkoxy optionally substituted with from one to three fluorine atoms;
Y is (CH₂)ₗ wherein I is an integer from one to three, or Y is a group of the formula 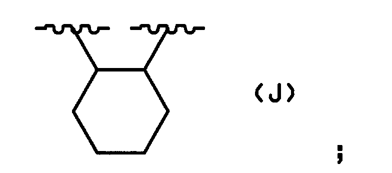
Z is oxygen, sulfur, amino, (C₁-C₃)alkylamino or (CH₂)ₙ wherein n is zero, one or two;
o is two or three;
p is zero or one;
R⁴ is furyl, thienyl, pyridyl, indolyl, biphenyl, or phenyl optionally substituted with one or two substituents independently selected from halo, (C₁-C₁₀)alkyl optionally substituted with from one to three fluorine atoms, (C₁-C₁₀)alkoxy optionally substituted with from one to three fluorine atoms, carboxy, (C₁-C₃)alkoxycarbonyl and benzyloxycarbonyl;
R⁵ is thienyl, biphenyl or phenyl optionally substituted with one or two substituents independently selected from halo, (C₁-C₁₀)alkyl optionally substituted with from one to three fluorine atoms and (C₁-C₁₀)alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
R⁶ is a radical selected from hydrogen, (C₁-C₆) straight or branched alkyl, (C₃-C₇)-cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl(C₂-C₆)alkyl, benzhydryl and benzyl, wherein each of said aryl and heteroaryl groups and the phenyl moieties of said benzyl, phenyl(C₂-C₆)alkyl and benzhydryl may optionally be substituted with one or more substituents independently selected from halo, nitro, (C₁-C₁₀)alkyl optionally substituted with from one to three fluorine atoms, (C₁-C₁₀)alkoxy optionally substituted with from one to three fluorine atoms, amino, hydroxy(C₁-C₆)alkyl, (C₁-C₆)alkoxy(C₁-C₆)alkyl, (C₁-C₆)-alkylamino, (C₁-C₆)alkyl-OC(=O)-, (C₁-C₆)alkyl-OC(=O)(C₁-C₆)alkyl, (C₁-C₆)alkyl-C(=O)-O-, (C₁-C₆)alkyl-C(=O)(C₁-C₆)alkyl-O-, (C₁-C₆)alkyl-C(=O)-, (C₁-C₆)alkyl-C(=O)(C₁-C₆)alkyl-, di(C₁-C₆)alkylamino, -C(=O)NH(C₁-C₆)alkyl, (C₁-C₆)alkyl-C(=O)NH(C₁-C₆)alkyl, -NHC(=O)H and -NHC(=O)(C₁-C₆) alkyl; and wherein one of the phenyl moieties of said benzhydryl may optionally be replaced by naphthyl, thienyl, furyl or pyridyl;
R⁷ is hydrogen, phenyl or (C₁-C₆)alkyl;
or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
R⁸ and R⁹ are each independently selected from hydrogen, hydroxy, halo, amino, oxo (=O), nitrile, hydroxy(C₁-C₆)alkyl, (C₁-C₆)alkoxy(C₁-C₆)alkyl, (C₁-C₆)alkylamino, di(C₁-C₆)alkylamino, (C₁-C₆)alkoxy, (C₁-C₆)alkyl-OC(=O)-, (C₁-C₆)alkyl-OC(=O)(C₁-C₆)alkyl,-(C₁-C₆)alkyl-C(=O)O-, (C₁-C₆)alkyl-C(=O)(C₁-C₆)alkyl-O-, (C₁-C₆)alkyl-C(=O)-, (C₁-C₆)alkyl-C(=O)(C₁-C₆)alkyl-, and the radicals set forth in the definition of R⁶;
R¹⁰ is NHCR¹², NHCH₂R¹², NHSO₂R¹² or one of the radicals set forth in any of the definitions of R⁶, R⁸ and R⁹;
R¹¹ is oximino (=NOH) or one of the radicals set forth in any of the definitions of R⁶, R⁸ and R⁹; and
R¹² is (C₁-C₆)alkyl, hydrogen, phenyl(C₁-C₆)alkyl or phenyl optionally substituted with (C₁-C₆)alkyl;
with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, and (d) when R⁸ and R⁹ are attached to the same carbon atom, then either each of R⁸ and R⁹ is independently selected from hydrogen, fluoro, (C₁-C₆)alkyl, hydroxy(C₁-C₆)alkyl and (C₁-C₆)alkoxy(C₁-C₆)alkyl, or R⁸ and R⁹, together with the carbon to which they are attached, form a (C₃-C₆) saturated carbocyclic ring that forms a spiro compound with the nitrogen-containing ring to which they are attached.

3. A pharmaceutical composition according to claim 1, wherein the NK-1 receptor antagonist or pharmaceutically acceptable salt thereof is selected from compounds of the formula IXa or IXb, as defined below, and their pharmaceutically acceptable salts: 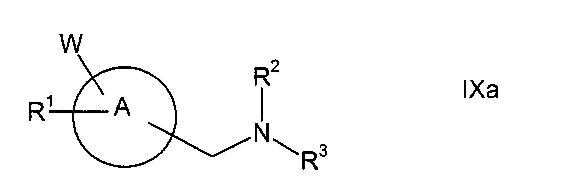 or 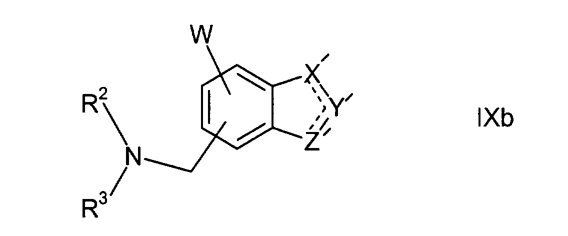 and their pharmaceutically acceptable salts, wherein A is a ring system selected from phenyl, naphthyl, thienyl, quinolinyl and indolinyl, and wherein the side chain containing NR²R³ is attached to a carbon atom of ring system A;
W is hydrogen, (C₁-C₆)alkyl optionally substituted with from one to three fluorine atoms,
-S(O)ᵥ(C₁-C₆)alkyl wherein v is zero, one or two, halo, benzyloxy or (C₁-C₆)alkoxy optionally substituted with from one to three fluorine atoms;
R¹ is a 4-, 5- or 6-membered heterocyclic ring containing from one to three heteroatoms selected from oxygen, nitrogen and sulfur (*e.g.* thiazolyl, azetidinyl, pyrrolyl, pyrazolyl, 1,2,3-triazolyl, 1,2,4-triazolyl, isothiazolyl, imidazolyl, isoxazolyl, oxazolyl, pyridyl,
pyrimidinyl, pyrazolyl or thiophenyl), wherein said heterocyclic ring may contain from zero to three double bonds and may optionally be substituted with one or more substituents, preferably one or two substituents, independently selected from (C₁-C₆)alkyl optionally substituted with from one to three fluorine atoms and (C₁-C₆)alkoxy optionally substituted with from one to three fluorine atoms;
the dotted lines in formula Ib indicate that one of the X'-Y' and Y'-Z' bonds may optionally be a double bond;
X' is selected from =CH-, -CH₂-, -O-, -S-, -SO-, -SO₂-, -N(R⁴)-, -NH-, =N-, -CH(C₁-C₆)alkyl-, =C(C₁-C₆)alky-, -CH(C₆H₅)- and =C(C₆H₅)-;
Y' is selected from C=O, C=NR⁴, C=S, =CH-, -CH₂-, =C(C₁-C₆)alkyl-,
-CH(C₁-C₆)alkyl-, =C(C₆H₅)-, -CH(C₆H₅)-, =N-, -NH-, -N(R⁴)-, =C(halo)-, =C(OR⁴)-, =C(SR⁴)-, =C(NR⁴)-, -O-, =C(CF₃)-, =C(CH₂C₆H₅)-, -S- and SO₂, wherein the phenyl moieties of said =C(C₆H₅)- and -CH(C6H5)- may optionally be substituted with from one to three substituents independently selected from trifluoromethyl and halo, and wherein the alkyl moieties of said =(C₁-C₆)alkyl- and -CH(C₁-C₆)alkyl- may optionally be substituted with from one to three fluorine atoms;
Z' is selected from =CH-, -CH₂-, =N-, -NH-, -S-, -N(R⁴)-, =C(C₆H₅)-, -CH(C₆H₅)-, =C(C₁-C₆)alkyl- and -CH(C₁-C₆)alkyl-;
or X', Y' and Z', together with the two carbon atoms shared between the benzo ring and the X'Y'Z' ring, form a fused pyridine or pyrimidine ring;
R² is hydrogen or -CO₂(C₁-C₁₀)alkyl;
R³ is selected from 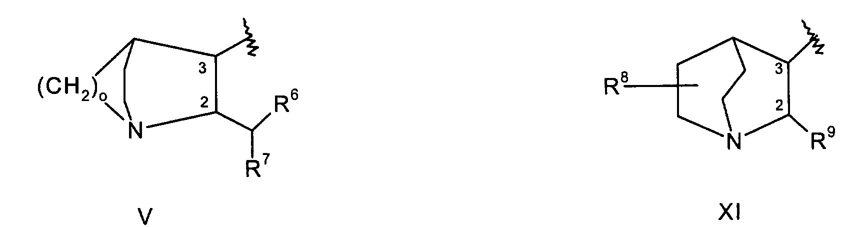 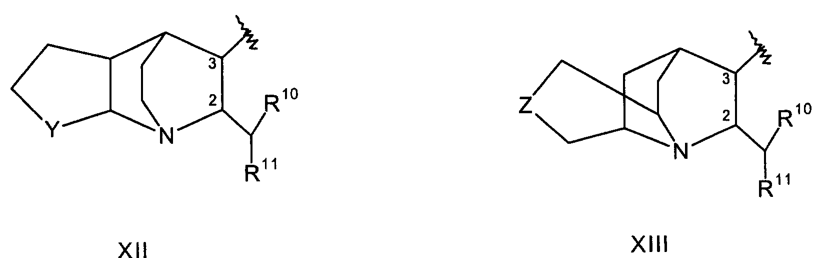 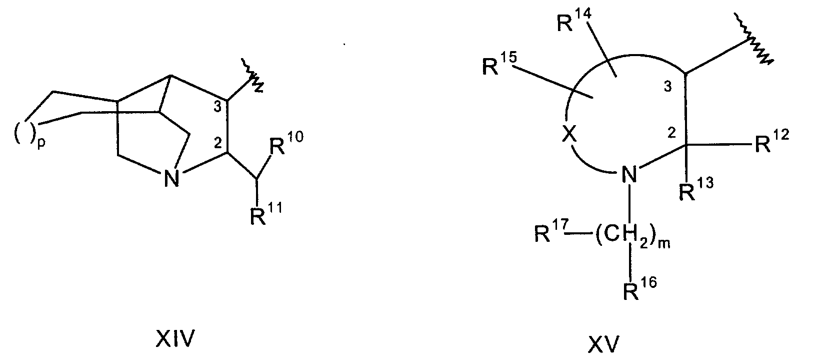 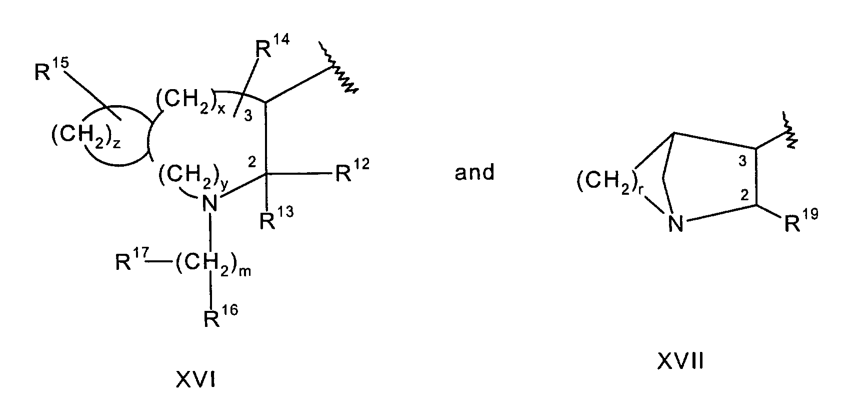
wherein R⁶ and R¹⁰ are independently selected from furyl, thienyl, pyridyl, indolyl, biphenyl and phenyl, wherein said phenyl may optionally be substituted with one or two substituents independently selected from halo, (C₁-C₁₀)alkyl optionally substituted with from one to three fluorine atoms, (C₁-C₁₀)alkoxy optionally substituted with from one to three fluorine atoms, carboxy, benzyloxycarbonyl and (C₁-C₃)alkoxycarbonyl;
R⁴ is (C₁-C₆)alkyl or phenyl;
R⁷ is selected from (C₃-C₄) branched alkyl, (C₅-C₆) branched alkenyl, (C₅-C₇)cycloalkyl, and the radicals named in the definition of R⁶;
R⁸ is hydrogen or (C₁-C₆)alkyl;
R⁹ and R¹⁹ are independently selected from phenyl, biphenyl, naphthyl, pyridyl, benzhydryl, thienyl and furyl, and R⁹ and R¹⁹ may optionally be substituted with from one to three substituents independently selected from halo, (C₁-C₁₀)alkyl optionally substituted with from one to three fluorine atoms and (C₁-C₁₀)alkoxy optionally substituted with from one to three fluorine atoms;
Y is (CH₂)ₗ wherein I is an integer from one to three, or Y is a group of the formula 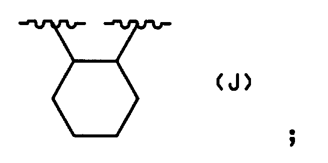
Z is oxygen, sulfur, amino, (C₁-C₃)alkylamino or (CH₂)ₙ wherein n is zero, one or two;
x is zero, one or two;
y is zero, one or two;
z is three, four or five;
o is two or three;
p is zero or one;
r is one, two or three;
the ring containing (CH₂)_{z} may contain from zero to three double bonds, and one of the carbon atoms of (CH₂)_{z} may optionally be replaced by oxygen, sulfur or nitrogen;
R¹¹ is thienyl, biphenyl or phenyl optionally substituted with one or two substituents independently selected from halo, (C₁-C₁₀)alkyl optionally substituted with from one to three fluorine atoms and (C₁-C₁₀)alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R¹⁴, and wherein any one of the carbon atoms of said (CH₂)_{q} may optionally be substituted with R¹⁵;
m is an integer from 0 to 8, and any one of the carbon-carbon single bonds of (CH₂)ₘ, wherein both carbon atoms of such bond are bonded to each other and to another carbon atom of the (CH₂)ₘ chain, may optionally be replaced by a carbon-carbon double bond or a carbon-carbon triple bond, and any one of the carbon atoms of said (CH₂)ₘ may optionally be substituted with R¹⁷;
R¹² is a radical selected from hydrogen, (C₁-C₆) straight or branched alkyl, (C₃-C₇)-cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl(C₂-C₆)alkyl, benzhydryl and benzyl, wherein the point of attachment on R¹² is a carbon atom unless R¹² is hydrogen, and wherein each of said aryl and heteroaryl groups and the phenyl moieties of said benzyl, phenyl(C₂-C₆)alkyl and benzhydryl may optionally be substituted with one or more substituents independently selected from halo, nitro, (C₁-C₁₀)alkyl optionally substituted with from one to three fluorine atoms, (C₁-C₁₀)alkoxy optionally substituted with from one to three fluorine atoms, amino, hydroxy(C₁-C₆)alkyl, (C₁-C₆)alkoxy(C₁-C₆)alkyl, (C₁-C₆)-alkylamino, (C₁-C₆)alkyl-OC(=O)-, (C₁-C₆)alkyl-OC(=O)(C₁-C₆)alkyl, (C₁-C₆)alkyl-C(=O)-O-, (C₁-C₆)alkyl-C(=O)(C₁-C₆)alkyl-O-, (C₁-C₆)alkyl-C(=O)-, (C₁-C₆)alkyl-, di(C₁-C₆)alkylamino, -C(=O)NH(C₁-C₆)alkyl, (C₁-C₆)alkyl-C(=O)NH(C₁-C₆)alkyl, -NHC(=O)H and -NHC(=O)(C₁-C₆)alkyl; and wherein one of the phenyl moieties of said benzhydryl may optionally be replaced by naphthyl, thienyl, furyl or pyridyl;
R¹³ is hydrogen, phenyl or (C₁-C₆)alkyl;
or R¹² and R¹³, together with the carbon to which they are attached, form a saturated carbocyclic ring having from 3 to 7 carbon atoms wherein one of said carbon atoms that is neither the point of attachment of the spiro ring nor adjacent to such point of attachment may optionally be replaced by oxygen, nitrogen or sulfur;
R¹⁴ and R¹⁵ are each independently selected from hydrogen, hydroxy, halo, amino, oxo (=O), cyano, hydroxy(C₁-C₆)alkyl, (C₁-C₆)alkoxy(C₁-C₆)alkyl, (C₁-C₆)alkylamino, di(C₁-C₆)alkylamino, (C₁-C₆)alkoxy, -C(=O)OH, (C₁-C₆)alkyl-OC(=O)-, (C₁-C₆)alkyl-OC(=O)-(C₁-C₆)alkyl, (C₁-C₆)alkyl-C(=O)O-, (C₁-C₆)alkyl-C(=O)(C₁-C₆)alkyl-O-, (C₁-C₆)alkyl-C(=O)-, (C₁-C₆)alkyl-C(=O)(C₁-C₆)alkyl-, and the radicals set forth in the definition of R¹²;
R¹⁶ is NHC(=O)R¹⁸, NHCH₂R¹⁸, SO₂R¹⁸, CO₂H or one of the radicals set forth in any of the definitions of R¹², R¹⁴ and R¹⁵;
R¹⁷ is oximino (=NOH) or one of the radicals set forth in any of the definitions of R¹², R¹⁴ and R¹⁵; and
R¹⁸ is (C₁-C₆)alkyl, hydrogen, phenyl or phenyl(C₁-C₆)alkyl;
with the proviso that (a) when m is 0, one of R¹⁶ and R¹⁷ is absent and the other is hydrogen, (b) when R³ is a group of the formula XVI, R¹⁴ and R¹⁵ cannot be attached to the same carbon atom, (c) when R¹⁴ and R¹⁵ are attached to the same carbon atom, then either each of R¹⁴ and R¹⁵ is independently selected from hydrogen, fluoro, (C₁-C₆)alkyl, hydroxy(C₁-C₆)alkyl and (C₁-C₆)alkoxy(C₁-C₆)alkyl, or R¹⁴ and R¹⁵, together with the carbon to which they are attached, form a (C₃-C₆) saturated carbocyclic ring that forms a spiro compound with the nitrogen-containing ring to which they are attached; (d) R¹² and R¹³ can not both be hydrogen, and (e) when R¹⁴ or R¹⁵ is attached to a carbon atom of X or (CH₂)_{y} that is adjacent to the ring nitrogen, then R¹⁴ or R¹⁵, respectively, must be a substituent wherein the point of attachment is a carbon atom.

4. A pharmaceutical composition according to claim 1, wherein the NK-1 receptor antagonist or pharmaceutically acceptable salt thereof is selected from compounds of the formula XVIII, as depicted and defined below, and their pharmaceutically acceptable salts: 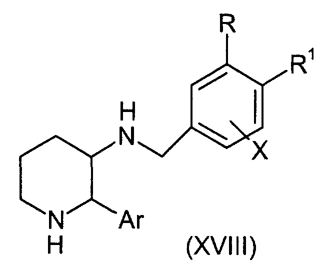 wherein
R is halo(C₁-C₈)alkyl, halo(C₂-C₈)alkenyl, halo(C₂-C₈)alkynyl or halo(C₁-C₈)alkyl substituted by hydroxy or (C₁-C₈)alkoxy; R¹ is hydrogen, halo or (C₁-C₆)alkoxy; or
R and R¹, together with the two carbon atoms shared between the benzene ring and the R and R¹, complete a fused (C₄-C₆)cycloalkyl wherein one carbon atom is optionally replaced by oxygen and wherein one or two of the carbon atoms are optionally substituted by up to five subtituents selected from halo, (C₁-C₆)alkyl and halo(C₁-C₆)alkyl;
X is (C₁-C₆)alkoxy, halo(C₁-C₆)alkoxy, phenoxy or halo; and
Ar is phenyl optionally substituted by halo.

5. A pharmaceutical composition according to claim 1, wherein the NK-1 receptor antagonist or pharmaceutically acceptable salt thereof is selected from compounds of the formula XIX, as depicted and defined below, and their pharmaceutically acceptable salts: 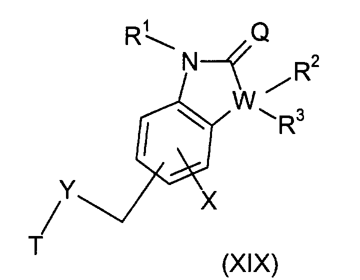 wherein
W is methylene, ethylene, propylene, vinylene, -CH₂O, -OCH₂-, -CH₂S- or -SCH₂-;
R¹, R² and R³ are independently hydrogen, (C₁-C₃)alkyl, (C₁-C₃)alkoxy or halo(C₁-C₃)-alkyl, provided that when W is methylene, both R² and R³ are not hydrogen;
X is halo, (C₁-C₃)alkoxy, (C₁-C₃)alkoxy or (C₁-C₃)alkenyl;
Y is imino or oxy;
Q is oxygen or sulfur; and
T is (2S,3S)-2-diphenylmethylquinuclidin-3-yl,(2S,3S)-2-phenylpiperdin-3-yl or (2S,3S)-2-diphenylmethyl-1-azanorbornan-3-yl.

6. A pharmaceutical composition according to claim 1, wherein the NK-1 receptor antagonist or pharmaceutically acceptable salt thereof is selected from compounds of the formula XX, as depicted and defined below, and their pharmaceutically acceptable salts: 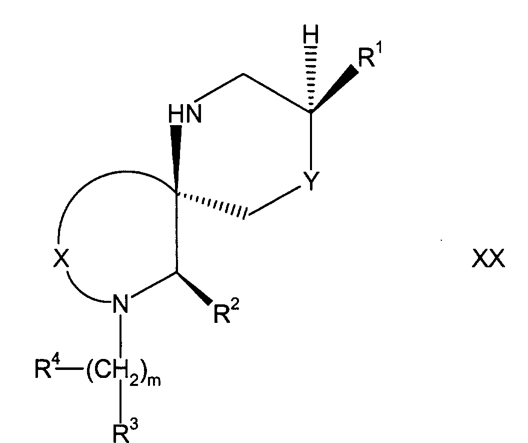 wherein
R¹ is phenyl optionally substituted with one or more substituents, preferably with from one to three substituents, independently selected from hydrogen, halo, nitro, (C₁-C₁₀)alkyl optionally substituted with from one to three fluorine atoms, (C₁-C₁₀)alkoxy optionally substituted with from one to three fluorine atoms, trifluoromethyl, hydroxy, phenyl, cyano, amino, (C₁-C₆)alkylamino, di(C₁-C₆)alkylamino, -C(=O)NH(C₁-C₆)alkyl, (C₁-C₆)alkyl-C(=O)NH(C₁-C₆) alkyl, hydroxy(C₁-C₄)alkyl, -NHC(=O)H, -NHC(=O)(C₁-C₆) alkyl, (C₁-C₄)alkoxy(C₁-C₄)alkyl, -S(O)ᵥ(C₁-C₁₀)-alkyl wherein v is zero, one or two, -S(O)ᵥ-aryl wherein v is zero, one or two, -O-aryl, -SO₂NR⁴R⁵ wherein each of R⁴ and R⁵ is, independently, (C₁-C₆)alkyl, or R⁴ and R⁵, together with the nitrogen to which they are attached, form a saturated ring containing one nitrogen and from 3 to 6 carbons, (SO₂-(C₁-C₁₀)alkyl)((C₁-C₁₀)alkyl)N wherein one or both of the alkyl moieties may optionally be substituted with from one to three fluorine atoms, -N(SO₂(C₁-C₁₀)alkyl)₂ and (SO₂-aryl) ((C₁-C₁₀)alkyl)N; and wherein the aryl moieties of said -S(O)ᵥ-aryl, -O-aryl and (SO₂-aryl) ((C₁-C₁₀)alkyl)N are independently selected from phenyl and benzyl and may optionally be substituted with from one to three substituents independently selected from (C₁-C₄)alkyl, (C₁-C₄)alkoxy and halo;
or R¹ is phenyl substituted with a group having the formula 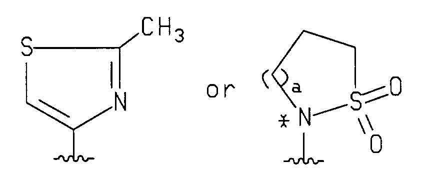
wherein a is 0, 1 or 2 and the asterisk represents a position meta to the point of attachment of R¹;
R² is selected from (C₁-C₆) straight or branched alkyl, (C₃-C₇)cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl(C₂-C₆)alkyl and benzhydryl may optionally be substituted with one or more substituents, preferably with from one to three substituents, independently selected from halo, nitro, (C₁-C₁₀)alkyl optionally substituted with from one to three fluorine atoms, (C₁-C₁₀)alkoxy optionally substituted with from one to three fluorine atoms, amino, hydroxy(C₁-C₆)alkyl, (C₁-C₆)alkoxy(C₁-C₆)alkyl, (C₁-C₆)-alkylamino, (C₁-C₆)alkyl-OC(=O)-, (C₁-C₆)alkyl-OC(=O)(C₁-C₆)alkyl, (C₁-C₆)alkyl-C(=O)-O-, (C₁-C₆)alkyl-C(=O)(C₁-C₆)alkyl-O-, (C₁-C₆)alkyl-C(=O)-, (C₁-C₆)alkyl-C(=O)(C₁-C₆)alkyl-, di(C₁-C₆)alkylamino, -C(=O)NH(C₁-C₆)alkyl, (C₁-C₆)alkyl-C(=O)NH(C₁-C₆)alkyl, -NHC(=O)H and -NHC(=O)(C₁-C₆)alkyl; and wherein one of the phenyl moieties of said benzhydryl may optionally be replaced by naphthyl, thienyl, furyl or pyridyl;
m is an integer from 0 to 8, and any one of the carbon-carbon single bonds of (CH₂)ₘ, wherein both carbon atoms of such bond are bonded to each other and to another carbon atom in the (CH₂)ₘ chain, may optionally be replaced by a carbon-carbon double bond or a carbon-carbon triple bond, and any one of the carbon atoms of said (CH₂)ₘ may optionally be substituted with R⁴;
R³ is selected from NHC(=O)R⁸, NHCH₂R⁸, SO₂R⁸, AR⁵, CO₂H and the radicals set forth in the definitions of R², R⁶ and R⁷;
A is CH₂, nitrogen, oxygen, sulfur or carbonyl;
R⁸ is (C₁-C₆)alkyl, hydrogen, phenyl or phenyl(C₁-C₆)alkyl;
R⁴ is selected from oximino (=NOH) and the radicals set forth in the definitions of R², R⁶ and R⁷;
R⁵ is a monocyclic or bicyclic heterocycle selected from the group consisting of pyrimidinyl, benzoxazolyl, 2,3-dihydro-3-oxobenzisosulfonazol-2-yl, morpholin-1-yl, thiomorpholin-1-yl, benzofuranyl, benzothienyl, indolyl, isoindolyl, isoquinolinyl, furyl, pyridyl, isothiazolyl, oxazolyl, triazolyl, tetrazolyl, quinolyl, thiazolyl, thienyl, and groups of the formulae 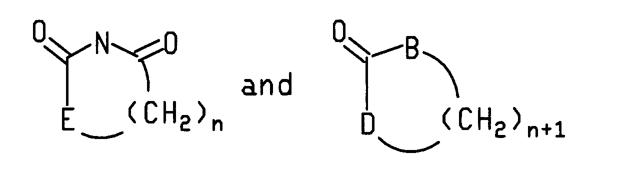
wherein B and D are selected from carbon, oxygen and nitrogen, and at least one of B and D is other than carbon; E is carbon or nitrogen; n is an integer from 1 to 5; any one of the carbon atoms of said (CH₂)ₙ and (CH₂)ₙ₊₁ may be optionally substituted with (C₁-C₆)alkyl or (C₂-C₆)spiroalkyl; and either any one pair of the carbon atoms of said (CH₂)ₙ and (CH₂)ₙ₊₁ may be bridged by a one or two carbon atom linkage, or any one pair of adjacent carbon atoms of said (CH₂)ₙ and (CH₂)ₙ₊₁ may form, together with from one to three carbon atoms that are not members of the carbonyl containing ring, a (C₃-C₅) fused carbocyclic ring;
X is (CH₂)_{q} wherein q is two or three and wherein one of the carbon-carbon single bonds in said (CH₂)_{q} may optionally be replaced by a carbon-carbon double bond, and wherein any one of the carbon atoms of said (CH₂)_{q} may optionally be substituted with R⁶, and wherein any one of the carbon atoms of said (CH₂)_{q} may optionally be substituted with R⁷;
R⁶ and R⁷ are independently selected from hydrogen, hydroxy, halo, amino, oxo (=O), cyano, hydroxy(C₁-C₆)alkyl, (C₁-C₆)alkoxy(C₁-C₆)alkyl, (C₁-C₆)alkylamino, di(C₁-C₆)alkylamino, (C₁-C₆)alkoxy, -C(=O)OH, (C₁-C₆)alkyl-OC(=O)-, (C₁-C₆)alkyl-OC(=O)-(C₁-C₆)alkyl, (C₁-C₆)alkyl-C(=O)O-, (C₁-C₆)alkyl-C(=O)(C₁-C₆)alkyl-O-, (C₁-C₆)alkyl-C(=O)-, (C₁-C₆)alkyl-C(=O)(C₁-C₆)alkyl- and the radicals set forth in the definition of R²; and
Y is (CH₂)_{z} wherein z is zero or one;
with the proviso that: (a) when A is -(CH₂)- or carbonyl, R⁵ cannot be furyl, pyridyl, isothiazolyl, oxazolyl, triazolyl, tetrazolyl, quinolyl, thiazolyl or thienyl; (b) when m is zero, one of R³ and R⁴ is absent and the other is hydrogen; (c) when R⁶ or R⁷ is attached to a carbon atom of X that is adjacent to the ring nitrogen, then R⁶ or R⁷, respectively, must be a substituent wherein the point of attachment is a carbon atom.

7. A pharmaceutical composition according to claim 1, wherein the NK-1 receptor antagonist or pharmaceutically acceptable salt thereof is selected from compounds of the formula XXI, as depicted and defined below, and their pharmaceutically acceptable salts: 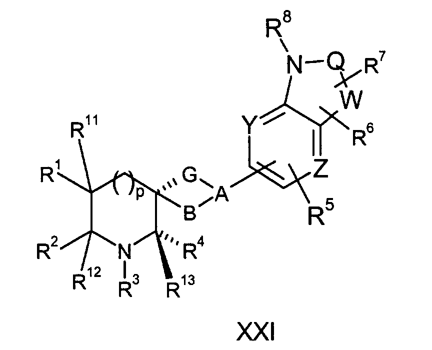
wherein Q is C=NH, C=CH₂, C=S, C=O, SO or SO₂;
A is CH, CH₂, C(C₁-C₆)alkyl, CH(C₁-C₆)alkyl, C(CF₃) or CH(CF₃), with the proviso that when B is present, A must be either CH, C(C₁-C₆)alkyl or C(CF₃);
B is absent or is methylene or ethylene;
each of Y and Z is N or CH, with the proviso that Y and Z can not both be N;
G is NH(CH₂)_{q}, S(CH₂)_{q} or O(CH₂)_{q}, wherein q is zero or one;
W is a one carbon linking group (*i.e*. methylene) or a saturated or unsaturated two or three carbon linking group, wherein each of the foregoing W groups can optionally be substituted with one substituent R⁷ or two substituents R⁷ and R⁶, or W is a one carbon linking group that forms, together with a 2-, 3-, 4- or 5-carbon chain, a 3-, 4-, 5- or 6-membered spiro ring, respectively;
or W is a saturated two carbon chain linking group that forms, together with a separate 1-, 2- or 3-carbon chain, a fused 3-, 4- or 5-membered ring, respectively;
or W is a saturated two carbon chain linking group, wherein one of the two carbons in the chain forms, together with a separate 2-, 3-, 4- or 5-carbon chain, a 3-, 4-, 5- or 6-membered spiro ring, respectively;
p is zero, one or two;
R³ is selected from hydrogen, COR⁹, CO₂R⁹, optionally substituted phenyl, optionally substituted heterocyclic rings, and optionally substituted (C₁-C₈)alkyl wherein one of the CH₂ groups of said (C₁₋C₈)alkyl may optionally be replaced with a sulfur, oxygen or carbonyl group and wherein said (C₁-C₈)alkyl can optionally be substituted with from one to three substituents, preferably with zero substituents or one substituent, independently selected from hydroxy, oxo, phenyl(C₁-C₃)alkoxy, phenyl, cyano, halo, optionally substituted heterocyclic rings, NR⁹COR¹⁰, NR⁹CO₂R¹⁰, CONR⁹R¹⁰, COR⁹, -CO₂R⁹, NR⁹R¹⁰, and (C₁-C₆)alkoxy optionally substituted with from one to seven fluorine atoms, preferably with from zero to three fluorine atoms;
and wherein the heterocyclic rings of R³ and the heterocyclic ring substituents on the alkyl groups of R³ are selected, independently, from 3- to 7-membered saturated or unsaturated monocyclic rings containing from 1 to 4 ring heteroatoms, and 8- to 12-membered saturated or unsaturated bicyclic rings containing from 1 to 4 ring heteroatoms, wherein said heteroatoms are selected independently from oxygen, nitrogen and sulfur, with the proviso that there cannot be two adjacent ring oxygen atoms or two adjacent ring sulfur atoms in either the monocyclic or bicyclic heterocyclic rings, and with the proviso that heterocyclic rings formed from NR⁹R¹⁰ or CONR⁹R¹⁰ must contain at least one nitrogen atom;
and wherein the heterocyclic rings of R³ and the heterocyclic ring substituents on the alkyl groups of R³ can optionally be substituted with one or more substituents, preferably with zero, one or two substituents, independently selected from oxo, hydroxy, thioxo, halo, cyano, phenyl, (CH₂)ₘNR⁹R¹⁰, NR⁹COR¹⁰, (CH₂)ₘOR⁹, wherein m is zero, one or two, and (C₁-C₆)alkyl optionally substituted with one or more substituents, preferably with from zero to two substituents, independently selected from halo, CF₃, methoxy and phenyl;
and wherein the phenyl groups of R³ and the phenyl substituents in the alkyl groups of R³ can optionally be substituted with one or more substitutents, preferably with from zero to two substituents, independently selected from the group consisting of halo, cyano, nitro, CF₃, (CH₂)ₘNR⁹R¹⁰, wherein m is zero, one or two, NR⁹COR¹⁰, NR⁹CO₂R¹⁰, CONR⁹R¹⁰, CO₂NR⁹R¹⁰, COR⁹, CO₂R⁹, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆)alkenyl optionally substituted with from one to seven fluorine atoms, preferably with from zero to three fluorine atoms;
each of R¹, R², R¹¹, R¹² and R¹³ are selected independently from hydrogen and (C₁-C₆)alkyl optionally substituted with one or more substituents, preferably with zero, one or two substituents, that are selected independently from hydroxy, oxo, (C₁-C₆)alkoxy and cyano;
or R¹ and R², together with the carbon atoms to which they are attached, or R² and R³, together with the carbon and nitrogen to which they are attached, respectively, form a 5- or 6-membered saturated heterocyclic ring containing one or two heteroatoms that are selected independently from nitrogen, oxygen and sulfur, with the proviso that said ring cannot contain two adjacent oxygen atoms or two adjacent sulfur atoms; or R¹ and R², together with the carbons to which they are attached, form a 5- or 6-membered, saturated or unsaturated carbocyclic ring, and wherein said heterocyclic and carbocyclic rings formed by R¹ and R² or by R² and R³ can be substituted with one or more substituents, preferably with zero substituents or one substituent, independently selected from halo, oxo, NR⁹R¹⁰, (C₁-C₆)alkyl optionally substituted with from one to seven fluorine atoms, preferably with from zero to three fluorine atoms, and (C₁-C₆)alkoxy optionally substituted with from one to seven fluorine atoms, preferably with from zero to three fluorine atoms;
or R¹² and R¹³, together with the carbon atoms to which they are attached, form a 5-or 6-membered saturated heterocyclic ring containing one or two heteroatoms that are selected, independently, from nitrogen, oxygen and sulfur, with the proviso that said ring cannot contain two adjacent oxygen atoms or two adjacent sulfur atoms, or R¹² and R¹³, together with the carbons to which they are attached, form a 5- or 6-membered, saturated or unsaturated carbocyclic ring, and wherein said heterocyclic and carbocyclic rings formed by R¹² and R¹³ can be substituted with one or more substituents, preferably with zero substituents or one substituent, independently selected from NR⁹R¹⁰, halo, phenyl-S-, phenyl-SO-, phenyl-SO₂-, oxo, (C₁₋C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms:
with the proviso that no more than one of R¹ and R², R² and R³, and R¹² and R¹³ can form a ring;
R⁴ is selected from phenyl, 2-, 3- or 4-pyridyl, 2- or 3-thienyl, and pyrimidyl, wherein R⁴ can be optionally substituted with one or more substituents, preferably with zero or one substituent, selected independently from halo, (C₁-C₆)alkyl optionally substituted with from one to seven fluorine atoms, preferably with from zero to three fluorine atoms, (C₁-C₆)alkoxy optionally substituted with from one to seven fluorine atoms, preferably with from zero to three fluorine atoms, and (C₂-C₆)alkenyl optionally substituted with from one to seven fluorine atoms, preferably with from zero to three fluorine atoms;
R⁵ and R⁸ are selected independently from hydrogen, -SO(C₁-C₆)alkyl, -SO₂(C₁-C₆)alkyl, -SO-aryl, -SO₂-aryl, CF₃, halo, phenyl, phenyl(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected independently from hydroxy, oxo, (C₁-C₆)alkoxy, phenyl(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹;
R⁶ and R⁷ are selected independently from -SO(C₁-C₆)alkyl, -SO₂(C₁-C₆)alkyl, -SO-aryl, -SO₂-aryl, CF₃, halo, phenyl, phenyl(C₁-C₂)alkyl, hydroxy, aryloxy, heteroaryloxy, pyridyl, tetrazolyl, oxazolyl, thiazolyl, (C₁-C₆)alkoxy optionally substituted with from one to seven fluorine atoms, preferably with from zero to three fluorine atoms, (C₁-C₆)alkyl optionally substituted with from one to seven fluorine atoms, preferably with from zero to three fluorine atoms, and (C₁-C₆)alkyl substituted with one or more substituents, preferably with from zero to two substituents selected independently from hydroxy, oxo, (C₁-C₆)alkoxy, phenyl(C₁-C₃)alkoxy, phenyl, cyano, chloro, bromo, iodo, NR⁹R¹⁰, NR⁹COR¹⁰, NR⁹CO₂R¹⁰, CONR⁹R¹⁰, COR⁹ and CO₂R⁹; each R⁹ and each R¹⁰ is selected independently from hydrogen, (C₁-C₆)alkyl, hydroxy(C₁-C₆)alkyl, phenyl and CF₃;
or R⁹ and R¹⁰, when R³ is NR⁹R¹⁰ or CONR⁹R¹⁰, can form, together with the nitrogen to which they are attached, an optionally substituted heterocyclic ring that contains at least one nitrogen atom;
and wherein the phenyl groups in the definition of R⁵, R⁶, R⁷ and R⁸ and the phenyl moiety of phenyl(C₁-C₂)alkyl in the definition of R⁵, R⁶, R⁷ and R⁸ can optionally be substituted with one or more substituents, preferably with from zero to two substituents, that are selected independently from halo, hydroxy, (C₁-C₆)alkoxy optionally substituted with from one to seven fluorine atoms, preferably with from zero to three fluorine atoms, and (C₁-C₆)alkyl optionally substituted with from one to seven fluorine atoms, preferably with from zero to three fluorine atoms;
with the proviso that: (a) R⁸ cannot be halo, hydroxy, cyano, aryloxy, heteroaryloxy, substituted or unsubstituted (C₁-C₆)alkoxy or methyl substituted with from one to three fluorine atoms; and (b) when Q is C=O or C=S, and Y and Z are both carbon, and W is a methylene, ethylene or propylene group that is optionally substituted with (C₁-C6)alkyl or fluoro substituted (C₁-C₆)alkyl, and all of R¹, R², R¹¹, R¹² and R¹³ are hydrogen, and R⁵, R⁶, R⁷, and R⁸ are selected from hydrogen, halo, (C₁-C₆)alkyl optionally substituted with from 1 to 7 fluorine atoms, (C₁-C₆) alkoxy optionally substituted with from 1 to 7 fluorine atoms, then R³ cannot be hydrogen.

8. A pharmaceutical composition comprising an NK-3 antagonist or a pharmaceutically acceptable salt thereof and a CNS-penetrant NK-1 receptor antagonist or pharmaceutically acceptable salt thereof for use in medicine.

9. Use of an NK-3 antagonist or a pharmaceutically acceptable salt thereof for the manufacture of a medicament combined with a CNS-penetrant NK-1 receptor antagonist or pharmaceutically acceptable salt thereof for the treatment of anxiety.

10. Use of a CNS-penetrant NK-1 receptor antagonist or pharmaceutically acceptable salt thereof for the manufacture of a medicament combined with an NK-3 antagonist or a pharmaceutically acceptable salt thereof for the treatment of depression.

11. A pharmaceutical composition according to claim 1 when the NK-3 antagonist or a pharmaceutically acceptable salt thereof that is employed in such a composition is selected from the group of quinoline derivatives consisting of:
(R,S)-N-(α-methylbenzyl)-2-phenylquinoline-4-carboxamide;
(+)-(S)-N-(α-methylbenzyl)-2-phenylquinoline-4-carboxamide;
(-)-(R)-N-(α-methylbenzyl)-2-phenylquinoline-4-carboxamide;
(R,S)-N-[α-methoxycarbonylbenzyl]-2-phenylquinoline-4 carboxamide;
(+)-(S)-N-[α-methoxycarbonylbenzyl]-2-phenylquinoline-4-carboxamide;
(-)-(R)-N-[α-methoxycarbonylbenzyl]-2-phenylquinoline-4-carboxamide;
(R,S)-N-[α-methoxycarbonylbenzyl]-7-methoxy-2-phenylquinoline-4-carboxamide;
(R,S)-N-[α-methoxycarbonylbenzyl]-7-hydroxy-2-phenylquinoline-4-carboxamide;
(R,S)-N-[α-carboxybenzyl]-7-methoxy-2-phenylquinoline-4-carboxamide, hydrochloride;
(R,S)-N-[α-methylaminocarbonylbenzyl]-2-phenylquinoline-4-carboxamide;
(R,S)-N-[α-methoxycarbonylbenzyl]-2-(2-thienyl)quinoline-4-carboxamide;
(R,S)-N-[α-methoxycarbonylbenzyl]-2-(2-furyl)quinoline-4-carboxamide;
(R,S)-N-[α-methoxycarbonylbenzyl]-2-(4-pyridyl)quinoline-4-carboxamide;
(R,S)-N-[α-methoxycarbonyl-2-thienylmethyl]-2-phenylquinoline-4-carboxamide;
(R,S)-N-[α-methoxycarbonylmethylbenzyl]-2-phenylquinoline-4-carboxamide;
(-)-(R)-N-[α-methoxycarbonyl-1,4-cyclohexadienylimethyl]-2-phenylquinoline-4-carboxamide;
(R,S)-N-[α-(l-hydroxyethyl)benzyl]-2-phenylquinoline-4-carboxamide (single diastereomer);
(R,S)-N-(α-ethylbenzyl)-3-methoxy-2-phenylquinoline-4-carboxamide;
(R,S)-N-(α-ethylbenzyl)-3-*n*-butyl-2-phenylquinoline-4-carboxamide;
(R,S)-N-[α-methoxycarbonylbenzyl]benzo-1,3-cycloheptadieno[1,2-b]quinoline-8-carboxamide;
(R,S)-N-(α-ethylbenzyl)-3-hexyl-2-phenylquinoline-4-carboxamide;
(-)-(S)-N-(α-ethylbenzyl)-3-methyl-2-phenylquinoline-4-carboxamide;
(+)-(R)-N-(α-ethylbenzyl)-3-methyl-2-phenylquinoline-4-carboxamide;
(R,S)-N-[α-methoxycarbonylbenzyl-2-(2-methoxyphenyl)quinoline-4-carboxamide;
(R,S)-N-(α-ethylbenzyl)-3-phenyl-2-phenylquinoline-4-carboxamide;
(R,S)-N-[α-methoxycarbonylbenzyl]-2-(2-fluorophenyl)quinoline-4-carboxamide;
(R,S)-N-[α-ethyl-3,4-dichlorobenzyl]-2-phenylquinoline-4-carboxamide;
(R,S)-N-[α-hydroxymethylbenzyl]-2-phenylquinoline-4-carboxamide;
(R,S)-N-(α-ethylbenzyl)-2-phenylquinoline-4-carboxamide;
(R,S)-N-[α-methoxycarbonylbenzyl]-3-methyl-2-phenylquinoline-4-carboxamide;
(R,S)-N-(α-ethylbenzyl)-3-methyl-2-phenylquinoline-4-carboxamide;
(R,S)-N-[α-methoxycarbonylbenzyl]-7-chloro-2-phenylquinoline-4-carboxamide;
(R,S)-N-[α-methoxycarbonylbenzyl]-6-methyl-2-phenylquinol ine-4-carboxamide;
(R,S)-N-[α-methoxymethylbenzyl]-2-phenylquinoline-4-carboxamide;
(R,S)-N-[α-methoxycarbonylbenzyl]-6-chloro-2-phenylquinoline-4-carboxamide;
(R,S)-N-[α-methoxycarbonylbenzyl]-3-ethyl-2-phenylquinoline-4-carboxamide;
(R,S)-N-(α-*n*-propylbenzyl)-2-phenylquinoline-4-carboxamide;
(R,S)-N-(α-ethylbenzyl)-3-ethyl-2-phenylquinoline-4-carboxamide;
(R,S)-N-(α-ethylbenzyl)-3-phthamido-2-phenylquinoline-4-carboxamide;
(R,S)-N-(α-ethylbenzyl)-3-*n*-propyl-2-phenylquinoline-4-carboxamide;
(-)-(S)-N-(α-ethylbenzyl)-6-bromo-3-methyl-2-(4-bromophenyl)quinoline-4-carboxamide;
(-)-(S)-N-(α-ethylbenzyl)-6-bromo-3-methyl-2-phenylquinoline-4-carboxamide;
(R,S)-N-[α-methoxycarbonylbenzyl]-6-methoxy-2-phenylquinoline-4-carboxamide;
(R,S)-N-[α-methoxycarbonylbenzyl]-2-(2-benzofuryl)quinoline-4-carboxamide;
(R,S)-N-[(1,2-diphenyl)ethyl]-2-phenylquinoline-4-carboxamide;
(R,S)-N-(α-trifluoromethylbenzyl)-2-phenylquinoline-4-carboxamide;
(-)-(S)-N-(α-ethylbenzyl)-3-methoxy-2-phenylquinoline-4-carboxamide;
(-)-(S)-N-(α-ethylbenzyl)-3-ethyl-2-phenylquinoline-4-carboxamide;
(R,S)-N-(α-ethyl)-4-chlorobenzyl]-2-phenylquinoline-4-carboxamide;
(R,S)-N-[α-methoxycarbonylbenzyl]-N-methyl-2-phenylquinoline-4-carboxamide;
(R,S)-N-[α-methoxycarbonylbenzyl]-2-(3-thienyl)quinoline-4-carboxamide;
(R,S)-N-[α-methoxycarbonylbenzyl]-5,6-dihydrobenzo[a]acridine-7-carboxamide;
(R,S)-N-[α-methoxycarbonylbenzyl]-2-(2-pyrryl)quinoline-4-carboxamide;
(R,S)-N-[(α-methoxycarbonylbenzyl]-2-(2-thiazolyl)quinoline-4-carboxamide;
(R,S)-N-(1-indanyl)-2-phenylquinoline-4-carboxamide;
(R,S)-N-(α-*n*-butylbenzyl)-2-phenylquinoline-4-carboxamide;
(R,S)-N-[α-methoxycarbonylbenzyl]-2-(4-methylphenyl)quinoline-4-carboxamide;
(R,S)-N-(α-heptylbenzyl)-2-phenylquinoline-4-carboxamide;
(R,S)-N-[α-methoxycarbonylbenzyl]-2-(2-methylphenyl)quinoline-4-carboxamide;
(R,S)-N-[α-methoxycarbonylbenzyl]-2-(4-methoxyphenyl)quinoline-4-carboxamide; N-(1-phenylcyclopentyl)-2-phenylquinoline-4-carboxamide;
(R,S)-N-[α-methoxycarbonylbenzyl]-2-(4-hydroxyphenyl)quinoline-4-carboxamide;
(R,S)-N-[α-methoxycarbonylbenzyl]-2-(3,4-methylendioxyphenyl)quinoline-4-carboxamide;
N-(α,α-dimethylbenzyl)-2-phenylquinoline-4-carboxamide;
(R,S)-N-[α-ethyl-4-methylbenzyl]-2-phenylquinoline-4-carboxamide;
(R,S)-N-[α-methoxycarbonylbenzyl]-2-(3-pyrryl)quinoline-4-carboxamide;
(R,S)-N-[α-methoxycarbonylbenzyl]-2-(3,4-dichlorohenyl)quinoline-4-carboxamide;
(-)-(R)-N-[α-aminomethylbenzyl]-2-phenylquinoline-4-carboxamide;
(-)-(S)-N-(α-ethylbenzyl)-3-amino-2-phenylquinoline-4-carboxamide;
(-)-(S)-N-(α-ethylbenzyl)-3-chloro-2-phenylquinoline-4-carboxamide;
(-)-(S)-N-(α-ethylbenzyl)-3-bromo-2-phenylquinoline-4-carboxamide;
(R,S)-N-(α-isopropylbenzyl)-2-phenylquinoline-4-carboxamide;
(-)-(S)-N-(α-ethylbenzyl)-2-phenylquinoline-4-carboxamide;
(+)-(R)-N-(α-ethylbenzyl)-2-phenylquinoline-4-carboxamide;
(R,S)-N-[α-methoxycarbonylbenzyl]-6-fluoro-2-phenylquinoline-4-carboxamide;
(R,S)-N-[α-methoxycarbonylbenzyl]-2-cyclohexylquinoline-4-carboxamide;
(R,S)-N-[α-methoxycarbonylbenzyl]-2-(3-chlorophenyl)quinoline-4-carboxamide;
(R,S)-N-[α-methoxycarbonylbenzyl]-2-(2-chlorophenyl)quinoline-4-carboxamide;
(R,S)-N-[α-ethylbenzyl)-3-hydroxy-2-phenylquinoline-4-carboxamide;
(R,S)-N-[α-methoxycarbonylbenzyl]-8-acetyloxy-2-phenylquinoline-4-carboxamide;
(R,S)-N-[α-methoxycarbonylbenzyl]-8-hydroxy-2-phenylquinoline-4-carboxamide;
(R,S)-N-[α-methoxycarbonylbenzyl]-2-(2,4-dichlorophenyl)quinoline-4-carboxamide;
(-)-(R)-N-[α-methoxycarbonyl-4-hydroxybenzyl]-2-phenylquinoline-4-carboxamide, hydrochloride;
N-diphenylmethyl-2-phenylquinoline-4-carboxamide;
(-)-(S)-N-(α-ethylbenzyl)-3-hydroxy-2-phenylquinoline-4-carboxamide;
(+)-(R)-N-(α-ethylbenzyl)-3-hydroxy-2-phenylquinoline-4-carboxamide;
(-)-(R)-N-[α-methoxycarbonylbenzyl]-3-hydroxy-2-phenylquinoline-4-carboxamide;
(-)-(R)-N-[α-dimethylaminomethylbenzyl]-2-phenylquinoline-4-carboxamide;
(R,S)-N-[α-dimethylaminocarbonylbenzyl]-2-phenylquinoline-4-carboxamide;
(R,S)-N-[α-aminocarbonylbenzyl]-2-phenylquinoline-4-carboxamide;
(R,S)-N-[α-(1-pyrrolidinylcarbonyl)benzyl]-2-phenylquinoline-4-carboxamide;
(-)-(R)-N-[α-carboxybenzyl]-2-phenylquinoline-4-carboxamide, hydrochloride;
(R,S)-N-[α-methoxycarbonylbenzyl]-2-(4-chlorophenyl)quinoline-4-carboxamide;
(R)-N-[α-methoxycarbonyl-4-methoxybenzyl]-2-phenylquinoline-4-carboxamide;
(R,S)-N-[α-methoxycarbonyl-α-methylbenzyl]-N-methyl-2-phenylquinoline-4-carboxamide, hydrochloride;
(R,S)-N-[α-methylcarbonylbenzyl]-2-phenylquinoline-4-carboxamide;
(R,S)-N-[α-(2-hydroxyethyl)benzyl]-2-phenylquinoline-4-carboxamide;
(-)-(S)-N-(α-ethylbenzyl)-3-(2-dimethylaminoethoxy)-2-phenylquinoline-4-carboxamide, hydrochloride;
(-)-(S)-N-(α-ethylbenzyl)-3-acetylamino-2-phenylquinoline-4-carboxamide;
(-)-(S)-N-(α-ethylbenzyl)-3-(3-dimethylinopropoxy)-2-phenylquinoline-4-carboxamide, hydrochloride;
(-)-(S)-N-(α-ethylbenzyl)-3-[2-(1-phthaloyl)ethoxy-2-phenylquinoline-4-carboxamide,
hydrochloride;
(-)-(S)-N-(α-ethylbenzyl)-3-(2-aminoethoxy)-2-phenylquinoline-4-carboxamide, hydrochloride;
(+)-(S)-N-(α-ethylbenzyl)-3-[2-(1-pyrrolidinyl)ethoxy-2-phenylquinoline-4-carboxamide, hydrochloride;
(-)-(S)-N-(α-ethylbenzyl)-3-dimethylaminoacetylamino-2-phenylquinoline-4-carboxamide;
N-(α,α-dimethylbenzyl)-3-hydroxy-2-phenylquinoline-4-carboxamide;
N-(α,α-dimethylbenzyl)-3-amino-2-phenylquinoline-4-carboxamide;
(-)-(S)-N-(α-ethylbenzyl)-5-methyl-2-phenylquinoline-4-carboxamide;
(R,S)-N-[α-(1-hydroxyethyl)benzyl]-3-methyl-2-phenylquinoline-4-carboxamide;
(R,S)-N-[α-methylcarbonylbenzyl]-3-methyl-2-phenylquinoline-4-carboxamide;
(R,S)-N-[α-ethyl-4-pyridylmethyl]-2-phenylquinoline-4-carboxamide;
(R,S)-N-[α-ethyl-2-thienylmethyl]-2-phenylquinoline-4-carboxamide;
(+)-(S)-N-(α-ethylbenzyl)-3-dimethylaminomethyl-2-phenylquinoline-4-carboxamide, hydrochloride;
(S)-N-(α-ethylbenzyl)-3-methyl-7-methoxy-2-phenylquinoline-4-carboxamide;
(S)-N-(α-ethylbenzyl)-3-amino-5-methyl-2-phenylquinoline-4-carboxamide;
(S)-N-(α-ethylbenzyl)-3-methoxy-5-methyl-2-phenylquinoline-4-carboxamide;
(-)-(S)-N-(α-ethylbenzyl)-3-hydroxy-2-phenylquinoline-4-carboxamide;
(-)-(S)-N-(α-ethylbenzyl)-3-hydroxy-2-phenylquinoline-4-carboxamide, hydrochloride; N-(2-methoxycarbonylphenyl)-2-phenyl)-2-phenylquinoline-4-carboxamide;
(R,S)-N-[1-methoxycarbonyl-2-phenylethyl]-2-phenylquinoline-4-carboxamide;
4-[N-benzylaminomethyl]-7-methoxy-2-phenylquinoline, dihydrochloride;
N-benzyl-7-hydroxy-2-phenylquinoline-4-carboxamide;
N-(3,4-dimethoxybenzyl)-7-methoxy-2-phenylquinoline-4-carboxamide;
N-[3,5-bis(trifluoromethyl)benzyl]-7-methoxy-2-phenylquinoline-4-carboxamide;
N-(2-pyridylmethyl)-7-methoxy-2-phenylquinoline-4-carboxamide;
N-benzyl-2-phenylquinoline-4-carboxamide;
N-phenyl-7-methoxy-2-phenylquinoline-4-carboxamide;
N-(2-methoxybenzyl)-7-methoxy-2-phenylquinoline-4-carboxamide;
N-phenethyl-7-methoxy-2-phenylquinoline-4-carboxamide;
N-benzyl-7-methoxy-2-phenylquinoline-4-carboxamide;
(±)-(R)-N-[1-methoxycarbonyl-2-methyl)propyl]-2-phenylquinoline-4-carboxamide;
N-benzyl-3-methyl-2-phenylquinoline-4-carboxamide;
(R,S)-N-(α-methylbenzyl)-2-phenylquinoline-4-carboxamide;
(+)-(S)-N-(α-methylbenzyl)-2-phenylquinoline-4-carboxamide;
(-)-(R)-N-(α-methylbenzyl)-2-phenylquinoline-4-carboxamide;
(R,S)-N-[α-methoxycarbonylbenzyl]-2-phenylquinoline-4-carboxamide;
(+)-(S)-N-[α-methoxycarbonylbenzyl]-2-phenylquinoline-4-carboxamide;
(-)-(R)-N-[α-methoxycarbonylbenzyl]-2-phenylquinoline-4-carboxamide;
(R,S)-N-[α-methoxycarbonylbenzyl]-7-methoxy-2-phenylquinoline-4-carboxamide;
(R,S)-N-[α-methoxycarbonylbenzyl]-7-hydroxy-2-phenylquinoline-4-carboxamide;
(R,S)-N-[α-carboxybenzyl]-7-methoxy-2-phenylquinoline-4-carboxamide, hydrochloride;
(R,S)-N-[α-methylaminocarbonylbenzyl]-2-phenylquinoline-4-carboxamide:
(R,S)-N-[α-methoxycarbonylbenzyl]-2-(2-thienyl)quinoline-4-carboxamide;
(R,S)-N-[α-methoxycarbonylbenzyl]-2-(2-furyl)quinoline-4-carboxamide;
(R,S)-N-[α-methoxycarbonylbenzyl]-2-(4-pyridyl)quinoline-4-carboxamide;
(R,S)-N-(α-methoxycarbonyl-2-thienylmethyl]-2-phenylquinoline-4-carboxamide;
(R,S)-N-[α-methoxycarbonylmethylbenzyl]-2-phenylquinoline-4-carboxamide;
(-)-(R)-N-[α-methoxycarbonyl-1,4-cyclohexadienylmethyl]-2-phenylquinoline-4-carboxamide;
(R,S)-N-[α-(1-hydroxyethyl)benzyl]-2-phenylquinoline-4-carboxamide (single diastereomer);
(R,S)-N-(α-ethylbenzyl)-3-methoxy-2-phenylquinoline-4-carboxamide;
(R,S)-N-(α-ethylbenzyl)-3-*n*-butyl-2-phenylquinoline-4-carboxamide;
(R,S)-N-[α-methoxycarbonylbenzyl]benzo-1,3-cycloheptadieno[1,2-b]quinoline-8-carboxamide;
(R,S)-N-(α-ethylbenzyl)-3-hexyl-2-phenylquinoline-4-carboxamide;
(-)-(S)-N-(α-ethylbenzyl)-3-methyl-2-phenylquinoline-4-carboxam ide;
(+)-(R)-N-(α-ethylbenzyl)-3-methyl-2-phenylquinoline-4-carboxamide;
(R,S)-N-[α-methoxycarbonylbenzyl]-2-(2-methoxyphenyl)quinoline-4-carboxamide;
(R,S)-N-(α-ethylbenzyl)-3-phenyl-2-phenylquinoline-4-carboxamide;
(R,S)-N-[α-methoxycarbonylbenzyl-2-(2-fluorophenyl)quinoline-4-carboxamide;
(R,S)-N-[α-ethyl-3,4-dichlorobenzyl-2-phenylquinoline-4-carboxamide;
(R,S)-N-[α-hydroxymethylbenzyl]-2-phenylquinoline-4-carboxamide;
(R,S)-N-(α-ethylbenzyl)-2-phenylquinoline-4-carboxamide;
(R,S)-N-[α-methoxycarbonylbenzyl]-3-methyl-2-phenylquinoline-4-carboxamide;
(R,S)-N-(α-ethylbenzyl)-3-methyl-2-phenylquinoline-4-carboxamide;
(R,S)-N-[α-methoxycarbonylbenzyl]-7-chloro-2-phenylquinoline-4-carboxamide;
(R,S)-N-[α-methoxycarbonylbenzyl]-6-methyl-2-phenylquinoline-4-carboxamide;
(R,S)-N-[α-methoxymethylbenzyl)-2-phenylquinoline-4-carboxamide;
(R,S)-N-[α-methoxycarbonylbenzyl]-6-chloro-2-phenylquinol ine-4-carboxamide;
(R,S)-N-[α-methoxycarbonylbenzyl]-3-ethyl-2-phenylquinoline-4-carboxamide;
(R,S)-N-(α-*n*-propylbenzyl)-2-phenylquinoline-4-carboxamide;
(R,S)-N-(α-ethylbenzyl)-3-ethyl-2-phenylquinoline-4-carboxamide;
(R,S)-N-(α-ethylbenzyl)-3-phthalimido-2-phenylquinoline-4-carboxamide;
(R,S)-N-(α-ethylbenzyl)-3-n-propyl-2-phenylquinoline-4-carboxamide;
(-)-S)-N-(α-ethylbenzyl)-6-bromo-3-methyl-2-(4-bromophenyl)quinoline-4-carboxamide;
(-)-(S)-N-(α-ethylbenzyl )-6-bromo-3-methyl-2-phenylquinoline-4-carboxamide;
(R,S)-N-[α-methoxycarbonylbenzyl]-6-methoxy-2-phenylquinoline-4-carboxamide;
(R,S)-N-[α-methoxycarbonylbenzyl]-2-(2-benzofuryl)quinoline-4-carboxamide;
(R,S)-N-(1,2-diphenylethyl)-2-phenylquinoline-4-carboxamide;
(R,S)-N-(α-trifluoromethylbenzyl)-2-phenylquinoline-4-carboxamide;
(-)-(S)-N-(α-ethylbenzyl)-3-methoxy-2-phenylquinoline-4-carboxamide;
(-)-(S)-N-(α-ethylbenzyl)-3-ethyl-2-phenylquinoline-4-carboxamide;
(R,S)-N-[α-ethyl-4-chlorobenzyl]-2-phenylquinoline-4-carboxamide;
(R,S)-N-[α-methoxycarbonylbenzyl]-N-methyl-2-phenylquinoline-4-carboxamide;
(R,S)-N-[α-methoxycarbonylbenzyl]-2-(3-thienyl)quinoline-4-carboxamide;
(R,S)-N-[α-methoxycarbonylbenzyl]-5,6-dihydrobenzo[a]acridine-7-carboxamide;
(R,S)-N-[α-methoxycarbonylbenzyl]-2-(2-pyrryl)quinoline-4-carboxamide;
(R,S)-N-[α-methoxycarbonylbenzyl]-2-(2-thiazolyl)quinoline-4-carboxamide;
(R,S)-N-(1-indanyl)-2-phenylquinoline-4-carboxamide;
(R,S)-N-(α-*n*-butylbenzyl)-2-phenylquinoline-4-carboxamide;
(R,S)-N-[α-methoxycarbonylbenzyl]-2-(4-methylphenyl)quinoline-4-carboxamide;
(R,S)-N-(α-heptylbenzyl)-2-phenylquinoline-4-carboxamide;
(R,S)-N-[α-methoxycarbonylbenzyl]-2-(2-methylphenyl)quinoline-4-carboxamide;
(R,S)-N-[α-methoxycarbonylbenzyl]-2-(4-methoxypheno)quinoline-4-carboxamide;
N-(1-phenylcyclopentyl)-2-phenylquinoline-4-carboxamide;
(R,S)-N-[α-methoxycarbonylbenzyl]-2-(4-hydroxyphenyl)quinoline-4-carboxamide;
(R,S)-N-[α-methoxycarbonylbenzyl]-2-(3,4-methylendioxyphenyl)quinoline-4-carboxamide;
N-(α,α-dimethylbenzyl)-2-phenylquinoline-4-carboxamide;
(R,S)-N-[α-ethyl-4-methylbenzyl]-2-phenylquinoline-4-carboxamide;
(R,S)-N-[α-methoxycarbonylbenzyl]-2-(3-pyrryl)quinoline-4-carboxamide;
(R,S)-N-[α-methoxycarbonylbenzyl]-2-(3,4-dichloropheno)quinoline-4-carboxamide;
(-)-(R)-N-[α-aminomethylbenzyl]-2-phenylquinoline-4-carboxamide;
(-)-(S)-N-(α-ethobenzyl)-3-amino-2-phenylquinoline-4-carboxamide;
(-)-(S)-N-(α-ethylbenzyl)-3-chloro-2-phenylquinoline-4-carboxamide;
(-)-(S)-N-(α-ethylbenzyl)-3-bromo-2-phenylquinoline-4-carboxamide;
(R,S)-N-(α-isopropylbenzyl)-2-phenylquinoline-4-carboxamide;
(-)-(S)-N-(α-ethylbenzyl)-2-phenylquinoline-4-carboxamide;
(+)-(R)-N-(α-ethylbenzyl)-2-phenylquinoline-4-carboxamide;
(R,S)-N-[α-methoxycarbonylbenzyl]-6-fluoro-2-phenylquinoline-4-carboxamide;
(R,S)-N-[α-methoxycarbonylbenzyl]-2-cyclohexylquinoline-4-carboxamide;
(R,S)-N-[α-methoxycarbonylbenzyl]-2-(3-chlorophenyl)quinoline-4-carboxamide;
(R,S)-N-[α-methoxycarbonylbenzyl]-2-(2-chlorophenyl)quinoline-4-carboxamide;
(R,S)-N-(α-ethylbenzyl)-3-hydroxy-2-phenylquinoline-4-carboxamide;
(R,S)-N-[α-methoxycarbonylbenzyl]-8-acetyloxy-2-phenylquinoline-4-carboxamide;
(R,S)-N-[α-methoxycarbonylbenzyl]-hydroxy-2-phenylquinoline-4-carboxamide;
(R,S)-N-[α-methoxycarbonylbenzyl]-2-(2,4-dichlorophenyl)quinoline-4-carboxamide;
(-)-(R)-N-[α-methoxycarbonyl-4-hydroxybenzyl]-2-phenylquinoline-4-carboxamide, hydrochloride;
N-diphenylmethyl-2-phenylquinoline-4-carboxamide;
(-)-(S)-N-(α-ethylbenzyl)-3-hydroxy-2-phenylquinoline-4-carboxamide;
(+)-(R)-N-(α-ethylbenzyl)-3-hydroxy-2-phenylquinoline-4-carboxamide;
(-)-(R)-N-[α-methoxycarbonylbenzyl]-3-hydroxy-2-phenylquinoline-4-carboxamide;
(-)-(R)-N-[α-dimethylaminomethylbenzyl]-2-phenylquinoline-4-carboxamide;
(R,S)-N-[α-dimethylaminocarbonylbenzyl]-2-phenylquinoline-4-carboxamide;
(R,S)-N-[α-aminocarbonylbenzyl]-2-phenylquinoline-4-carboxamide;
(R,S)-N-[α-(1-pyrrolidinylcarbonyl)benzyl]-2-phenylquinoline-4-carboxamide;
(-)-(R)-N-[α-carboxybenzyl-2-phenylquinoline-4-carboxamide, hydrochloride;
(R,S)-N-[α-methoxycarbonylbenzyl]-2-(4-chlorophenyl)quinoline-4-carboxamide:
(R)-N-[α-methoxycarbonyl-4-methoxybenzyl]-2-phenylquinoline-4-carboxamide;
(R,S)-N-[α-methoxycarbonyl-a-methylbenzyl]-N-methyl-2-phenylquinoline-4-carboxamide, hydrochloride;
(R,S)-N-[α-methylcarbonylbenzyl]-2-phenylquinoline-4-carboxamide;
(R,S)-N-[α-(2-hydroxyethyl)benzyl]-2-phenylquinoline-4-carboxamide;
(-)-(S)-N-(α-ethylbenzyl)-3-(3-dimethylaminopropoxy)-2-phenylquinoline-4-carboxamide, hydrochloride;
(-)-(S)-N-(α-ethylbenzyl)-3-[2-(1-phthaloyl)ethoxy]-2-phenylquinoline-4-carboxamide, hydrochloride;
(-)-(S)-N-(α-ethylbenzyl)-3-(2-aminoethoxy)-2-phenylquinoline-4-carboxamide, hydrochloride;
(+)-(S)-N-(α-ethylbenzyl)-3-[2-(1-pyrrolidinyl)ethoxy]-2-phenylquinoline-4-carboxamide, hydrochloride;
(-)-(S)-N-(α-ethylbenzyl)-3-dimethylaminoacetylamino-2-phenylquinoline-4-carboxamide;
N-(α,α-dimethylbenzyl)-3-hydroxy-2-phenylquinoline-4-carboxamide;
N-(α,α-dimethylbenzyl)-3-amino-2-phenylquinoline-4-carboxamide;
(-)-(S)-N-(α-ethylbenzyl)-S-methyl-2-phenylquinoline-4-carboxamide;
(R,S)-N-[α-(1-hydroxyethyl)benzyl-3-methyl-2-phenylquinoline-4-carboxamide;
(R,S)-N-[α-methylcarbonylbenzyl]-3-methyl-2-phenylquinoline-4-carboxamide;
(R,S)-N-[α-ethyl-4-pyridylmethyl]-2-phenylquinoline-4-carboxamine;
(R,S)-N-[α-ethyl-2-thienylmethyl]-2-phenylquinoline-4-carboxamide;
(+)-(S)-N-(α-ethylbenzyl)-3-dimethylaminomethyl-2-phenylquinoline-4-carboxamide, hydrochloride;
(S)-N-(α-ethylbenzyl)-3-methyl-7-methoxy-2-phenylquinoline-4-carboxamide;
(S)-N-(α-ethylbenzyl)-3-amino-5-methyl-2-phenylquinoline-4-carboxamide;
(S)-N-(α-ethylbenzyl)-3-methoxy-5-methyl-2-phenylquinoline-4-carboxamide;
(S)-N-(α-ethylbenzyl)-3-[(S)-2-(methoxycarbonyl)pyrrolidin-1-yl]-2-phenylquinoline-4-carboxamide, hydrochloride;
(S)-N-(α-ethylbenzyl)-3-[(S)-2-(hydroxymethyl)pyrrolidin-1-yl]-2-phenylquinoline-4-carboxamide;
(S)-N-(α-ethylbenzyl)-3-[(S)-2-carboxypyrrolidine-1-yl]-2-phenylquinoline-4-carboxamide;
(S)-N-(α-ethylbenzyl)-3-[4-oxopiperidin-1-ylmethyl]-2-phenylquinoline-4-carboxamide;
(S)-N-(α-ethylbenzyl)-3-[4-hydroxypiperidin-1-ylmethyl]-2-phenylquinoline-4-carboxamide;
(S)-N-(α-ethylbenzyl)-3-[piperazin-1-ylmethyl]-2-phenylquinoline-4-carboxamide; and
(S)-N-(α-ethylbenzyl)-3-[(3-oxo)pyrrolidin-1-ylmethyl]-2-phenylquinoline-4-carboxamide.

12. The pharmaceutical composition according to Claim 1 wherein the NK-3 antagonist or a pharmaceutically acceptable salt that is employed in such a composition is selected from the group of piperidine derivatives consisting of
1 -Benzoyl-3-(3,4-dichlorophenyl)-3-[3-[4-(pyrrolidin-1 -yl)carbonyl)piperid-1-yl]propyl]piperidine;
1-Benzoyl-3-(3,4-dichlorophenyl)-3-[3-(4-piperidinopiperid-1-yl)propyl]piperidine;
1-Benzoyl-3-(3,4-dichlorophenyl)-3-[3-(4-carbamoyl-4-piperidinopiperid-1-yl)propyl]piperidine;
3-[3-[4-(Acryloyl-N-methylamino)-4-phenylpiperid-1-yl]propyl]-1-benzoyl-3-(3,4-dichlorophenyl)piperidine;
3-[3-(4-(2-Aminothiazol-4-yl)-4-phenylpiperid-1-yl]propyl]-1-benzoyl-3-(3,4-dichlorophenyl)piperidine;
3-[3-(4-Acetyl-4-benzylpiperid-1-yl)propyl]-1-benzoyl-3-(3,4-dichlorophenyl) piperidine;
3-[3-[4-Acetylamino-4-benzylpiperid-1-yl]propyl]-1benzoyl-3-(3,4-dichlorophenyl) piperidine;
1-Benzoyl-3-[3-[4-benzyl-4-propionylaminomethylpiperid-1-yl]propyl]-3-(3,4-dichlorophenyl)piperidine;
1-Benzoyl-3-[3-[4-benzyl-4-ethoxycarbonylaminopiperid-1-yl]propyl],-3-(3,4-dichlorophenyl)piperidine;
1-Benzoyl-3-[3-[4-benzyl-4-pyrrolidin-1-ylcarbonylpiperid-1-yl]propyl]-3-(3,4-dichlorophenyl)piperidine;
1-Benzoyl-3-(3,4-dichlorophenyl)-3-[3-[4-dimethylaminocarbonyl-4-phenylpiperid-1-yl]propyl]perhydroazepine;
1-Benzoyl-3-(3,4-dichlorophenyl)-3-[3-[4-(2-hydroxyethoxy)-4-phenylpiperid-1-yl]propyl]piperidine;
3-[3-[4-(2-Acetyloxyethoxy)-4-phenylpiperid-1-yl]propyl]-1-benzoyl-3-(3,4-dichlorophenyl)piperidine;
1-Benzoyl-3-(3,4-dichlorophenyl)-3-[3-[4-(2-furoylamino)-4-phenylpiperid-1-yl]propyl]piperidine;
1-Benzoyl-3-(3,4-dichlorophenyl)-3-[3-[4-(2-thenoylamino)-4-phenylpiperid-1-yl]propyl]piperidine;
3-(3,4-Dichlorophenyl)-1-isonicotinoyl-3-[3-[4-phenyl-4-(pyrrolidin-1-ylcarbonyl)-piperid-1-yl]propyl]piperidine;
1-Benzoyl-3-(3,4-dichlorophenyl)-3-[3-spiro(indoline-3,4'-piperid-1-yl)propyl]piperidine;
1-Benzoyl-3-(3,4-dichlorophenyl)-3-[3-(1-acetyl)-spiro(indoline-3,4'-piperid-1'-yl)propyl] piperidine;
3-(3,4-Dichlorophenyl)-3-[3-[4-phenyl-4-pyrrolidin-1-ylcarbonylpiperid-1-yl]propyl]-1-(2-thenoyl)piperidine;
3-(3,4-Dichlorophenyl)-3-[3-[4-phenyl-4-pyrrolidin-1-yllcarbonylpiperid-1-yl]propyl]-1-(3-thenoyl)piperidine;
3-(3,4-Dichlorophenyl)-1-(2-furoyl)-3-[3-[4-phenyl-4-pyrrolidin-1 -ylcarbonylpiperid-1-yl]propyl]piperidine;
3-(3,4-Dichlorophenyl)-1-(3-furoyl)-3-[3-[4-phenyl-4-pyrrolidin-1-ylcarbonylpiperid-1-yl]propyl]piperidine;
3-[3-[4-(2-Amino-1,3,4-oxadiazol-5-yl)-4-phenylpiperid-1 -yl]propyl]-1-benzoyl-3-(3,4-dichlorophenyl)piperidine;
1-Benzoyl-3-(3,4-dichlorophenyl)-3-[3-[4-ethoxalylamino-4-phenylpiperid-1-yl]propyl]piperidine;
1-Benzoyl-3-(3,4-dichlorophenyl)-3-[3-(4-carbamoyl-4-morpholinopiperid-1-yl)propyl]piperidine;
1-Benzoyl-3-(3,4-dichlorophenyl)-3-[3-[1-methoxycarbonyl-spiro(indoline-3,4'-piperid-1'-yl)]propyl]piperidine;
1-Benzoyl-3-(3,4-dichlorophenyl)-3-[3-(1-(N,N-dimethylcarbamoyl)-spiro(indoline-3,4'-piperid-1'-yl)]propyl]piperidine;
1-Benzoyl-3-(3,4-dichlorophenyl)-3-(3-[1-methanesulfonyl-spiro(indoline-3,4'-piperid-1'-yl)]propyl]piperidine;
(R)-{3-(3,4-Dichlorophenyl)-3-[3-(4-phenylpiperidin-1-yl)-propyl}piperidin-1-yl)phenyl-methanone, hydrochloride;
(S)-{3-(3,4-Dichlorophenyl)-3-[3-(4-hydroxy-4-phenylpiperidin-1-yl)propyl}piperidin-1-yl)-phenylmethanone, hydrochloride;
(S)-[3-[3-(4-Benzenesulfonylmethyl-4-hydroxypiperidin-1-yl)propyl]-3-(3,4-dichloro-phenyl)piperidin-1-yl]phenylmethanone, hydrochloride;
(R)-(3-(3,4-Dichlorophenyl)-3-[3-(4-hydroxy-4-phenylpiperidin-1-yl)propyl]piperidin-1-yl}naphthalene-2-ylmethanone;
(R)-{3-(3,4-Dichlorophenyl)-3-[3-(4-hydroxy-4-phenyl-piperidin-1-yl)propyl]piperidin-1-yl)pyridin-4-ylmethanone;
N-(1-{3-[3-(3,4-Dichlorophenyl)-I-(1H-imidazole-2-carbonyl)piperidin-3-yl]propyl)-4-phenylpiperidin-4-yl)acetamide;
(R)-[3-(3,4-Dichlorophenyl)-3-(3-(4-hydroxy-4-phenylpiperidin-1-yl)propyl]piperidin-1-yl}phenylmethanone;
(R)-(3-(3,4-Dichlorophenyl)-3-[3-(4-phenylpiperidin-1-yl)propyl]piperidin-1-yl)phenylmethanone;
(3-(3,4-Dichlorophenyl)-3-[3-[4-(4-fluorophenyl)-4-hydroxypiperidin-1-yl]propyl)piperidin-1-yl)phenylmethanone;
[3-[3-(4-Benzenesulfonylmethyl-4-hydroxypiperidin-1-yl)propyl]-3-(3,4-dichlorophenyl)piperidin-1-yl]phenylmethanone;
(3-(4-Fluorophenyl)-3-[3-(4-hydroxy-4-phenylpiperidin-1-yl)propyl]piperidin-1-yl)phenylmethanone;
(3-(3,4-Dimethoxyphenyl)-3-[3-(4-hydroxy-4-phenylpiperidin-1-yl)propyl]piperidin-1-yl]phenylmethanone;
(3-(3,4-Dimethylphenyl)-3-[3-(4-hydroxy-4-phenylpiperidin-1-yl)propyl]piperidin-1-yl)phenylmethanone;
(3-[3-(4-Hydroxy-4-phenylpiperidin-1-yl)-propyl]-3-(3,4,5-trichlorophenyl)piperidin-1-yl]phenylmethanone;
(3-(3,4-Dichlorophenyl)-3-[4-(4-hydroxy-4-phenylpiperidin-1-yl)-butyl]piperidin-1-yl)-phenylmethanone; and
{3-(3,4-Dichlorophenyl)-3-[6-(4-hydroxy-4-phenylpiperidin-1-yl)-hexyl]piperidin-1-yl)phenylmethanone.

13. A pharmaceutical composition according to claim 1 wherein the NK-3 antagonist or a pharmaceutically acceptable salt thereof that is employed in such a composition is selected from the group of ureidopiperidines consisting of
Benzenesulfonate of 4-phenyl-4-ureidopiperidine;
*p*-Toluenesulfonate of 4-(N',N'-diethylureido)-4-phenylpiperidine;
*p*-Toluenesulfonate of 4-(N',N'-diethylureido)-4-phenylpiperidine; and
N-methyl-N-(4-phenylpiperidin-4-yl)pyrrolidine-1-carboxamide.

14. The pharmaceutical composition according to Claim 1 wherein the NK-3 antagonist or a pharmaceutically acceptable salt thereof that is employed in such a composition is selected from the group of piperazine derivatives consisting of
2-(3,4-Dichlorophenyl)piperazine-2(R)-(3,4-dichlorophenyl)-4-(3,5-dimethylbenzoyl)-1-[[[2-phenylmethylazabicyclo[2.2.1]heptan-5-yl]amino]acetyl]piperazine;
N-[2-[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]-2-oxo-1-phenylethyl]acetamide;
(±)-1,1-Dimethylethyl-4-[[2-[2-(3,4-dichlorophenyl)-1-(3,5-dimethylbenzoyl)-1-piperazinyl]-2-oxoethyl]amino]-1-piperidine carboxylate;
(-)-1,1-Dimethylethyl-4-[[2-[2(R)-(3,4-dichlorophenyl)-1-(3,5-dimethylbenzoyl)-1-piperazinyl]-2-oxoethyl]amino]-1-piperidinecarboxylate;
(±)-2-(3,4-Dichlorophenyl)-4-[3,5-dimethylbenzoyl]-1-[(4-piperidinylamino)acetyl]piperazine, dihydrochloride;
(-)-2(R)-(3,4-Dichlorophenyl)-4-[3,5-dimethylbenzoyl]-1-[(4-piperidinylamino)acetyl]piperazine, dihydrochloride;
5-(1-Cyanoimino)-1-methylthio]-2-[3-[2(R)-(3,4-dichlorophenyl)-4-(3,5-dimethylbenzoyl)-1-piperazinyl]-3-oxopropyl-2,5-diazabicyclo[2.2.1]heptane;
5-[1-(Cyanoimino)-1-phenylaminomethyl]-2-[3-[2(R)-(3,4-dichlorophenyl)-4-(3,5-dimethylbenzoyl)-1-piperazinyl]-3-oxopropyl-2,5-diazabicyclo[2.2.1]heptane;
5-[1-(Cyanoimino)-1-phenylmethylaminomethyl]-2-[3-[2(R)-(3,4-dichlorophenyl)-4-(3,5-dimethylbenzoyl)-1-piperazinyl]-3-oxopropyl-2,5-diazabicyclo[2.2.1]heptane;
2(R)-(3,4-Dichlorophenyl)-4-(3,5-dimethylbenzoyl)-1-[[[6-phenylmethyl-2-azabicyclo[2.2.2]octan-6-yl]methylamino]acetyl]piperazine;
2(R)-(3,4-Dichlorophenyl)-4-(3,5-dimethylbenzoyl)-1-[[[6-phenylmethyl-6-azabicyclo[3.2.2]nonan-3-yl]amino]acetyl]piperazine;
Methyl [1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenyl]carbamate;
N-[1(R)-[(5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenylethyl]-N'-methylurea;
5-[3-[2(R)-(3,4-Dichlorophenyl)-4-(3,5-dimethylbenzoyl)-1-piperazinyl]-3-oxopropyl]-2-2(R)-[Methylaminocarbonylamino-1-oxo-3-(2-thienyl)propyl]-1(S),4(S)-2,5-diazabicyclo[2.2.1]heptane;
2-[3-[2-(R)-(3,4-Dichlorophenyl)-4-(3,5-dimethylbenzyoyl)-1-piperazinyl]-3-oxopropyl]-5-[2-iminomethylaminomethylamino-1-oxo-3-phenylpropyl]-1(S),4(S)-2,5-diazobicyclo[2.2.1]heptane;
5-[3-[2(R)-(3,4-Dichlorophenyl)-4-(3,5-dimethylbenzoyl)-1-piperazinyl]-3-oxopropyl]-2-(2(R)-hydroxy-1-oxo-3-phenylpropyl)-1(S),4(S)-2,5-diazabicyclo-[2.2.1]heptane;
5-[3-[2(R)-(3,4-Dichlorophenyl)-4-(3,5-dimethylbenzoyl)-1-piperazinyl]-3-oxopropyl]-2-(2(S)-hydroxy-1-oxo-3-phenylpropyl)-1(S),4(S)-2,5-diazabicyclo-[2.2.1]heptane;
2-[2(S)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[22.1]heptane;
2-[2(R)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
2-[2(R)-2-(Aminohydroxyimino)ethoyl]-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
[1(R)-[[5-(3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate;
[1(S)-[[5-[3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate;
2-[2(S)-Methoxy-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
(1R,4R)-1,1-Dimethylethyl-5-hydroxy-2-azabicyclo[2.2.1]heptane-2-carboxylate and (1S,4S)-1,1-dimethylethyl-5-hydroxy-2-azabicyclo[2.2.1]heptane-2-carboxylate;
*Exo*-1,1-dimethylethyl-5-[2-[2(R)-(3,4-dichlorophenyl)-4-(3,5-dimethylbenzoyl)-1-piperazinyl]-2-oxoethoxy]-1-(R),4(R)-2-azabicyclo[2.2.1]heptane-2-carboxylate;
2(R)-(3,4-Dichlorophenyl)-4-(3,5-dimethylbenzoyl)-1-[[[*exo*-1(R),4(R)-2-azabicyclo[2.2.1]heptan-5-yl]oxy]acetyl]piperazine;
2(R)-(3,4-Dichlorophenyl)-4-(3,5-dimethylbenzoyl)-1-[[[*exo*-2-(3-thienylmethyl)-1(S),4(S)-2-azabicyclo[2.2.1]heptan-5-yl)oxy]acetyl]piperazine;
*Exo*-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[*exo*-8-aza-[3.2.1]octan-3-yl]oxy]acetyl]piperazine, hydrochloride (enantiomer B);
*Endo*-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[*endo*-8-aza-[3.2.1]octan-3-yl]oxy]acetyl]piperazine, hydrochloride (enantiomer);
N-1(R)-[[5-[3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylsulfonamide (enantiomer);
2-[2(R)-Cyanomethylamino-1-oxo-3-phenylpropyl]-5-[3-[2-(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
2-[2(R)-[[2-Aminohydroxyiminoethyl]amino]-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
(±)-2(R)-(3,4-Dichlorophenyl)-4-(3,5-dimethylbenzoyl)-1-[[[*endo*-2-[(3,5-dimethyl-4-isoxazoyl)methyl]-2-azabicyclo[2.2.1]heptan-5-yl]amino]acetyl]piperazine;
1,1-Dimethylethyl[1(R)-[[*endo*-5-[[2-[2(R)-(3,4-dichlorophenyl)-4-(3,5-dimethylbenzoyl)-1-piperazinyl]-2-oxoethyl]amino]-2-azabicyclo[2.2.1]heptan-2-yl]carbonyl]-2-phenylethyl]carbamate;
*Endo*-2-(2(R)-amino-1-oxo-3-phenylpropyl)-5-[[2-[2(R)-(3,4-dichlorophenyl)-4-(3,5-dimethylbenzoyl)-1-piperazinyl]-2-oxoethyl]amino]-2-aza[2.2.1]heptane, dihydrochloride;
2-(R)-(3,4-Dichlorophenyl)-4-(3,5-dimethylbenzoyl)-1-[[[8-azabicyclo-[3.2.1]octan-3-yl]endo-amino]acetyl]piperazine, hydrochloride;
2-(R)-(3,4-Dichlorophenyl)-4-(3,5-dimethylbenzoyl)-1-[[[8-azabicyclo-[3.2.1]octan-3-yl]exo-amino]acetyl]piperazine, hydrochloride;
1,1-Dimethylethyl-[1(S)-[[*exo*-3-[[2-2(R)-(3,4-dichlorophenyl)-4-(3,5-dimethy-lbenzoyl)-1-piperazinyl]-2-oxoethyl]amino]-8-aza[3.2.1]octan-8-yl]carbonyl]-2-phenylethyl]carbamate (enantiomer);
*Exo*-8-(2(S)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
1,1-Dimethylethyl-[1(S)-[[*endo*-3-[[2-2(R)-(3,4-dichlorophenyl)-4-(3,5-dimethylbenzoyl)-1-piperazinyl]-2-oxoethyl]amino]-8-aza[3.2.1 ]octan-8-yl]carbonyl]-2-phenylethyl]carbamate (enantiomer);
*Endo*-8-(2(S)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
1,1-Dimethylethyl-[1(R)-[[*exo*-3-[[2-2(R)-(3,4-dichlorophenyl)-4-(3,5-dimethylbenzoyl)-1-piperazinyl]-2-oxoethyl]amino]-8-aza[3.2.1]octan-8-yl]carbonyl]-2-phenylethyl]carbamate (enantiomer);
*Exo*-8-(2(R)-amino-1-oxo-3-phenylpropyl)-3-[[2-[2(R)-(3,4-dichlorophenyl)-4-(3,5-dimethylbenzoyl)-1-piperazinyl]-2-oxoethyl]amino]-8-aza[3.2.1]octane, dihydrochloride (enantiomer);
(±)-N-[4-[[*Endo*-5-[[2-[2(R)-(3,4-dichlorophenyl)-4-(3,5-dimethyl-benzoyl)-1-piperazinyl]-2-oxoethyl]amino]-2-azabicyclo[2.2.1]heptan-2-yl]methyl]phenyl]acetamide;
(±)-N-[3-[[*Endo*-5-[[2-[2(R)-(3,4-dichlorophenyl)-4-(3,5-dimethyl-benzoyl)-1-piperazinyl]-2-oxoethyl]amino]-2-azabicyclo[2.2.1]heptan-2-yl]methyl]phenyl]acetamide;
(±)-1,1-Dimethylethyl-*trans*-2-[[5-[3-[2(R)-(3,4-dichlorophenyl)-4-(3,5-dimethylbenzoyl)-1-piperazinyl]-3-oxopropyl]-1(S),4(S)-2,5-diaza-bicyclo-[2.2.1]heptan-2-yl]carbonyl]-3-phenyl-1-azetidinecarboxylate (enantiomer);
(±)-5-[3-[2(R)-(3,4-Dichlorophenyl)-4-(3,5-dimethylbenzoyl)-1-piperazinyl]-3-oxopropyl]-2-[*trans*-3-phenyl-2-azetidinylcarbonyl]-1(S),4(S)-2,5-diazabicyclo[2.2.1]heptane, dihydrochloride;
(±)-3,5-Dimethylbenzoyl-3-(3,4-dichlorophenyl)piperazine;
(±)Bromoacetyl-2-(3,4-dichlorophenyl)-4-(3,5-dimethylbenzoyl)piperazine;
(±)-3,5-Dimethylbenzoyl-3-(3,4-dichlorophenyl)piperazine;
(±)-Bromoacetyl-2-(3,4-dichlorophenyl)-4-(3,5-dimethylbenzoyl)piperazine;
(±)-2-(3,4-dichlorophenyl)-4-(3,5-dimethylbenzoyl)-1-[[[1-[1-oxo-2-phenyl)ethyl]-4-piperidinyl]amino]acetyl]piperazine;
(±)-1,1-Dimethylethyl-4-[[2-[2-(3,4-dichlorophenyl)-1-(3,5-dimethylbenzoyl)-1-piperazinyl]-2-oxoethyl]amino]-1-piperidinecarboxylate;
(±)-1,1-Dimethylethyl-4-[[2-[2-(3,4-dichlorophenyl)-1-(3,5-dimethylbenzoyl)-1-piperazinyl]-2-oxoethyl]amino]-1-piperidinecarboxylate;
(±)-2-(3,4-Dichlorophenyl)-4-[3,5-dimethylbenzoyl]-1-[(4-piperidinylamino)acetyl]piperazine, dihydrochloride;
(+)-1-Benzoyl-4-[[2-]2-(3,4-dichlorophenyl)-4-(3,5-dimethylbenzoyl)-1-piperazinyl]-2-oxoethyl]amino]piperidine;
(±)-2-(3,4-Dichlorophenyl)-4-(3,5-dimethylbenzoyl)-1-[[[1-(2-oxo-2-phenylethyl)-4-piperidinyl]amino]acetyl]piperazine;
(±)-2-(3,4-Dichlorophenyl)-4-(3,5-dimethylbenzoyl)-1-[[[1-(3-phenylpropyl)-4-piperidinyl]amino]acetyl]piperazine;
(±)-N-[4-[[[-[[[2-[2-(3,4-dichlorophenyl)-4-(3,5-dimethylbenzoyl)-1-piperazinyl]-2-oxoethyl]amino]-1-piperidinyl]methyl]-2-thiazoyl]acetamide;
(±)-2-(3,4-Dichlorophenyl)-4-[3, 5-dimethylbenzoyl]-1-[[[3-methyl-1-phenylmethyl-4-piperidinyl]amino]acetyl]piperazine;
2-(3,4-Dichlorophenyl)-4-[3,5-dimethylbenzoyl]-1-[[[1-(phenylmethyl)-3-piperidinyl]amino]acetyl]piperazine (diastereomers);
2-(3,4-Dichlorophenyl)-4-(3,5-dimethylbenzoyl)-1-[[[8-(phenylmethyl)-8-azabicyclo[3.2.1]oct-3-yl]amino]acetyl]piperazine;
2-(3,4-Dichlorophenyl)-4-(3,5-dimethylbenzoyl)-1-[[[8-methyl-8-azabicyclo]3.2.1]oct-3-yl]amino]acetyl]piperazine;
2-(3,4-Dichlorophenyl)-4-[3,5-dimethylbenzoyl]-1-[[[1,3-bis(phenylmethyl)-4-piperidinyl]amino]acetyl]piperazine (diastereomers);
2-(3,4-Dichlorophenyl)-4-[3,5-dimethylbenzoyl]-1-[[[3-methyl-1-phenylmethyl-4-piperidinyl]amino]acetyl]piperazine;
2-(3,4-Dichlorophenyl)-4-[3,5-dimethylbenzoyl]-1-[[[1-(phenylmethyl)-3-(2-propenyl)-4-piperidinyl]amino]acetyl]piperazine;
Trichloroethyl-4-[[[-(3,4-dichlorophenyl)-4-(3,5-dimethylbenzoyl)-1-piperazinyl]-2-oxoethyl]amino]-2-phenyl-1-piperidinecarboxylate;
2-(3,4-Dichlorophenyl)-4-(3,5-dimethylbenzoyl)-1-[3-[[5-phenylmethyl-(1S,4S)-2,5-diazabicyclo[2.2.1]heptane-2-yl]-1-oxopropyl]piperazine;
(-)-Phenyl-4-[[2-[2-(3,4-dichlorophenyl)-4-(3,5-dimethylbenzoyl)-1-piperazinyl-2-oxoethyl]amino]-1-piperidinecarboxylate, hemihydrate;
2-(3,4-Dichlorophenyl)-4-(3,5-dimethylbenzoyl)-1-[[[1-[(1H-pyrrol-2-yl)methyl]-4-piperidinyl]amino]acetyl]piperazine, hemihydrate;
2-(3,4-Dichlorophenyl)-4-(3,5-dimethylbenzoyl)-1-[[[1-[(1H-pyrrol-2-yl)carbonyl]-4-piperidinyl]amino]acetyl]piperazine, hemihydrate;
(-)-2-(3,4-Dichlorophenyl)-4-(3,5-dimethylbenzoyl)-1-[[[1-[(1H-imidazol-2-yl)methyl]-4-piperidinyl]amino]acetyl]piperazine, dihydrate;
2-(3,4-Dichlorophenyl)-4-(3,5-dimethylbenzoyl)-1-[1-oxo-3-[[1-phenylmethyl-4-piperidinyl]amino]propyl]piperazine;
2-(3,4-Dichlorophenyl)-4-(3,5-dimethylbenzoyl)-1-[1-oxo-3-N-methyl-[[1-phenylmethyl-4-piperidinyl]amino]propyl]piperazine;
(-)-1,2-Dimethylethyl-5-[3-[2-(3,4-dichlorophenyl)-4-(3,5-dimethylbenzoyl)-1-piperazinyl]-3-oxopropyl]-(1S,4S)-2,5-diazabicyclo[2.2.1]heptane-2-carboxylate;
(-)-1-[3-(1S,4S)-2,5-Diazabicyclo[2.2.1]heptan-2-yl-1-oxopropyl]-2-(3,4-dichlorophenyl)-4-(3,5-dimethylbenzoyl) piperazine, dihydrochloride;
(-)-N-[4-[[5-[3-[2-(3,4-dichlorophenyl-4-(3,5-dimethylbenzoyl]-1-piperazinyl]-3-oxopropyl]-(1S,4S)-2,5-diazabicyclo[2.2.1]heptan-2-yl]methyl-2-thiazolyl]acetamide;
(-)-N-[4-[[5-[3-[2-(3,4-dichlorophenyl-4-(3,5-dimethylbenzoyl)-1-piperazinyl]-3-oxopropyl]-(1S,4S)-2,5-diazabicyclo[2.2.1]heptan-2-yl]methyl]phenyl]acetamide;
2-[3,4-Dichlorophenyl-4-(3,5-dimethylbenzoyl)-1-[3-[5-(1-H-pyrroll-2-yl)methyl]-(1S,4S)-2,5-diazabicyclo[2.2.1]heptan-2-yl]-1-oxopropyl]piperazine;
(+,-)-2-(3,4-Dichlorophenyl)-4-[4-fluoro-1-naphthalenylcarbonyl]-1-[[[1-phenylmethyl-4-piperidinyl]amino]acetyl]piperazine;
(+,-)-1-[[3,5*-Bis*(trifluoromethyl)phenyl]methyl]-3-phenyl-piperazine, dihydrochloride, quarter hydrate;
(+,-)-4-[[3,5-*Bis*(trifluoromethyl)phenyl]methyl]-2-phenyl-1-[[[1-phenylmethyl-4-piperidinyl]amino]acetyl]piperazine, trihydrochloride, dihydrate;
(+,-)-4-[[3,5*-Bis*(trifluoromethyl)phenyl]methyl]-2-phenyl-N-[1-phenylmethyl-4-piperidinyl]-1-piperazineethanamine, tetrahydrochloride, hydrate;
2-(3,4-dichlorophenyl)piperazine;
(+,-)-4-[[3,5-*Bis*(trifluoromethyl)phenyl]methyl]-2-(3,4-dichlorophenyl)-1-[[[1-phenylmethyl-4-piperidinyl]amino]acety]]piperazine;
(+,-)-4-[[3,5-*Bis*(trifluoromethyl)phenyl]acetyl]-2-phenyl-1-[[[1-phenylmethyl-4-piperidinyl]amino]acetyl]piperazine;
(+,-)-4-[2-[3,5*-Bis*(trifluoromethyl)phenyl]ethyl]-2-phenyl-N-[1-phenylmethyl-4-piperidinyl]-1-piperazineethanamine, tetrahydrochloride, hemihydrate;
(+,-)-2-Phenyl-1-[[[1-phenylmethyl-4-piperidinyl]amino]acetyl]-4-[(3,4,5-trimethoxylphenyl)acetyl]piperazine, hemihydrate;
(+/-)-4-[2-[3,5-*Bis*(trifluoromethyl)phenyl)ethyl]-2-phenyl-1-[[[1-phenylmethyl-4-piperidinyl]amino]acetyl]piperazine, trihydrochloride;
(+,-)-2-Phenyl-1-[[[1-phenylmethyl-4-piperidinyl]amino]acetyl]-4-[2-(3,4,5-trimethoxyphenyl)ethyl]piperazine;
(+,-)-[3,5*-Bis*(trifluoromethyl)benzoyl]-3-(3,4-dichlorophenyl)piperazine;
(+,-)-4-[3,5-*Bis*(trifluoromethyl)benzoyl]-2-(3,4-dichlorophenyl)-1-[[[1-phenylmethyl-4-piperidinyl]amino]acetyl]piperazine;
(+)-4-[3,5-*Bis*(trifluoromethyl)benzoyl]-2-(3,4-dichlorophenyl)-1-[[[1-phenylmethyl-4-piperidinyl]amino]acetyl]piperazine, dihydrochloride, dihydrate;
(-)-4-[3,5-*Bis*(trifluoromethyl)benzoyl]-2-(3,4-dichlorophenyl)-1-[[[1-phenylmethyl-4-piperidinyl]amino]acetyl]piperazine, dihydrochloride, dihydrate;
(+,-)-2-(3,4-Dichlorophenyl)-1-[[[1-phenylmethyl-4-piperidinyl]amino]acetyl]-4-(3,4,5-trimethoxybenzoyl)piperazine;
(+,-)-2-(3,4-Dichlorophenyl)-4-[3-(1-methylethoxy)benzoyl]-1-[[[I-phenylmethyl-4-piperidinyl]amino]acetyl]piperazine, hemihydrate;
(+,-)-2-(3,4-Dichlorophenyl)-4-[2-methoxybenzoyl]-1-[([1-phenylmethyl-4-piperidinylamino]acetyl]piperazine;
(+,-)-4-[3,5*-Bis*(trifluoromethyl)benzoyl]-2-(3,4-dichlorophenyl)-1-[[5-phenylmethyl-2,5-diazabicyclo[2.2.1]heptan-2-yl]acetyl]piperazine;
(+,-)-4-[[3,5-*Bis*(trifluoromethyl)phenyl]acetyl]-2-phenyl-1-[[[I-1-phenylmethyl-4-piperidinyl]amino]acetyl]piperazine;
(+,-)-2-Phenyl-1-[[1-phenylmethyl-4-piperidinyl]aminolacetyl]-4-[(3,4,5-trimethoxylphenyl)acetyl]piperazine, hemihydrate;
(+,-)-4-[3,5-*Bis*(trifluoromethyl)benzoyl]-2-phenyl-1-[[[1-phenylmethyl-4-piperidinyl]amino]acetyl]piperazine;
(+,-)-4-[3,5-Dimethylbenzoyl]-2-(3,4-dichlorophenyl)-1-[[[1-phenylmethyl-4-piperidinyl]amino]acetyl]piperazine;
(+,-)-2-(3,4-Dichlorophenyl)-4-(3,5-dimethylbenzoyl)-1-(([1-(2-furanylmethyl)-4-piperidinyl)amino]acetyl]]piperazine;
(+,-)-1-[[[1-[[[1,1'-Biphenyl]-4-yl]methyl]-4-piperidinyl]amino]acetyl]-2-(3,4-dichlorophenyl)-4-(3,5-dimethylbenzoyl)piperazine;
(+,-)-2-(3,4-Dichlorophenyl)-4-[(4-fluoro-1-5-naphthalenyl)carbonyl]-1-[[[1-phenylmethyl-4-piperidinyl]amino]acetyl]piperazine;
(+,-)-4-(3,5-Dimethylbenzoyl)-1-[[[1-phenylmethyl-4-piperidinyl]amino]acetyl]-2-[4-(trifluoromethyl)phenyl]piperazine,1.2 hydrate;
(+,-)-4-[3,5-*Bis*(trifluoromethyl)benzoyl]-2-(3-hydroxyphenyl)-1-[[[1-phenylmethyl-4-piperidinyl]amino]acetyl]piperazine, dimaleate, hemihydrate;
(+,-)-4-[3,5-Bis(trifluoromethyl)benzoyl]-2-(4-hydroxyphenyl)-1-[[[1-phenylmethyl-4-piperidinyl]amino]acetyl]piperazine, dimaleate, hemihydrate;
2-(R,S)-(3,4-Dichlorophenyl)-4-(3,5-dimethylbenzoyl)-1-[3-hydroxy-1-oxo-2(S)-[[1-phenylmethyl-4-piperidinyl]amino]propyl]piperazine, hemihydrate;
2-(R,S)-(3,4-Dichlorophenyl)-4-(3,5-dimethylbenzoyl)-1-[4-methyl-1-oxo-2(R,S)-[[1-phenylmethyl-4-piperidinyl]amino]pentyl]piperazine;
(+,-)-N-[2-[2-(3,4-Dichlorophenyl)-4-(3,5-dimethylbenzoyl)-1-piperazinyl]-2-oxoethyl]-N-(1-phenylmethyl)-4-piperidinyl]acetamide, hemihydrate;
(+,-)-2-(3,4-Dichlorophenyl)-4-(3,5-dimethylbenzoyl)-1-[[[2-hydroxyethyl][1-phenylmethyl-4-piperidinyl]amino]acetyl]piperazine, hemihydrate;
(+,-)-N-[2-[2-(3,4-dichlorophenyl)-4-(3,5-dimethylbenzoyl)-1-piperazinyl]-2-oxoethyl]-N,N-dimethylamino-1-phenylmethyl-4-piperidinaminium bromide, dimethanolate;
(+,-)-2-(3,4-Dichlorophenyl)-4-(3,5-dimethylbenzoyl)-1-[[methyl[1-phenylmethyl-4-piperidinyl]amino]acetyl]piperazine;
(+,-)-2-(3,4-Dichlorophenyl)4-(3,5-dimethylbenzoyl)-2-methyl-1-[[[1-phenylmethyl-4-piperidinyl]amino]acetyl]piperazine, 0.6 methanol;
(+,-)-2-(3,4-Dichlorophenyl)-4-(4-fluoro-1-naphthalenylcarbonyl)-2-methyl-1-[[[1-phenylmethyl-4-piperidinyl]amino]acetyl]piperazine;
(+,-)-1-[3,5-*Bis*(trifluoromethyl)benzoyl]-2-(3,4-dichlorophenyl)-4-[[[1-phenylmethyl-4-piperidinyl]amino]acetyl]piperazine, dimaleate, hydrate;
(+,-)-2-(3,4-Dichlorophenyl)-4-(3,5-dimethylbenzoyl)-1-[[[1-[1-oxo-2-phenyl)ethyl]4-piperidinyl]amino]acetyl]piperazine;
(+,-)-1-Dimethylethyl-4-[[2-[2-(3,4-dichlorophenyl)-1-(,3,5-dimethylbenzoyl)-1-piperazinyl]-2-oxoethyl]amino]-1-piperidinecarboxylate;
(+,-)-2-(3,4-Dichlorophenyl)-4-[3,5-dimethylbenzoyl]-1-[(4-piperidinylamino)acetyl]-piperazine, dihydrochloride;
(-)-2-(3,4-Dichlorophenyl)-4-[3,5-dimethylbenzoyl]-1-[(4-piperidinylamino)acetyl]-piperazine, dihydrochloride;
(+,-)-1-Benzoyl-4-[[2-[2-(3,4-dichlorophenyl)-4-(3,5-dimethylbenzoyl)-1-piperazinyl]-2-oxoethyl]amino]piperidine;
(+,-)-2-(3,4-Dichlorophenyl)-4-(3,5-dimethylbenzoyl)-1-[[[1-(2-oxo-2-phenylethyl)-4-piperidinyl]amino]acetyl]piperazine;
(+,-)-2-(3,4-Dichlorophenyl)-4-(3,5-dimethylbenzoyl)-1-[[[1-(3-phenylpropyl)-4-piperidinyl]amino]acetyl]piperazine;
2-(3,4-Dichlorophenyl)-4-[3,5-dimethylbenzoyl]-1-[[[3-methyl-1-phenylmethyl-4-piperidinyl]amino]acetyl]piperazine (diastereomers);
2-(3,4-Dichlorophenyl)-4-[3,5-dimethylbenzoyl]-1-[[[1-phenylmethyl-3-piperidinyl]amino]acetyl]piperazine;
(+,-)-3,5-Dimethylbenzoyl-3-(3,4-dichlorophenyl)piperazine;
(+,-)-Bromoacetyl-2-(3,4-dichlorophenyl)-4-(3,5-dimethylbenzoyl)piperazine;
(+)-3,5-Dimethylbenzoyl-3-(3,4-dichlorophenyl)piperazine (enantiomer);
2-(3,4-Dichlorophenyl)-4-(3,5-dimethylbenzoyl)-1-[3-[1,2-dihydro-1-methanesulfonyl-spiro[3H-indole-3,4'-piperidine]-1'-yl]-1-oxopropyl]piperazine (enantiomer);
2-(3,4-Dichlorophenyl)-1-[3-[3,4-dihydro-4-oxo-6-methoxy-spiro[2H-1-benzopyran-2,4'-piperidin]-1'-yl]-1-oxopropyl]-4-(3,5-dimethylbenzoyl)piperazine (enantiomer);
2-(3,4-Dichlorophenyl)-4-(3,5-dimethylbenzoyl)-1-[3-(1-phenyl-4-oxo-1,3,8-triazaspiro[4.5]decan-8-yl)-1-oxopropyl]piperazine (enantiomer);
(+,-)-1-[2-[4-[[3,5-*Bis*(trifluoromethyl)phenyl]methyl]-2-(3,4-dichlorophenyl)-1-piperazinyl]ethyl]-4-phenyl-4-piperidinol;
(+,-)-[3,5-*Bis*(trifluoromethyl)benzoyl]-3-(3,4-dichlorophenyl)piperazine;
(+,-)-4-[3,5*-Bis*(trifluoromethyl)benzoyl]-2-(3,4-dichlorophenyl)-1-[[(4-hydroxy-4-phenyl-1-piperidinyl)]acetyl]piperazine;
(+,-)-2-(3,4-Dichlorophenyl)-4-(2-methoxybenzoyl)-1-[[(4-hydroxy-4-phenyl-1-piperidinyl)]acetyl]piperazine;
(+,-)-4-[3,5*-Bis*(trifluoromethyl)benzoyl]-2-phenyl-1-[[(4-hydroxy-4-phenyl-1-piperidinyl)]acetyl]piperazine;
(+,-)-[3,5-Dimethylbenzoyl]-3-(3,4-dichlorophenyl)piperazine;
(+)-[3,5-Dimethylbenzoyl]-3-(3,4-dichlorophenyl)piperazine;
4-[3,5*-Bis*(trifluoromethyl)benzoyl]-2-(3,4-dichlorophenyl)-1[1,2-dioxo-2-[4-(phenylmethyl)-1-piperazinyl]ethyl]piperazine;
1-[3,5-Dimethylbenzoyi]-3-(3,4-dichlorophenyl)piperazine (enantiomer);
2-(3,4-Dichlorophenyl)-4-(3,5-dimethylbenzoyl)-1-[[(4-carbethoycyclohexyl) amino]acetyl]piperazine (enantiomer);
2-(3,4-Dichlorophenyl)-4-(3,5-dimethylbenzoyl)-1-[[(3-methylcyclohexyl)amino]acetyl]piperazine (diastereomers);
1-Cyclohexylaminoacetyl-2-(3,4-dichlorophenyl)-4-(3,5-dimethylbenzoyl)piperazine (enantiomer);
1-Cycloheptylaminoacetyl-2-(3,4-dichlorophenyl)-4-(3,5-dimethylbenzoyl)piperazine (enantiomer);
1-[(4-Cyano-4-phenylcyclohexyl)amino]acetyl-2-(3,4-dichlorophenyl)-4-(3,5-dimethylbenzoyl)piperazine (enantiomer);
(+/-)-2-(3,4-Dichlorophenyl)-4-(3,5-dimethylbenzoyl)-1-[[(4-phenylcyclohexyl]amino]acetfl]piperazine;
2-(3,4-Dichlorophenyl)-4-(3,5-dimethylbenzoyl)-1-[3-[4-(2-keto-1-benzimidazolinyl)piperidinyl]-1-oxopropyl]piperazine (enantiomer);
2-(3,4-Dichlorophenyl)-4-(3,5-dimethylbenzoyl)-1-[3-[4-(phenylmethyl)-1-piperidinyl]-1-oxopropyl]piperazine (enantiomer);
2-(3,4-Dichlorophenyl)-4-(3,5-dimethylbenzoyl)-1-[1-oxo-3-[4-phenylmethyl-1-piperidinyl]propyl]piperazine (enantiomer);
2-(3,4-Dichlorophenyl)-4-(3,5-dimethylbenzoyl)-1-[3-[4-hydroxy-4-phenyl-1-piperidinyl]-1-oxopropyl]piperazine (enantiomer);
(+,-)-[3,5-Dimethylbenzoyl]-3-(3,4-dichlorophenyl)piperazine;
(+,-)-Bromoacetyl-2-(3,4-dichlorophenyl)-4-(3,5-dimethylbenzoyl)piperazine;
(+)-[3,5-Dimethylbenzoyl]-3(R)-(3,4-dichlorophenyl)piperazine;
(-)-Bromoacetfl-2(R)-(3,4-dichlorophenyl)-4-(3,5-dimethylbenzoyl)piperazine (enantiomer);
1,1-Dimethylethyl-5-[[2(R)-[2-(3,4-dichlorophenyl)4-(3,5-dimethylbenzoyl)-1-piperazinyl]-2-oxoethyllamino]-2-azabicyclo[2.2.1]heptane-2-carboxylate (diastereomers);
2(R)-(3,4-Dichlorophenyl)-4-(3,5-dimethylbenzoyl)-1-[[[2-azabicyclo[2.2.1]heptan-5-yl]amino]acetyl]piperazine (diastereomers);
2(R)-(3,4-Dichlorophenyl)-4-(3,5-dimethylbenzoyl)-1-[[[2-phenylmethylazabicyclo[2.2.1]heptan-5-yl]amino]acetyl]piperazine (diastereomers);
2(R)-(3,4-Dichlorophenyl)-4-(3,5-dimethylbenzoyl)-1-[[[2-phenylmethylazabicyclo[2.2.1]heptan-5-yl]amino]acetyl]piperazine (diastereomers);
2(R)-(3,4-Dichlorophenyl)-4-(3,5-dimethylbenzoyl)-1-[[[2-phenylmethylazabicyclo[2.2.1]heptan-5-yl]amino]acetyl]piperazine (enantiomers);
2(R)-(3,4-Dichlorophenyl)-4-(3,5-dimethylbenzoyl)-1-[[[2-phenylmethylazabicyclo[2.2.1]heptan-5-yl]amino]acetyl]piperazine (enantiomers);
2(R)-(3,4-Dichlorophenyl)-4-(3,5-dimethylbenzoyl)-1-[[[2-(4-acetyaminophenylmethyl)azabicyclo[2.2.1]heptan-5-yl]amino]acetyl]piperazine;
N-[4-[[5-[[2-[2(R)-(3,4-dichlorophenyl)-4-(3,5-dimethylbenzoyl)-1-piperazinyl]-2-oxoethyl]amino]-2-azabicyclo[2.2.1]heptan-2-yl]methyl-2-thiazolyl]acetamide (diastereomers);
(-)-1,1-Dimethylethyl-2-[3-[2(R)-(3,4-dichlorophenyl)-4-(dimethylbenzoyl)-1-piperazinyl]-3-oxopropyl]-1(S),4(S)-2,5-diazabicyclo[2.2.1]heptane-5-carboxylate;
(-)-1-[3-[(1S),4(S)-2,5-Diazabicyclo[2.2.1]heptan-2-yl)-1-oxopropyl]-2(R)-(3,4-dichlorophenyl)-4-(3,5-dimethylbenzoyl)piperazine;
1,1-Dimethylethyl-[2-[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]-2-oxo-1(R)-phenylethyl]carbamate;
2-[(R)-Aminophenylacetyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane, dihydrochloride (enantiomer);
(-)-1,1-Dimethylethyl-4-[(2-[2(R)-(3,4-dichlorophenyl)-1-(3,5-dimethylbenzoyl)-1-piperazinyl]-2-oxoethyl]amino]-1-piperidinecarboxylate (enantiomer);
(+,-)-2-(3,4-Dichlorophenyl)-4-[3,5-dimethylbenzoyl]-1-[(4-piperidinylamino)acetyl]piperazine, dihydrochloride;
(-)-2(R)-(3,4-Dichlorophenyl)-4-[3,5-dimethylbenzoyl]-1-[(4-piperid inylamino)acetyl]piperazine, dihydrochloride (enantiomer);
2(R)-(3,4-Dichlorophenyl)-4-(3,5-dimethylbenzoyl)-1-[1-oxo-3-[5-phenylsulfonyl)-2,5-diazabicyclo[2.2.1]heptan-2-yl]propyl]piperazine;
5-[1-Cyanoimino-1-methylthio]-2-[3-[2(R)-(3,4-dichlorophenyl)-4-(3,5-dimethylbenzoyl)-1-piperazinyl]-3-oxopropyl-2,5-diazabicyclo[2.2.1]heptane;
5-[1-Cyanoimino-1-phenylaminomethyl]-2-[3-[2(R)-(3,4-dichloro-phenyl)-4-(3,5-dimethylbenzoyl)-1-piperazinyl]-3-oxopropyl-2,5-diazabicyclo[2.2.1]heptane;
5-[1-Cyanoimino-1-phenylmethylaminomethyl]-2-[3-[2(R)-(3,4-dichlorophenyl)-4-(3,5-dimethylbenzoyl)-1-piperazinyl]-3-oxopropyl-2,5-diazabicyclo[2.2.1]heptane;
2(R)-(3,4-Dichlorophenyl)-4-(3,5-dimethylbenzoyl)-1-[[6-(phenylmethyl)-2-azabicyclo[2.2.2]octan-6-yl]methylamino]acetyl]piperazine;
2(R)-(3,4-Dichlorophenyl)-4-(3,5-dimethylbenzoyl)-1-[[6-(phenylmethyl)-6-azabicyclo[3.2.2.]nonan-3-yl]amino]acetyl]piperazine;
Methyl [1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenyl]carbamate (enantiomer);
N-[1(R)-[[5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl-2-phenylethyl]-N'-methylurea (enantiomer);
5-[3-[2(R)-(3,4-Dichlorophenyl)-4-(3,5-dimethylbenzoyl)-1-piperazinyl]-3-oxopropyl]-2-[2(R)-methylaminocarbonylamino-1-oxo-3-(2-thienyl)propyl]-1(S),4(S)-2,5-diazabicyclo[2.2.1]heptane (enantiomer);
2-[3-[2-(R)-(3,4-Dichlorophenyl)-4-(3,5-dimethylbenzyoyl)-1-piperazinyl]-3-oxopropyl]-5-[2-iminomethylaminomethylamino]-1-oxo-3-phenylpropyl]-1(S),4(S)-2,5-diazobicyclo[2.2.1]heptane;
5-[3-[2(R)-(3,4-Dichlorophenyl)-4-(3,5-dimethylbenzoyl)-1-piperazinyl]-3-oxopropyl]-2-(2(R)-hydroxy-1-oxo-3-phenylpropyl)-1(S),4(S)-2,5-diazabicyclo-[2.2.1]heptane (enantiomer);
5-[3-[2(R)-(3,4-Dichlorophenyl)-4-(3,5-dimethylbenzoyl)-1-piperazinyl]-3-oxopropyl]-2-(2(S)-hydroxy-1-oxo-3-phenylpropyl)-1(S),4(S)-2,5-diazabicyclo-[2.2.1]heptane (enantiomer);
2-[2(S)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1]heptane (enantiomer);
2-[2(R)-(Cyanomethoxy)-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1]heptane (enantiomer);
2-[2(R)-2-(Aminohydroxyimino)ethoyl]-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo[2.2.1]heptane;
[1(R)-[[5-[3-[2(R)-(3,4-Dichlorophenyl)-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylcarbamate (enantiomer);
[1(S)-[[5-[3-[2(R)-(3,4-Dichlorophenyl)-4-(3,5-dimethylbenzoyl)-1-piperazinyl]-3-oxopropyl]-1[S),4(S)-2,5-diazabicyclo[2.2.1]heptan-2-yl]carbonyl]-2-phenylethyl]methylcarbamate (enantiomer);
2-[2(S)-Methoxy-1-oxo-3-phenylpropyl]-5-[3-[2(R)-(3,4-dichlorophenyl)-4-(3,5-dimethylbenzoyl)-1-piperazinyl]-3-oxopropyl]-1(S),4(S)-2,5-diazabicyclo-[2.2.1]heptane (enantiomer);
(1R,4R) -1,1-Dimethylethyl-5-hydroxy-2-azabicyclo[2.2.1]heptane-2-carboxylate and (1S,4S)-1,1-dimethylethyl-5-hydroxy-2-azabicyclo [2.2.1]heptane-2-carboxylate;
*Exo*-1,1-dimethylethyl-5-[2-[2(R)-(3,4-dichlorophenyl)-4-(3,5-dimethylbenzoyl)-1-piperazinyl]-2-oxoethoxy]-1-(R),4(R)-2-azabicyclo[2.2.1]heptane-2-carboxylate (enantiomer);
2(R)-(3,4-Dichlorophenyl)-4-(3,5-dimethylbenzoyl)-1-[[[*exo*-1(R),4(R)-2-azabicyclo[2.2.1]heptan-5-yl]oxy]acetyl]piperazine, hydrochloride (enantiomer B);
2(R)-(3,4-Dichlorophenyl)-4-(3,5-dimethylbenzoyl)-1-[[[*exo*-2-(3-thienylmethyl)-1(S),4(S)-2-azabicyclo[2.2.1]heptan-5-yl]oxy]acetyl]piperazine (enantiomer);
1,1-Dimethylethyl-3-exo-hydroxy-8-azabicyclo[3.2.1]octane-8-carboxylate and 1,1-dimethylethyl-3-*endo*-hydroxy-8-azabicyclo [3.2.1]octane-8-carboxylate;
*Exo*-1,1-dimethylethyl-3-[2-[2(R)-(3,4-dichlorophenyl)-4-(3,5-dimethylbenzoyl)-1-piperazinyl]-2-oxoethoxy]-8-aza[3.2.1]octane-8-carboxylate (enantiomer);
2(R)-(3,4-Dichlorophenyl)-4-(3,5-dimethylbenzoyl)-1-[[[*exo*-8-aza-3.2.1]1]octan-3-yl]oxy]acetyl]piperazine, hydrochloride (enantiomer);
*Endo*-1,1-dimethylethyl-3-[2-[2(R)-3,4-dichlorophenyl-4-(3,5-dimethylbenzoyl)-1-piperazinyl]-2-oxoethoxy]-8-aza[3.2.1]octane-8-carboxylate (enantiomer B);
2(R)-3,4-Dichlorophenyl-4-(3,5-dimethylbenzoyl)-1-[[[*endo*-8-aza-3.2.1]octan-3-yl]oxy]acetyl]piperazine, hydrochloride (enantiomer);
N-1(R)-[[5-[3-[2(R)-3,4-dichlorophenyl-4-(3,5-dimethylbenzoyl)-1-piperazinyl]-3-oxopropyl]-1(S),4(S)-2,5-diazabicyclo[2.2.1]heptan-2-yl]carbonyl]-2-phenylethyl]methylsulfonamide (enantiomer);
2-[2(R)-(Cyanomethylamino)-1-oxo-3-phenylpropyl]-5-[3-[2-(R)-3,4-dichlorophenyl-4-(3,5-dimethylbenzoyl)-1-piperazinyl]-3-oxopropyl]-1(S),4(S)-2,5-diazabicyclo[2.2.1]heptane;
1,1-Dimethylethyl[1(R)-[[*endo*-5-[[2-[2(R)-3,4-dichlorophenyl-4-(3,5-dimethylbenzoyl)-1-piperazinyl]-2-oxoethyl]amino]-2-azabicyclo[2.2.1]-heptan-2-yl]carbonyl]-2-phenylethyl]carbamate (enantiomer);
*Endo*-2-(2(R)-amino-1-oxo-3-phenylpropyl)-5-[[2-[2(R)-3,4-dichlorophenyl-4-(3,5-dimethylbenzoyl)-1-piperazinyl]-2-oxoethyl]amino]-2-aza[2.2.1]heptane, dihydrochloride (enantiomer);
1,1-Dimethylethyl-3-phenylmethylamino-8-azabicyclo[3.2.1]octane-8-carboxylate (*exo* and *endo* products);
1,1-Dimethylethyl-3-[[2-[2-(R)-3,4-dichlorophenyl-2-oxoethyl]*endo*-amino]-8-azabicyclo[3.2.1]octane-8-carboxylate;
2-(R)-3,4-Dichlorophenyl-4-(3,5-dimethylbenzoyl)-1-[[[8-azabicyclo-[3.2.1]octan-3-yl]*endo*-amino]acetyl]piperazine, hydrochloride;
1,1-Dimethylethyl-3-[[2-[2-(R)-3,4-dichlorophenyl-2-oxoethyl]*exo*-amino]-8-azabicyclo[3.2.1]octane-8-carboxylate;
2-(R)-3,4-Dichlorophenyl-4-(3,5-dimethylbenzoyl)-1-[[[8-azabicyclo-[3.2.1]octan-3-yl]*exo*-amino]acetyl]piperazine, hydrochloride;
*Exo*-8-(2(S)-amino-1-oxo-3-phenylpropyl)-3-[[2-[2(R)-3,4-dichlorophenyl-4-(3,5-dimethylbenzoyl)-1-piperazinyl]-2-oxoethyl]amino]-8-aza[3.2.1]octane, dihydrochloride (enantiomer);
1,1-Dimethylethyl[1(S)-[[*endo*-3-[[2-2(R)-3,4-dichlorophenyl-4-(3,5-dimethylbenzoyl)-1-piperazinyl]-2-oxoethyl]amino]-8-aza[3.2.1]octan-8-yl]carbonyl]-2-phenylethyl]carbamate (enantiomer);
*Endo*-8-(2(S)-amino-1-oxo-3-phenylpropyl)-3-[[2-[2(R)-3,4-dichlorophenyl-4-(3,5-dimethylbenzoyl)-1-piperazinyl]-2-oxoethyl]amino]-8-aza[3.2.1]octane, dihydrochloride (enantiomer);
1,1-Dimethylethyl-[1-(R)-[[*exo*-3-[[2-2(R)-3,4-dichlorophenyl-4-(3,5-dimethylbenzoyl)-1-piperazinyl]-2-oxoethyl]amino]-8-aza[3.2.1]octan-8-yl]carbonyl]-2-phenylethyl]carbamate (enantiomer);
*Exo*-8-(2(R)-amino-1-oxo-3-phenylpropyl)-3-[[2-[2(R)-3,4-dichlorophenyl-4-(3,5-dimethylbenzoyl)-1-piperazinyl]-2-oxoethyl]amino]-8-aza[3.2.1]octane, dihydrochloride (enantiomer);
(+,-)-N-[4-[[*Endo*-5-[[2-[2(R)-3,4-dichlorophenyl-4-(3,5-dimethylbenzoyl)-1-piperazinyl]-2-oxoethyl]amino]-2-azabicyclo[2.2.1]heptan-2-yl]methyl]phenyl]-acetamide;
(+,-)-N-[3-[[*Endo*-5-[[2-[2(R)-3,4-dichlorophenyl-4-(3,5-dimethyl-benzoyl)-1-piperazinyl]-2-oxoethyl]amino]-2-azabicyclo[2.2.1]heptan-2-yl]methyl]phenyl]acetamide;
(+,-)-1,1-Dimethylethyl-*trans*-2-[[5-[3-[2(R)-3,4-dichlorophenyl-4-(3,5-dimethylbenzoyl)-1-piperazinyl]-3-oxopropyl]-1(S),4(S)-2,5-diazabicyclo-[2.2.1]heptan-2-yl]carbonyl]-3-phenyl-1-azetidinecarboxylate (enantiomer); and
(+,-)-5-[3-[2(R)-3,4-Dichlorophenyl-4-(3,5-dimethylbenzoyl)-1-piperazinyl]-3-oxopropyl]-2-[(trans-3-phenyl-2-azetidinyl)carbonyl]-1(S),4(S)-2,5-diazabicyclo[2.2.1]heptane, dihydrochloride (enantiomer).
